# EUROPEAN PATENT APPLICATION

(11) **EP 2 631 295 A2**
(43) Date of publication of application: **28.08.2013**
(21) Application number: 13155896.7
(22) Date of filing: 12.02.2008
(51) Int. Cl.: C12N 15/67, C12N 15/80, C12N 1/14, C12P 1/02, C12P 21/00, C12N 9/02, C12N 9/34

(54) **A recombinant host cell for the production of a compound of interest**

(30) Priority: 15.02.2007 EP 07102493
(62) Divisional of application: 08708913.2
(71) Applicant: DSM IP Assets B.V., 6411 TE Heerlen (NL)
(72) Inventor: Sagt, Cornelis, Maria, Jacobus, 6100 AC Echt (NL); Van Peij, Noël Nicolaas Maria Elisabeth, 6100 AC Echt (NL); Wösten, Herman Abel Bernard, 6100 AC Echt (NL); Costa Rodrigues Alves, Alexandra Maria, 6100 AC Echt (NL); De Vries, Ronald Peter, 6100 AC Echt (NL); Levin Chucrel, Ana Marcela, 6100 AC Echt (NL)
(74) Representative: Monaco, Vania

(57) **Abstract**

The present invention relates to a recombinant host cell for the production of a compound of interest. The present invention also relates to isolated fungal promoter DNA sequences, to DNA constructs, vectors, and fungal host cells comprising these promoters in operative association with coding sequences encoding a compound of interest. The present invention also relates to methods for expressing a gene of interest and/or producing compounds of interest using a promoter according to the invention.

SEQUENCE LISTING

<110>  DSM IP Assets B.V.

<120>  A recombinant host cell for the production of a compound of
       interest

<130>  25226WO

<160>  55

<170>  PatentIn version 3.2

<210>  1
<211>  648
<212>  DNA
<213>  Schizophyllum commune

<400>  1
cgaccgagcg cgcgccaccc agcctatccc gcgcgggtcg ggacccaaaa taagcgggcc      60

ccgccgcgcc ccgtcgggcg agcgggtgta tctacgaacg gaactgggag gcgactcgga     120

agagtttggt tagaaagggg aacaccatcg cggacggccc agtgctctgg dcagctgagc     180

gtgcattgtg ttcaattctg acctgtggca tgtaaggaac gtgctcggga tcggagggtg     240

gcgcgagagc ctcttcggtg tgagattagt aactgtactg cgaagccgcg gaggggttag     300

gatgagaggt agacagggtc gcagcccagg tgcgagaagg actgcgaagg actgttcttc     360

gaccgcgcac ctgcaattgc gcgcatggat agaatagagc gtcgccctcg agggggactc      420

gaccagggct ggtggtggcg cccgacggga ctggctgggc atttgcagat ggcgcgcagt     480

ccaggccgcc gccgatgtgt tcatcccgtt ttgtcagtat cgatcggatc tttcgggcgt     540

gggtataaaa gcgcgccgcc cgccgtctcc ctctttctcc agcactccca tccagagcac     600

ttccctctcc catcgcatcc catcacacaa taatgcccat caccatgg                  648

<210>  2
<211>  1033
<212>  DNA
<213>  Schizophyllum commune

<400>  2
agcttctccg gccccgaatc gaacggcagg atgtgtgggc gtgtccaata ttgccatgaa      60

aatctgtcag aagtgagccc tctcgtcacc ctgtacagct tcgctgagtt gaaaagcagg     120

gttcatcttg ggctcactga tgcactgagc tcgaccggag aactaaatga ccagccggag     180

tgttcactaa cttaacgccg ggtattcagg gcagcttctc tatgttgcgc ctacgacgta     240

gatcaccgcc catgaacggg ggaaacgggg aggggtgcgt ttggtacgtc tttacgtctg     300

gctatgttgt attgaccagc gtctgcagaa gatgggcacg acgatgcgcc gagccggcca 360

gtgtcgtcgg atgtccactg ttgaggccat ccttttgcta gacagacgga agagctttgg 420

aggtgcgatt cctctacgaa tgggaagggg cttagatgga gagtgacacg tctgagctcc 480

ccaacacgcc ttcgccgagg gtgcgtctcc gcggacattc acctcagttc attgttctga 540

cctgcctaat tgtatagacc ggccaacaac cttgctgacg cccatcataa cagtgccctg 600

cacagagcct tcccactcag tcggcgcctc cctcaatcaa tcccactaac tcgccggctc 660

tgccccttcg ccgctcgaca cgtcgcttgg aagagcccgg gcacgggcgt ccgctccccc 720

cttccctccg cgtcgtcatg cacgcagcgt taatgttgct gcaggcgagc cgtaagtata 780

ttcaaaggcg tagcgaatga atagcaggcg cgcggggacc tggcacgcgc ggcatgaaca 840

tgcagacttg ggtgacgata acttgaactc agacgcggcg aatgaatatc caaacgcgcg 900

ggaagaaaat aatttacggg agcctcccca ggtataaaag cccctcaccc gctcactctt 960

tctccagtcg aacaccccag ttcaactacc cagcccttcc ttccttcgct atccttcytt 1020

acaacctgct cgc 1033

<210> 3
<211> 2527
<212> DNA
<213> Pycnoporus cinnabarinus

<400> 3
agatctccga accagaaatg cgattgcgtt caggcccaat taagaataaa gctgcgtcag 60

ggcagcgacg tatcttgatc catcattgac tcaccggcat cggcgtcaac accaaagcaa 120

gctcgtccca cccataggcg tgcaccggcc ggcgtgcgcc attgaggtac atgagcgggg 180

cgaaagtccg ccattggtag ccctgtcgtg gacgcgcggc gatgaaacgt ttcccaccat 240

tgggaagaaa cgtctgcggc ccatcatccc ttcaccggat gacaaggcgg cgtcgcgcct 300

ttgccgcaga ggccggcggg cgacatgcac agcgaaggtc cgttgcggat gggaagcagg 360

caatcagtgg gtgtcctacg ccgccacgat ggtcggggag cgtaggcgcc ctcccataag 420

gcggcaagca tcatgatgct ctccgattcg ggaagcctgg tgcgatgctg gagagactct 480

ctccgagaga ccagtgtgcg caacgttcct ggcctggaag actttaaagt gagtgtagaa 540

gggcgagcag aggacgatca tcggattgca ggaaccatcg gcatcctcag cctgggaagg 600

atggctcttg gtagacattc gcggaaggtg tcctagatgt gagcgggctt cttggatgat 660

catgtcgtaa ctttttctga cctcgtcggt ggtacgcatg gcaggattga gcattacggt 720

atgcctccca ttcataaacg ataacccctt ccttcaggtt ggtcatctcc atagagcggc 780

acgctctcaa ggcctaggct attcacacct ccttcgcaac atccctattc acggtgtctg 840

taaggaacga cttgtcatgg gatcacatga agtgcagcat actgttcgcc ggtctcgcag 900

tacagacgct agtacgggaa gtcgacatcc aagcgttcag tcaccacatg gcaaaaaagc 960

tgcaccatac tctttatggt gagttgttcg tgagtggtat acagtcattc atgagggaat 1020

gcccaccgga tagggtgtgg cggccgcaat attcatcgcc tggcaatagt cgatgtgcgt 1080

ccttgttcaa tgaatatcat gggtcacatg tggagacggt taaacagcgt tgactgtgaa 1140

tccctggtgt gtgttgggcc gaacaggtac gttgcaggaa caccaatatc tcttcggcag 1200

cccagttctt tgcgagcggc acaggcaggc atcgcgcaac agatcccagc catccggcct 1260

ctgacattcg ggatacctga agcccttcag gtacggagcg aagaggtggg ctctctgcag 1320

cgattggcgg acggatagct gtatttcctc tctcaccatt gggaagatgt gaaaggctcc 1380

atcatatagc ggctcaactc tacctcgaat gtccaaacac ggcgggaata cttatttatg 1440

tggacaaggc cgagctatga tagcttgctc ccgaagttgg taagtcccgc aatctgcggt 1500

tcaggcaaca gtctcggaaa aataagaaga atattgtagg tgcgtgtagg cgtatcgccc 1560

aaatgcgcac acacggaggc tttaggagat gaagcgcccg tgagcggtaa gggagttggt 1620

tcaccgccgc cccgaccgac tctctctctt tcccagcatc atgtctcggc gcaaacttta 1680

ccctctattg accaactcca cgagaaagca ggaacagctt ccttgtctct catgacgtcc 1740

gcaatccaga cccttagccg gttcgttact catcgttatc cctgccgcca tggtagtgga 1800

gtcagcctgg ccagtgcgta gtcccgtctc tcttgctgca ctagagaagc cccatgagac 1860

agcgtttttt gctttatttc tgctgtttct atagacacca taggggcaaa cgatcctgca 1920

cgcccagagg tattgggctc gtcagattcc cagtttttct cctcggtctg aatcggctgc 1980

acggcagata aatcggccgg aaatgctata gcccttcata gcccgctatg agagtcgcaa 2040

aaggcttgtc agtcaggtcg gtcgagtggc tctcacgaag agcgtcaact tcgcgcgaca 2100

gccgcctttc agggcaagat agatcctccc atcatcccct actgcgctca gcgccggtac 2160

cgaacaattg acttaccgac atcctccggg acgcgcaaat gctgttcgac ggaacgtaat 2220

cctcttcgtc ccgcctcttt tcgctctcac gcattccgtg tggttcgcgc gacggccgct 2280

catcaggacc agaccagtct caatgtctgg taccggcaca atggtgacac tgcggcaact 2340

gagtaggtct ggtcactctg gtgcaccgtc gcttacgctg accttcggga tactgtcctg 2400

cagacatctg gagcgcctgt ctttccccta gtataaatga tgtctgtccg caggtccttg 2460

aagaccgctc gagtcccact tgagttttag gtaggacctg tccaccaaac ccctctttct 2520

gatcatg 2527

<210> 4
<211> 2629
<212> DNA
<213> Pycnoporus cinnabarinus

<400> 4
aagcgggtct ctcccgcctc tgttggctct cacgcattcc gcatggttcg cgcgacggcc 60

gctcctcagg gccagaccag tgtcaatgtc tggtgccggt gcagtggtga cagtgcgaca 120

actgagtaag gctcgtcggt ttcctgcccc gccgcttatg ctgaccacgg gcgtgttgct 180

gtacagactt caagggcgtc tgtacttgcg tagtataaag gatgtcgctc gagagccgtt 240

cgaagacaac tcgagtccca cttgagtttc gacgaggaca tccctttcag cgacccttct 300

ttcagtcatg tccagattcc aatctctcct ctcctttgtc ctcgtctctc ttgccgctgt 360

ggccaacgct gccatcggtc ctgtggcaga cttgaccctc accaacgccg cggtcagccc 420

cgatggcttc agccgcgagg ctgttgtggt caatggcatc acccccgctc ctctcattgc 480

tggacaaaaa gtgagtacat attcctagct tacgaggggt attcacgtct gaccgaactg 540

gctagggcga ccgtttccag ctgaatgtca tcgacaatct gacgaaccat accatgctga 600

agactactag tatcgtaaga tatatacact ctctgttgac gccctctcgc tcatacgcgc 660

tcgctagcac tggcatggct tcttccagca cggtacgaat tgggccgacg gtgtgtcgtt 720

cgtcaaccag tgccccatcg cttcgggcca ttcgttcttg tacgacttcc aggttcccga 780

ccaagcaggt aatgagattt gacccgttct ttcattcggc gggcctgagc tcctctctct 840

ttgctaggga cgttctggta tcacagccat ctttccaccc agtactgtga tggtttgcgg 900

gggcccttcg tcgtctacga ccccaatgat cctcaggcta gcctgtatga cattgacaac 960

ggtgagcatt aaatgtcccg tgaccttccc tacgttctca tgcttccacc gctctcagat 1020

gacactgtca ttacattggc cgactggtac cacgtcgccg ctaagcttgg cccacgcttc 1080

ccgtacgctt tcttttgggc atctcggcgt ttcctgtggg ctcattctcg gggatcatag 1140

gcttggcgcg gatgcaactc tcatcaacgg gctgggtcga agccccggta ctaccactgc 1200

tgatctggca gttatcaagg tcacgcaggg caagcggttc gtatccacaa tcgccctcca 1260

gtcgctggct ctgacacttg ttcttcccgc aggtaccgat tccgtttggt gtccctttct 1320

tgcgacccga accacacctt tagcatcgat ggccacacta tgactgtgat cgaggcggac 1380

tcagtgaata ctcagcccct cgaagttgac tcgattcaga tcttcgccgc ccagcggtac 1440

tccttcgtgg taagtggaag ctcgctgaca tcatgccggt gatagatttc tcacacattg 1500

tcatacagct ggatgctagc cagcccgtcg acaactactg gatccgtgct aaccctgctt 1560

tcggaaacgt cggattcgcc ggtggaatca actctgctat tctgcggtat gacggcgcac 1620

ccgaggtgga gcctaccacg acccaaacca cttcgacgaa gcctctcaac gaggctgact 1680

tgcatcccct cacccctatg cctgtggtgc gtgtctccta aggcccatct cactgattgg 1740

atgcaaactg atggggtgca tatcagcctg gtcgtcccga ggccggtggt gttgacaagc 1800

ctctgaacat ggtcttcaac ttcgtgagca ttgccttcgc gccattgtgg gacaatatct 1860

gatgtccttg cagaacggca ccaacttctt catcaacaat cactcctttg tcccaccctc 1920

cgtcccggtt ctgctccaga ttctcagcgg tgctcaggcc gcgcaggacc tcgttccgga 1980

cggcagcgtc tacgttctcc cgagcaactc gtctattgag atctccttcc ccgcaactgc 2040

caatgctcct ggcacccctc accccttcca cctgcacggt gtacgtccgg tcctcgcgca 2100

tctgatgatg agttgatagc taacaatcac accagcacac cttcgctgtc gtccgaagcg 2160

ccggaagcag cgagtacaac tacgataacc cgatcttccg cgacgtcgtg agcaccggcc 2220

agcccggaga caacgtcacc atccggttcc agaccaacaa ccccggcccc tggttcctcc 2280

actgccacat cgacttccac ttggaagccg gttttgctgt cgtcctggcc gaggacactc 2340

cagacacggc ggcggtcaac cctgtccccc agtcgtggtc ggatctgtgc cccatctacg 2400

acgcacttga ccccagcgat ctctgagcgg tatcggtggc attatttgga aaacgttgaa 2460

tacgctggac ttccatcggt tacggattta cggattcggg ttaacattat gtacctcata 2520

cggcttggat gaaatggacg gtccgtccga gcttcattgt gcttcatgtt aacaaaaggg 2580

ttgtatgtag aataatttcc agggcaatca actcgacgct atcgatcac 2629

<210> 5
<211> 25
<212> DNA
<213> Artificial

<220>
<223> primer

<400> 5
ttctgaccat ggcgaggttc cagtc 25

<210> 6
<211> 30
<212> DNA
<213> Artificial

<220>
<223> primer

<400> 6
acagtaactg attcagctca gaggtcgctg 30


<210> 7
<211> 34
<212> DNA
<213> Artificial

<220>
<223> primer

<400> 7
accccctctt tctgaccatg gcgaggttcc agtc 34


<210> 8
<211> 36
<212> DNA
<213> Artificial

<220>
<223> primer

<400> 8
taacccgggc gctcagaggt cgctggggtc aagtgc 36


<210> 9
<211> 25
<212> DNA
<213> Artificial

<220>
<223> primer

<400> 9
tctgatcatg tcgaggttcc agtcc 25


<210> 10
<211> 27
<212> DNA
<213> Artificial

<220>
<223> primer

<400> 10

gtcttcaagg acctgcggac agacatc 27

<210> 11
<211> 30
<212> DNA
<213> Artificial

<220>
<223> primer

<400> 11
accaagctta gatctccgaa ccagaaatgc 30

<210> 12
<211> 33
<212> DNA
<213> Artificial

<220>
<223> primer

<400> 12
gactggaacc tcgccatggt cagaaagagg ggt 33

<210> 13
<211> 2000
<212> DNA
<213> Aspergillus niger

<400> 13
cctcaagctg tggcccagcc ttcctcaccc agccattttc gagctggttg gactccccaa 60
gtactacgac agtttgatcg aagaggccaa ccgtcggcgg tgccccaagt cgaagaagga 120
gcttagcgat cccagcatct gtctcttctg tggagatatt ttctgctcgc aggcagtatg 180
ctgcatggag aacaagctgg gcggatgcaa ccagcacgtt caaaagtagg tccattccca 240
cctctttggg gagtatctgg agaactcttc taacaacgaa tagatgtggc aagaatatcg 300
gtttgttcat caacatccgc aagtgcactg tgctctactt gcacaaccac aatggatcat 360
ggcactacgc accgtacctt gaccgacatg gcgaggtcga cccgggtctc cggcgcaacc 420
gccagctgat cctcaaccag aagcgctacg accgacttct tcgggatgtg tggttgtcgc 480
acagcatccc ggccaccatc agtcggaagc tggaggcgga catcaacaac ggcgggtggg 540
agacgatcta gacattggtc cctgggagtt attgtgttcc gtgtttcagc attgttgtgt 600
atcctgattt ggcttgatga cccgtgccaa aagctcatga tgactttgca tgcattttat 660
cgaactgttt gttcttgaaa ataatttaca tatatgtatc tacgatgcag agggcccttg 720
ttcttgttga tggcggttga tactatatag tactctatgc gttgtgcttg gcgagattca 780
ctgatgctgt cggtaatggt tgtttgacag tgtgaatctt gatctgaatc gtgatttatc 840
ctatcctgca cactgaacta ggtattgatc tacatgtaga cctcgattcg aaaccagatc 900
agccgtgtga ctcacatgga gcgggtgtct cgcaaggata actttagccc tggtactagc 960
ccccatacgc actgtcatta gtgaggtgct gagacaattt aagctttcgc ttcagccata 1020
gcttataaac agtaagatta tgagaggggg aaaagcttca accaagacta ttttcacccg 1080
tctttaggtt gttcataatg ggctacggct tcatggctct catgcctcca tcgtataatc 1140
gtcccttgta gacagtagcc agctacttac tagttagcac agtggacaca agccagcgaa 1200
tcggggtgaa gccaattagc attggatatc cacgaagtat gatcgtcacc ctccatgact 1260
tcacacggtt gctgcatcgg ttcaccgggt tgaacctagc cgggcaggat cggagcaagt 1320
cgctggatcc ctcacggatc acagagctcc agctccacga agaaaagcag gatgcgatcc 1380
ccgcacatcg gagaattgca taccgttgaa gcaatccaag caattggcag gtgtcatctc 1440

cattacgtac cgtgaccttc ataccctccg tatttatacc tgcccctccc tcccccgctt 1500
ctcctgcttt cttcctctct ctttctctct tccatcaact caacttccat ctacttcact 1560
acttcctgtc aagtcttcca tcgactttca tcacaagtca tcctctccca tgtttgtatt 1620
ccctgtatag accgtactga cctcttccct agaaaccctc gtgcaatgta attccacctg 1680
cgtcggcccc tccccggggt ctcgtctcaa tacttcatta cacacgatgg aagtcatgca 1740
atgcctttgg ctggactctc tcaatgatca ggtatctcag gtaaatcttg ggcgtggacc 1800
ggtgttcgtt cctatccgtt ggttgtgcag cattgctagc attgctgcct gccatcggct 1860
ctgggttcgt tctgagatta tacggttaaa cttgatctgg ataataccag cgaaaggatc 1920
atgccttccc tcgtttcccc ccacttgatg gaatggctaa caattcccag tcccacctac 1980
cacaccctta aagcaacatg 2000

<210> 14
<211> 2000
<212> DNA
<213> Aspergillus niger

<400> 14
ctcagtcaat caactgacgg aattcatgtt caacttcacc cagaagagtc ggcgccagcg 60
gatcaaccag cggaaccgga cagagcgtct gagtgacctc ctggactgga aacgcatggg 120
ccttgagtat gtcaaggctc ggcagttggc actccgtcga ggtatgtgta tttccaagcc 180
actattgatt agattgctca cacgttcatg cagcctatcc ttcgtctttc ggcacgcctg 240
atgatgttta cgacgtaatc ggtggcacgg agcagaagat atcgcgacca ttatccgtac 300
ccggatcccc gcgagatcgt tctggtatga tgactcccgg ggattttgcc tctttgcagg 360
aggtgaaaga aggcctgagt acggaggact atgtcgcatg gcgactgccg taagtccacc 420
accccacccc cttgggtgaa tgaatgacac aaggagcgct gatgctgata aactggctgt 480
ctagaactag cgaggaagag gagccagatg accagtattt cccgctcacc ctgcgcacga 540
agaagaatac cgaccgtccg gcgtcgccgc tcgatcagct gtcggtcaat ggtgacagtt 600
gatcgactgg tgagatagcg cggtgcgaca gtctggaatt ctgaatagcc tgcatattcg 660
aggtaccgtt cttggtggaa tgatcgtgtt cgcgtttact tgctctcact aatcgcccgc 720
gtgtgaaata ctgcttttgg tcacccacct gctattgtta tccaaagcaa gcttcattca 780
gaaaagatgg tagctgattt agcccatcga gctctgatca gcatggcatt tcactttgtt 840
gttccatttt tttcatcttg cattgctcgt ttcctccatt cttgcattgt tttatccctt 900
ctttgcatat gtcatcactg ttgtagccag cgaattgtat cttctgattg gattctaccg 960
cagtaggtcc aggttgagat agtcgaatgc acgatgaata ttgtatccct acagtaacat 1020
accatcacga gctccgtgct cccgtccatc gtatccagcc cgccgacgcg gtaaccgata 1080
attcgtgctg ttgagagcga cgatgcacaa gcagctaggc tgatcatccg atccgaagca 1140
gagccaagca gatccttcct caagagtgtc tagacccgcc agccaggacc gaagacatcc 1200
ggttgttgag actagcctgg ccaaccatat agagttgagt cataataacc ttgtccgttg 1260
tgcttccgag cgggtctcga agcgggggag aggatgagac aaggttcatg atgaggtggt 1320
tactgctgga gaaccggaaa agaacgccag gagcacacca ctccggcgac aggatctcca 1380
atgaggcatc tgcttcgttt tcgtgggggt cctggatgtg tttctcggtg agggaagacg 1440
acaatcgcgg atcctaactt agtagtgggg gtttagtcca gggtctcgct tgaccccgct 1500
gcttcccatt tatgccacgt cttctccctc ctctctcgct tgctttcctt tcccttcatc 1560
ttcccatctt ctcactatct atcttcagtt atattgtttc cgaataactt acttcttctt 1620
ccccaacaac ttcctcgtta accgtccggt acgactcaca atgaggccgc gcacgcagga 1680
tcagactccg ggtcttttcg ctatgggttc agatgggtca aggtatcgta caatagtata 1740
acagtcactg ctcgcgcatg acaggtgttc ggctcgtgct tctgttcctt tccttctggt 1800
ttggacagga gcgcggtcgt tctgagatta tactgtcaaa acttgatcta gataatacta 1860
gcgaaaggac atgcgtggca ctgattgtcc cctactattt gacctacaga agacgagagg 1920
gatctcgcat ccctctctgt tgctgacagt ttccagacct ttgcaattac cctcgacctg 1980
agtgtatttg tgccaaaatg 2000

<210> 15
<211> 2000
<212> DNA

<213>  Aspergillus niger

<400>  15
gaccgacctc ggaaatggat aggttaggaa aattgggggg gaagggccac acaaaaaatt      60
agcaagaaat taggagaaga gtgaaagaat tttgccccgg aatcctctcc ctgatccagg     120
tataagtagc agacggatgt cttcttggga agatgggaag aaaaacgggc tggcaggtga     180
gcaagaggat aaaaattaca caatgggttc ccgaagggat tctcttggtg gaaaggggtg     240
gtggccacga taacaagcaa caacactgca gggaaaaacc gcgagcaagc cagccactag     300
cggctgactg tgtgggatat gctttcgtcc ggttttccag aggccacggg atgatgtcga     360
tgggagggat tgggttggct tgcgccgcgc cgcccacttt tctcccctat ccagcttgcg     420
ccgcgccgac ccccccctga gatttgctcc ttattccccc gagagggatc aatttctctg     480
atgtatccaa aaagctatat aacccgtgca ccacccacgg agacaagcgc gctgaggcat     540
tgggggaagt cgtttttttt ttttctacca tagcgccaga ggtgtgtgga ctaacatttc     600
tgtgctattt gcgagctagc tcagggagct acggatcgtc gcgcgtttcc ccggcgaaag     660
gcgctccgta cggagccggg ttgtcccaaa acggcagata cccaatacga tgagtcggtg     720
aggcatttcc cgagggagca gaccaggagc acagcaacag aacacaattg gggaagcttc     780
cctccttcca gccgacgcgg cgcaaacctg cttgcgtctc agaagtggga attttcttgc      840
tcagaaactc cggtgctggt ggagccgcat gcccgattcg gcccggtttg attgcgaccg     900
agaagctggc catcatatta gctgggcctt ggctgcttcc cattggcttc cgggacaccg     960
ccaagtgggg gtgtaccact ctatcggacc catttggatg cagggaccca cccgcacgtc    1020
tggttttttc ccttctttgc ggtctcccgt tttgctgtga aaattttgca taccgtagct    1080
ggtctctatt gcttgtctct tgtcttgtcc tgtcttttcc ggtctgttgc tgggttaggt    1140
ggcccggtgt cagaagcgga agggagaagg ggacaattta tatgtaagga aagaatgaag    1200
ggagggaccc gtagagacaa gacaagaatg tttttttctc tcctttttgt gacgacacga     1260
gggaaaaaag gaattgaacg gaagggatcg gttcatacaa gtgtaaaata cacacacgac    1320
tacggaataa tcccatcaga tgcagcaatg ggttatctga aggggaagga gatgtgtgag    1380
tgaatgagag agtaagccaa tgctccatcg cggaccagca cggtcaggtg aagaccctga    1440
aaccattggc tgtaccagta gtaactcccc tggttacccc catcccgagt gatcccgaag    1500
ggtgtgtatg tgtgtatgtg tacacagtat gtgtaaggaa gtgtggtaag tgtgtatgtg    1560
cggtggaatg cccactgctt tcccggggga aggaaaaagg atgatgagcc aaaaacgagg    1620
cgccaaacac ggtgtaaggg aaaaagaagg gaaaggataa actagggata acggatgata     1680
ccaaagacag acacaaacag gaaaaacagg aacaatacaa tacaaacaaa cggtgccaaa    1740
acaccaaaca aaaaagtagg tagggctttt ttttctggtc ccaacaaagc gcactaacac    1800
ccgacggggg ggctgggtgg gaaaagggca aaaaaccgcg aaaatttagc gggagagtat    1860
ttatgtcccg gggggccttc tgttgtcact tttcctccag ctttttcctc cagaaaagtt    1920
ctccttcctt ctttcccttc ccaatcccat cattttctag agaaactcct ctctcagaac    1980
caccacaaac catcacaatg                                                2000


<210> 16
<211> 1992
<212> DNA
<213> Aspergillus niger

<400> 16
ctcgaggatt gcctgaacat tgacattcgg cgtccggccg ggaccaccgc ggactcgaag      60
ctgcctgtgc tggtctggat ctttggcgga ggctttgaac ttggttcaaa ggcgatgtat     120
gatggtacaa cgatggtatc atcgtcgata gacaagaaca tgcctatcgt gtttgtagca     180
atgaattatc gcgtgggagg tttcgggttc ttgcccggaa aggagatcct ggaggacggg     240
tccgcgaacc tagggctcct ggaccaacgc cttgccctgc agtgggttgc cgacaacatc     300
gaggcctttg gtggagaccc ggacaaggtg acgatttggg gagaatcagc aggagccatt     360
tccgtttttg atcagatgat cttgtacgac ggaaacatca cttacaagga taagcccttg     420
ttccgggggg ccatcatgga ctccggtagt gttgttcccg cagaccccgt cgatggggtc     480
aagggacagc aagtatatga tgcggtagtg gaatctgcag gctgttcctc ttctaacgac     540
accctagctt gtctgcgtga actagactac accgacttcc tcaatgcggc aaactccgtg     600
ccaggcattt taagctacca ttctgtggcg ttatcatatg tgcctcgacc ggacgggacg      660
gcgttgtcgg catcaccgga cgttttgggc aaagcaggga aatatgctcg ggtcccgttc     720

atcgtgggcg accaagagga tgaggggacc ttattcgcct tgtttcagtc caacattacg 780
acgatcgacg aggtggtcga ctacctggcc tcatacttct tctatgacgc tagccgagag 840
cagcttgaag aactagtggc cctgtaccca gacaccacca cgtacgggtc tccgttcagg 900
acaggcgcgg ccaacaactg gtatccgcaa tttaagcgat tggccgccat tctcggcgac 960
ttggtcttca ccattacccg gcgggcattc ctctcgtatg cagaggaaat ctcccctgat 1020
cttccgaact ggtcgtacct ggcgacctat gactatggca ccccagttct ggggaccttc 1080
cacggaagtg acctgctgca ggtgttctat gggatcaagc caaactatgc agctagttct 1140
agccacacgt actatctgag ctttgtgtat acgctggatc cgaactccaa ccggggggag 1200
tacattgagt ggccgcagtg gaaggaatcg cggcagttga tgaatttcgg agcgaacgac 1260
gccagtctcc ttacggatga tttccgcaac gggacatatg agttcatcct gcagaatacc 1320
gcggcgttcc acatctgatg ccattggcgg aggggtccgg acggtcagga acttagcctt 1380
atgagatgaa tgatggacgt gtctggcctc ggaaaaggat atatggggat catgatagta 1440
ctagccatat taatgaaggg catataccac gcgttggacc tgcgttatag cttcccgtta 1500
gttatagtac catcgttata ccagccaatc aagtcaccac gcacgaccgg ggacggcgaa 1560
tccccgggaa ttgaaagaaa ttgcatccca ggccagtgag gccagcgatt ggccacctct 1620
ccaaggcaca gggccattct gcagcgctgg tggattcatc gcaatttccc ccggcccggc 1680
ccgacaccgc tataggctgg ttctcccaca ccatcggaga ttcgtcgcct aatgtctcgt 1740
ccgttcacaa gctgaagagc ttgaagtggc gagatgtctc tgcaggaatt caagctagat 1800
gctaagcgat attgcatggc aatatgtgtt gatgcatgtg cttcttcctt cagcttcccc 1860
tcgtgcagat gaggtttggc tataaattga agtggttggt cggggttccg tgaggggctg 1920
aagtgcttcc tcccttttag acgcaactga gagcctgagc ttcatcccca gcatcattac 1980
accgtcaaaa tg 1992

<210> 17
<211> 1990
<212> DNA
<213> Aspergillus niger

<400> 17
ctcgaggaca acgcatcgtt tgatacactt cccgccaata tggacgttgt ccagaagcct 60
gttcagcatc gatctgggcg tctcgttctg taagcattct cctagttact gatgactttc 120
ctctcttatc tgtattccgt gaaagaggag ggccactgtc ctctatatag tttatggata 180
taaaaagttt gagcttcttg ccaatatgaa acagatttcc ccacattaag agctgtttct 240
ctataggttt ccaatcaata ttagtgccgt caaaacgttt gttcagatca gattgtccac 300
gttcgtttac agatactctg actgtagtat catctgatct cacacgttgg ttgtgacgta 360
tttttcgacg cataacattt tcagcatcct gtgttatctt cgcccagtgt gaactgggtg 420
ctacagccaa gtcctgttca gtgtcctttg acacagttcg gttgttcaga gttaccttcc 480
actcaatagt ataatgaata caaggctttc ctctatgttg cctcgtagtc ctttcttcgg 540
gctcctggaa gaaacccaga tgattgggct gggattgatg caagggagta taaggttcat 600
caagtacatg ttcaggtgat gggcaaaata cggatggcgt acgatctcta ccgaagtcac 660
caggggtggg ggcatacgat ggagtttgta tccacggatc aggtggctga agctgagagg 720
catcgtcatc gtagtaagga ctaaacgtca tcccctcaag gcagtagatg ccactgagaa 780
gcctagtgtt gggatcatca tatgttagcc tacaccatat gggtgtccca gcaagagtgt 840
ccgtgaggga agaggtgcag ctaacaaaac cagtaaaatg atcaggttca tggacaatga 900
actaagacag gtacagtatt gtagccctac ccgtcttggt taacctggta aggtcaaaaa 960
ggatcgaacc gtggctcagt acaaacaaaa ggaatgttaa cagtttgcgg gagatgcaag 1020
gcacatgctt tgtcatgttt gacgcgtttg cagtgtagaa gcttccagct accgtagatt 1080
actgatacaa actcaataca ctatttctat aaccttactg ttcaatacag tacgatcaaa 1140
atttccggaa tattaatgtt acggttacct tccatatgta gactagcgca cttggcatta 1200
gggttcgaaa tacgatcaaa gagtattggg gggggtgaca gcagtaatga ctccaactgt 1260
aaatcggctt ctaggcgcgc tccatctaaa tgttctggct gtggtgtaca ggggcataaa 1320
attacgcact acccgaatcg atagaactac tcatttttat atagaagtca gaattcatgg 1380
tgttttgatc attttaaatt tttatatggc gggtggtggg caactcgctt gcgcgggcaa 1440
ctcgcttacc gattacgtta gggctgatat ttacgtaaaa atcgtcaagg gatgcaagac 1500
caaagtacta aaaccccgga gtcaacagca tccaagccca agtccttcac ggagaaaccc 1560
cagcgtccac atcacgagcg aaggaccacc tctaggcatc ggacgcacca tccaattaga 1620

agcagcaaag cgaaacagcc caagaaaaag gtcggcccgt cggccttttc tgcaacgctg 1680
atcacgggca gcgatccaac caacaccctc cagagtgact aggggcggaa atttatcggg 1740
attaatttcc actcaaccac aaatcacagt cgtccccggt attgtcctgc agaatgcaat 1800
ttaaactctt ctgcgaatcg cttggattcc ccgcccctgg ccgtagagct taaagtatgt 1860
cccttgtcga tgcgatgtat cacaacatat aaatactagc aagggatgcc atgcttggag 1920
gatagcaacc gacaacatca catcaagctc tcccttctct gaacaataaa ccccacacac 1980
cgtcaaaatg 1990

<210> 18
<211> 1500
<212> DNA
<213> Aspergillus niger

<400> 18
gtcacgttgt ccataattga ataaatatga gcagtccttt gtggcgtgga aacatactta 60
gcatgtagag acaaaccttg gtgcgcggct tcaggcgggc atagttagta tgctacggta 120
ccaccgatct tattgtacca gaaaaagtcc cagccagtcc aatccccatt ctaagccaca 180
tgcatccgtt cgcatgcatc tgacatatca gattcgtcca tctggtgcag tatctaacag 240
aggccagagc atcaccaaca tgggtaccct cagcaataat atgcatgcat tgtgcccccc 300
ctatggagcc gtagctttca agcaattaga cacgcgcccg gccgaatgag atgaaccgtt 360
ggagccatca tcccactcat cccgctccag aaaggagaga aagaaaaaaa aaaaatatga 420
ccgagcgcgt gatgaccggt gaggactccg gtgaattgat ttgggtgacg ggagagaccc 480
aagaggggcc agaataataa gaatggggaa ggcgaaggta ccgcctttgg ggtccagcca 540
cgcgactcca acatggaggg gcactggact aacattattc cagcaccggg atcacgggcc 600
gaaagcggca aggccgcgca ctgcccctct ttttgggtga aagagctggc agtaacttaa 660
ctgtactttc tggagtgaat aatactacta ctatgaaaga ccgcgatggg ccgatagtag 720
tagttacttc cattacatca tctcatccgc ccggttcctc gcctccgcgg cagtctacgg 780
gtaggatcgt agcaaaaacc cgggggatag acccgtcgtc ccgagctgga gttccgtata 840
acctaggtag aaggtatcaa ttgaacccga acaactggca aaacattctc gagatcgtag 900
gagtgagtac ccggcgtgat ggagggggag cacgctcatt ggtccgtacg gcagctgccg 960
agggggagca ggagatccaa atatcgtgag tctcctgctt tgcccggtgt atgaaaccgg 1020
aaaggactgc tggggaactg gggagcggcg caagccggga atcccagctg acaattgacc 1080
catcctcatg ccgtggcaga gcttgaggta gcttttgccc cgtctgtctc cccggtgtgc 1140
gcattcgact gggcgcggca tctgtgcctc ctccaggagc ggaggaccca gtagtaagta 1200
ggcctgacct ggtcgttgcg tcagtccaga ggttccctcc cctacccttt ttctacttcc 1260
cctcccccgc cgctcaactt ttctttccct tttactttct ctctctcttc ctcttcatcc 1320
atcctctctt catcacttcc ctcttccctt catccaattc atcttccaag tgagtcttcc 1380
tccccatctg tccctccatc tttcccatca tcatctcccc tcccagctcc tcccctcctc 1440
tcgtctcctc acgaagcttg actaaccatt accccgccac atagacacat ctaaacaatg 1500

<210> 19
<211> 2000
<212> DNA
<213> Aspergillus niger

<400> 19
ttccaacaac tttaacccaa tcaacatgct acaaatccca attttgacca cacccaagac 60
acctaaacca cacccgaacc taccgttgct ggcacaacag gcgtagatga aaggtggtat 120
ccccatgttt tttagcgggt gggtcccgga cgaaaaggaa agagtttgtc aggtttgccc 180
gccccgtcga acaaacaaaa agatcaagaa ttaaaaataa aaattgaaaa aaaaggagga 240
aaggaagaag aaaatcgaaa gacagaaaga ggaggaccaa ggtggaaaat gaaactagtc 300
acattcgagc agaaacagtg ggtcccggct ggaacaattc ctgtttgggt aatgtaggtc 360
tcgtgtcttc tttggctgca tacgaaaata tgatgccggc acctgaaaag tgtggaagaa 420
tatctggatc ggacagatac actggtcgcg tgtatatctc ttgtatctac tgatatagcg 480
tggctattca gaacatgaag agtggtgata attgaatcaa ttacccctta gcacgcacat 540

aagactacta cctgcgatct tcggacacta ggatgaggga taaaaaggag tacggtctga 600
ggtatataac ctctaggaac aagcatatgc tactgctgtg cccttgccta cgaaatgcag 660
catagccatg tagtgtactc cgtagaaaaa ctaagcttgg acaatagcga ctgatattta 720
accggagaga gtatgataga gaccaaccta tgggatactt catattaccg acgttgcccg 780
tgtttgcgat cgctcccttg gccgccccct tctgtctctc tctctcgctc tctctccgta 840
cctgagaact tccgcccgga gcaagaaaaa cggggaaatc gcggcatgtc agccgaaaga 900
cgccgcacgc acctgatcag tcccggtggc gcgtatgaag gcccagaaac cgtggactcc 960
gtttggatgc tgcagataga taagtagtag cgtagatatt taagtaactg agttttgtga 1020
gttgtcttca aacaatttct acacaggagg ccgatgcaaa tttacaattt cggtcttgca 1080
attgtcgttg tgcacgagta agtcaagtac acagtaggag gctgagaggc aagcgttttg 1140
ccatcggata tccgggacca gatggaaaga aggggcggtt ccccatggat ggagcgcgaa 1200
ggtgtggact ccaagaaaac gctttggttc ggtcaagacg gggaaatggg agaggaatgg 1260
atatcttacg aagccgaatt acggagaaag ggcccaaaca tgtaaattat ggcctgtagg 1320
tcaatcagca aagggccgag cggtgattgc gagtacaccg atcggtagac tacggaaggg 1380
atgaaaaaga caggaaaata gcgagagcta catgccgttt cagaggctat cgaaatgata 1440
ttccaaagta tcaccagtag ccataaccac tataataaag gacgaagatg agagtgcctt 1500
cgttctcttt gaccagaaat tcactcatag tatcaaaggg tatttcccaa taatgtcagc 1560
ggtcggagtt ggttactggc gcgatcggga gatatcggct cttcgttgcc tgggccagca 1620
ttagcgccgg gtccaggttt tttttttgca aatttttttt tcttttcctg gctatgtttt 1680
ttttcgttcc ccctaacaat gggaaggacc tccctactcc gtaccgggcc aaccaatccg 1740
gccaatggaa actgggccgg gacgcccatc gccgccgctg ccactgcaaa ttcaggccag 1800
cgaaaaaccc aagagcgtcc tagcgtctcc gctcgcttct tcccgcttac aagagccctt 1860
cgctcgctat tttttcttcc ctcccttccc ttctctcttc ttttctttcc atcccctttg 1920
aagtgtcctg tttgactggc actatcatcc atctcctctc tttctttcct tagttttcgt 1980
tcatcacagc cgtcaaaatg 2000

<210> 20
<211> 2000
<212> DNA
<213> Aspergillus niger

<400> 20
ttttccagct gcagattcgg agtgcagacg agccgatgac cacattccta aaggtgggtg 60
cgcaccagcg gcttatgcgg ttctcatgca tctggcctga catgctttgc tgattgcggt 120
tgacagtgta ccacgtgtgg tgcccgatgg agagagaact gaagctactc cattattaga 180
gttttggaaa tccctggaga acaatgtgtg ttgttcaaat aatagagggg cgggggataa 240
cgtagaggat gaacgcgaag ggtagaaggg gacacgaaaa aggaaggctg gtgctacggt 300
gtatgattag gacgatgatc tgggccccct gcgattgtga ttgttcccat ttgcgatgat 360
ttgcatctaa attactagag atacccctca atggatgatg acaccaatcc atagcactac 420
tatacaagag tggttaaact acacgcctac tccctattga gggagctaag aaaagtatgt 480
cagattaagc gcaaaagaag aaattttctt cccgcctcgt tgatccaatt tatgattttt 540
tttttttttt ttttttagta cttaattatt ttttgattaa tttttttcgg ttcctatccc 600
ccccctccta caaaatttgc cgccgcgaac atactctacg tactaactat tcatggtaac 660
ctggcaacgg caaataatgg cccgattggg attggccaga tccaggtaag ctaccaagtt 720
agtaggagta tagtactttg taggaaggat cctttgtgtg tgttgtttct ggtacggtac 780
caaatcttga gtcatttttt tttctcctct cttctcttat cctctaataa ccactgtcgg 840
ggtcccaaag aggaaggggt tggtcaaggg tgggaaaaga aaaaagacaa aaatgagaga 900
aaagagaaag gctagatacc taaccgtact gtcaggtcaa gacactgagt gaggggcagt 960
gttgatggca aaaaaagacg tgggcaagaa aaaaagattt tccctcacat gttttgccgc 1020
accagccatc ccactatcaa aaagcgatga tgtttgagat tgtcgggtgt ccacatcttt 1080
tagtgtgaat cgctagtaga atttgggata ttattgagca tcatcccatg atagcgagta 1140
caagccccga gtaaatacca acattgctat gctgctgtgc tgctatctag tttgctacgt 1200
tggtcgttga cctcacaggg atttccacca aaaagtggac cggcgggcgg ccactcggcc 1260
gtgccacagc agcctgagag cggacaaata acaacagccg cctgccgcgg ggttcggttg 1320
caaacatgac caacaggcca ggccatcatc aacccaccgc tgcgttgatg cccaggattt 1380
cagtccaata atccacaatt taccaacgga tagagctagg tgaattagat agacaggagg 1440

gccagaggga ggggaccgag atgaaaaatt ttcgatgaaa gagtggtcaa ggtggggtcg 1500
tagttcggcg ctccgagggc gaggaaccaa ggaaaggcga ggaaaggaca ggctgatcgc 1560
gctgcgttgc tgggctgcaa gcgtgtccag ttgagtctgg aaaaggctcc gccgtgaaga 1620
ttctgcgttg gtcccgcacc tgcgcggtgg gggcattacc cctccatgtc caatgatttc 1680
aagtcaaagc caagggttga agcccgcccg cttagtcgcc ttctcgcttg acccctccat 1740
ataagtattt cccctcctcc ccctcccaca aatttttcct ttccctttcc tccctcgtcc 1800
gcttcagtac gtatatcttc cccccctctc tcttccttct cactcttctc tccttctttc 1860
ttgattcatc ctctctctaa ctgacttctt tgctcagcac ctctacgcgt tctggccgta 1920
gtatctgagc aatttttcta cagacttttt ctatctaatt ccaaaaaaga acttcgagtt 1980
cattcacaac cgtcatcatg 2000

<210> 21
<211> 1500
<212> DNA
<213> Aspergillus niger

<400> 21
accgcgaata caagccggac gtgcagctca agtacgtgga tgagcatggt cgcgcgatga 60
accagaagga agcgttcaag catctcagtc atcaattcca cggcaagggc agtggcaaga 120
tgaagacgga gaagcggttg aagaagatcg aagaggagaa gaagcgcgag gcgatgagtg 180
cactggacag cagtcaacat accggtatga acaacgccat gggagcgaca gcgcgcaaga 240
accgccaggc cggtgtgcgg ctgggctgag tgatgttgct tctctcggac tgttgtagta 300
gatgatgacc ttcaccgaag acctccattt ttggaggcac aataatgatc ctcagcggtt 360
actaggctat gcgatgtttg tacagattat accaatagaa cgtcacatat tattctatct 420
atgtccaata gatcgatccg ttttcggtgg tcgataaagc aagcaagcag cacgcggttt 480
tgtccgatgt tggatgggta gagaggtaag atggactcgg tgctccgaag atactgcagg 540
atgtcctcaa tactccgtcg atggtgtagc agagctattg aatcgggata ggcttggcgt 600
gtgggggaat ttaaccgagg ccgagtgata ttgtccgagg cggtggagtg caaccacggt 660
tctgtgcata gctagctatt caaggattgt tgtaaagcta ccgaggatat caagaacgta 720
gttagcagaa ggggaatgga gtcaatgctg cagatttata tacaagtagt agttgatggt 780
gagatgatgg gaagtggtga ctagcaagtg gtaggggggt gtagattaat taccccaact 840
cctcgtgagg ggaaggccaa cctcagccca tccatggatt ttccctcgat actaaaagag 900
ttcaccgggg agagcgggac gggctcatca tttgtggtgc gatctgtcaa tgagggaatc 960
cacgctccgt gatgatgaca tttgacatct catgttaatc agatagtagt caatcagtta 1020
ggacttagta gagatatata caattctatt caagatgcca ttgaataaat aatatactac 1080
gatggagttg catccaaggg ataatatgtg cagcctgctt cttgcttcct gcttcctgct 1140
tcctgcagct gccagccatg ccatgcaaac cagccaaaca agcaaaaagt cacctgctgg 1200
caatgcggag ggcgtggcca atccgatgcc tcccgcgttt ctccccggaa actccctaca 1260
ggactaactc gactagtcca aaggcagttc cagtgactca aaaaataaaa taactatcgc 1320
cgacctcgtc tctcccgctc gtctcctccc ccgattccag ccttcattca agtacttctt 1380
gccagctccc ttggccccgg ccttttcttc tgatcatctc ctccctggtc tcttggagtg 1440
cttgctcatt tcctcctctt ctttcttctc ttccctgttt tccatcctcc attcaccatg 1500

<210> 22
<211> 1500
<212> DNA
<213> Aspergillus niger

<400> 22
tatctgcaag taatacgaat gcatgatgat gatgtctatg tccatgggtg atagacatgc 60
actgaacctt ccatcaacat cgacggtcga tcccttccct gccgggccag gtgaccaact 120
ctaaccagaa tagactttgg gaccagacca tcagcgtcgc aacctcccag caatcaaacc 180
atttctcgct tgcaattgct actcgactag gagcgggcat atctcgggga ttcatcccta 240
cagggccaat cacacgagca ttaaaacact aaattaagca tcctggtggc gttcagctca 300
aacgaattcg ctctcacaac ccatgtaatt gggatgaaaa agaacgccca gactctcttt 360

tgttgactgc ccaagcctaa agcggctccg gtgagaaaaa aggagtgacc ctgaattcaa 420
ttgttgactg gggaggatgg caatccatgg ttgccaccca cgagcaaagc gtggctgagc 480
ccgctgaatg gccggagctt tgctggaatt ttcggttcgg gtacccagca accccggact 540
cacaggacca acacactgac accagggacg gaatcacctc agggtctgct caatcggtgt 600
gtcaaggaac cgtgtatatt tcctattgtg agctaggaga tttagatccc cagccggaaa 660
tagagacggg caagacaagt gggagatctg gcccgccggc cacgcgagtc tggctggccg 720
ttcccggcct cttttcccac tcttctgtct gcaccagccc gcgtctggcc tcatctccgc 780
gcctcttctc tctagatctc tctggatctc tctggatcca aaacagcccg cttccaaatc 840
actggccgcg atagctcgcg tgtgttatcc cgattctcat ctgaccgacc atgatcgtgg 900
ctgagaccgg tctggggacc cgtggatgat cgggatgatc tcggccaaat gcctcaattt 960
gacttttgtc cgcgccgtca tagtcaatcc tcggcacccc catgctctct ctctctcagc 1020
cagtctagtc gtctacagct tcatgttggc ttggcttgtc tcgaactcgg gcactgagtg 1080
gttccttcgt gtttatcccc cactcatggc gccctcggcc caacgttctt ctccagactc 1140
ggtacgtaca gtcgcagtga gactgcggag tctggggccc cgttctccca cctcgcccga 1200
ttccgtgtcc agtggcgtgc agagaatgac tgattccggc cggtctggtt gtcggtctgc 1260
cgggtctggg gacgaatcta cctgctcgct gccatccccg cgtcccgctc caaatactac 1320
ataaaggcct ccatgtcccg ggtctggggt agaatggatc tctcatcatc ttaccttctc 1380
cggctccatt gactctcacg cactgctaca ttgctcacct aatctaccta atctttgcct 1440
gactccacat cacctacctt tagtcgattg ctgtgcctct ggtatcccaa tcataccatg 1500

<210> 23
<211> 1500
<212> DNA
<213> Aspergillus niger

<400> 23
atattcatct gcgggtgagg agtcaatgag atatattggg agaattgaac tatttgccgg 60
acttgcgcga tatgttgact gtcccagaga gattgtaagc agattggtcc ctggagagga 120
gagtatggcc attttgattg ggagagcaaa actgataaaa ttgtcggtgg aaagcgccat 180
attcaatgat caagtgatct ttcagccgag cagagttcgc catgtacgtt tccgaacggg 240
taagctaatg tacccagtga gcatggcatt ccggcaatga ctgtacggcg gcatccaaaa 300
cacggtaagt ctttctcttg ttgcaacaga aattcatgaa ttcagacaac atccatcccc 360
catttttata acccgtgatg ccgttatacg atcaagtcaa gacaaagttc ccccttctaa 420
atccaaggtc acgcacaagt tgggcaccta cgtttctcct cgtcaagatc tcacacaggg 480
gtgagtggac cttaagtcgt tcttggtaaa tacccctagg agctctttct gggagacttg 540
agtgattatg tatgcgacgt tatggcaacc cccttttctg tagatgcttc ttgcgatggc 600
cctctagctc ggacatcact tactgtaatc gtgcgcaaac agtagtagat caagtagtca 660
ccgggaagat gggtaagatg gcacgcacaa ttgcgccatc attcaacatc accattggcc 720
ccaacagaac aagattgtcg gccgagccat tttcttgcaa ggtagtcggc cgacagctct 780
cgggagggaa gagagtcgaa acgctagcaa gcggcagata cgaacgaaca gagttgggca 840
ctcagcagta attatgtggt agtaggcacc acattgattt ggatgatgat atagatagta 900
atcacaataa ctatctacag aggaagagta gaagcggagg atgaacaaga ggcctggcct 960
cgcacggttc atctcctcct atagtttagg aacagtgccg gaactagagg acggtacatg 1020
agttaaggtc agtggttcgt gccttgggga atcccaatct cgccttggaa ttcaggttcc 1080
ttctctccaa aaagatcccg gagggggagg ggcatttccc aatgcggccg gcgctaaaca 1140
tcccacctgg aatgagctca tgcgggtcca atcagggccg ttctgccccg tgggcggtgg 1200
cagattgcgt gatcgggatc gcctgcccag cggctgtcat tcgttggagg ggcatccaag 1260
ctgtggttcg ggttcaatgt ggatgttgtc cccctctgcc tcgtagctcc atccccgttc 1320
cattcacggg ctcctcgtct cttggttccc cgacctcggg cctcatcggg gtatatttag 1380
tacttccagc ttcctccgtt gcattcatct tcttttgctt cctactcatc cttctcttct 1440
accattctaa ttaaaaaagt ttatacccac ccctctacaa ctcccttcac tttcacaatg 1500

<210> 24
<211> 1500
<212> DNA

<213>  Aspergillus niger

<400>  24
cataattaaa taaagaatag atttcaattc aagtctattt attattttag taattcccat      60
cttgattgat tctcccatca gcttatttaa tttgactaca agttatcaca tgcgactggg     120
gtaattattc ccaattccaa gagatccaag agaagtcccc gacaattact acagacgact     180
ccaagcattc atcagcagaa ggattaaatc taatgggaca gtgatgtgac accgtgactg     240
gttggcaacc aagtgtatcc tgtattcccc cctctatgag aaacctactt agactcaacc     300
aggctcaccg gtgaactccc catggtggtc gcatgatcag accgcccagt gaaccgtgtc     360
cgaccgggac aatcctctaa aagtatggga atcaccgtga ttgccgccca ttcccccacg     420
tcggggctca ccggggaaca tgggggaagg agtgaagaga ggcaacgtgt ctgtgtaagc     480
atggatatgt cctaatttcg gagtaaggat ctccggggag gggaaagaga gatagacatt     540
tgggaagggg aaatagggtg aagatgaggt tgtattggct gagtgcagag gatttgacga     600
tacgagtagg taaaggaaat ccggacatct gataagcacc tcccgtgcag cggggggagc     660
caggccaatg ggaagggaga tgtcaggtga tccgccagtc tgcaggatct cttggcgtgc     720
agggcaaatg cgagtttctc tccagatttc agcaggttct agattgcgac ggcgtattgc     780
ttatccttag taggactccc taatggattc cgagcaagaa aagactgttt ggcgtgtacc     840
aatggctcat agtaccagca agagaagaat tttctctctc gcttcgagaa agcaatcaaa     900
aaaaaatcct atcctaccct accctaccct aatacttcca ttgccacccg attcctcccg     960
atagtagagc gggcgactgc cattggcggg cgggccagcg gattcccgcc gatagataac    1020
gggcagattc tgtgacctca aactatcgac taacagcccg aacttcggcg ccaccgccaa    1080
acccgccccg gaagccggcc tcatttgccg tttggggcgt gccaggaaat gcccgcctgc    1140
agcggagact ccctagtgtg gtctgtgttg cctgtgtcgt ctgtgtagta tactagttac    1200
tagtctacta ctgtacagtg gatggcctga gggggggact ttatgtccga ctccggctgt    1260
tctcctccct ctatccactc taccctcttc cctctcttct gtctttctcc ccgctctcgc    1320
ccctcccctc ctcgaaaaca taaatcggcc tttccccctc gccatcttct tcttcttctc    1380
cctctccttt ctctttcttc ttcagactac ttctctttct ttcatctttt ctctatattc    1440
ctgttttcct agatacccca gttaaaaaag ttctctcaat caatcctccc cttcagaatg    1500

<210>  25
<211>  1500
<212>  DNA
<213>  Aspergillus niger

<400>  25
aaagtcggca cctccaggct acccggaccc cgcggtatga gccacctcca ttaacctgct      60
acgactggcc tgttcatttc ggcccgttgc tcgagccaaa gggggtatca cctcgactgg     120
agctactagt cttttagtcc caggatggct tcctggttta gcaggtggac cctccgcccg     180
agccatgacg cgggtcaggt atactccaga caggaggccg aagccatgtc cttcaagtgc     240
gagagaagcg taggctgaac catgatatgt cacggagcca tcatcacaga tattgcctcg     300
gtatatccgg tagacgactc gaatcattta gagactcttt gcgtgtacgt ggtgtgggca     360
tgccatgagt tgttgggccg gcctgaagga tccatcattg ggaccaaggg catcatccat     420
gcgctacgga gtactttcgg agaatcagca cccctgcaca aagcattgtc aatgtgtttt     480
cttatgtcaa aagctgacag agtctgaggc tcgctgacga tgggattcat gctaatgacg     540
gtccgaaaga gctttcacgt aacactggtg aacatcccac tcgggaagcc gagacttgtg     600
acctacttag tcaaatgaga tgattatcaa agccattaaa tgcctcgctg tcaggggccc     660
tggtaagtgt cttcattaat cgaaacccat cttcattcgt ccccgccttc agtgctcatc     720
attttaggtt tagaagcaag attgagtgcc acctgcttta caaaccagca tgggtagtct     780
gctgttgaaa ttcttcaccg ggagcattct ggggaaggtg caaaaggcgg cgcgaagtgg     840
tcgggtcgcg attgtagtct ggattggagc acaagaatcg tcagagccga agcccgaact     900
gagggggtct cggtcattta tcgggatgag agccaatcag cgtgcgctca tcatctgatc     960
gtctggctgc caggcccctc aggcatcaat acggtactcg gcagtatcca ctcccgtttc    1020
tccggtgcaa caaatcatcg ttggagaatc cccagctccc ccgccaactg gggtcgatgc    1080
ttctccagtt gtcctggttt ctcccatgaa ctcgcttacg ataagctgct gtaccagccc    1140
accagcacaa caatatcttc aatcaggtag gtgcttgttc gttacctgcc ccatcctctc    1200
ctcttcttcg gtcattatga actcaattcg gtcgctagct ttgccgattc tccgcagtcc    1260

ataaaaatat atctgcattt gccccttaca cgtcgggaat tcaccggcgc aatgagcctt 1320
cgggtatggt cgcacagcgt catgtcaata ggaggctgct cctagtggtg atctactagt 1380
cgcctcaaca cagcaatata taaataacaa gagcattcct tgagcacatc tgggtaatag 1440
ctgttccatt ctcatcaagg attacgcgac cgtgcctcga gcctccttaa gcgagccatg 1500

<210> 26
<211> 1500
<212> DNA
<213> Aspergillus niger

<400> 26
ataactaatg gtttgaattg cttactcagt ttctttctct cggacgagca gcgaaaaaga 60
acaggcaagg caggacaaac gacaagcgaa gtattccgat ccgtggatga caagcaccaa 120
gacaggaccc atgcagtgcg ttcttttttt tcccctctga aggatttgcc cccattgcca 180
ttgccatggg gggaagttct ttgatccaca ccagtcaaat cagtcagtga ggatggatcg 240
aaaagactgg ttccgatctc aagtctggaa cggtgcaggg cttgtgtgca cacacggctt 300
attactaagg gcagttatta ccacgcgtcg ggacaggggg agatgtattt ccacggcctc 360
cgaaggttgg tacacaactt tgactttttc gggcccagag aagggcgaag ttggggtttt 420
gtggagtgca gtcaatgaat gaatggatgg atgggtaaaa gaaaggaacg gaattgggtc 480
gctgccatgc tgggtgtgct gtggttaggg gggggaaagg gaggattatt gagtgcctat 540
ttttgggatc aatatgactg actatggtca tggctaggac tatagatttg gtggtgtaag 600
atttcattgt tcatattgga tgggattggc atggaatgaa agaactgaaa tgaggggaga 660
gagattctag acaagtatct attatgtatc tagtggatgg atcgaggtct agacgtagtg 720
agtatactaa gtggactagg gacaaggtaa ctatgtagta ggtagtgatg agtgtattcc 780
agaagtactg actgcaggcg ctgtcagcac ccacacgcac acgcacacac actaaatgca 840
ccacttccac aataccctaa tggagtgact acgatggatg gcagatgatg gcagatggca 900
gtggattccg tctcgtggcg tctccaggaa cttcttctcc acctgcgccc aacttccagc 960
acaacttgga aaaggcgaaa aagaaaagaa aaagaaaaaa agaaaaatgc aattcaatgg 1020
ctcgccaggg acactccaat ccacgtcagt ggccatgtgg accccgaagc caaacaaact 1080
ttttacccct gcgcctctgg cggcgattcg ttgaaacggc caggaacagc aggaacagca 1140
gtatgagcac tagccgatct cttcgccgac ggttggatgc cttgccgccg caaaaagcta 1200
tataaggatg catctcgctg caccttttgc ttcctcttca atccatcagc aaatccactg 1260
acactctctt gatcagcttt gtctgacctg tcattctctc tctttttttg atacatctga 1320
ttttagtctg tcgatcagac tagtctgcct tccttttagt cctctctctt ttggacttta 1380
ctactacaca tacaacaaac cactttctct tttttcagag gaaccaacaa cagtctttct 1440
tgacaaccca aaatctcatc ttttttgagt caatctctta aaacaaatct catcaaaatg 1500

<210> 27
<211> 1500
<212> DNA
<213> Aspergillus niger

<400> 27
cagcgcatgt ctgcagcgtg cgggcaagtc cgcgagcgtc tctggggtcg cttgcgcccg 60
cgcgccttca cactgcacga tgcgtatgac gaatacaggg gacctcctct ctacataaac 120
ggtacatagt gtctctacgg ttgcacgaat gggaatttct gcttgttgga aaaaatgaat 180
cctcctggtg gatggcgagg ggatgcgatc ggtctggtgg actggcgcgc gcctcgtgac 240
cggcaggggg aaattgtctg gcgtggggtc gggggattgt gattctggga cggggtcatc 300
ttgggatgtt ttcttttctt ttcttttctt ttcttttttt tattcctcct attatttgct 360
gcgatttcaa attcttcttc actgtgtatg tgtaactagg actatgggag ttgttgatca 420
accgatattg aatgtttcga tttggattga agtggagtct agatcaattg gtttggttca 480
gtatttagtt agtaatggac gggtgctcgc tggaagcaga agaggagctc agcccagtcc 540
ccttcagtcc acttcagtcc acttccagat ccgttcagct tagcttccgt gtggccgaac 600
agcagataac cgatacgggc cgagactagt ccgtcggcgc attcctggac gaagctcggt 660
gttgcctgct tcttctcaat tgaagtgcat ttatcgcaat gatatagggg gggaagggta 720

tataattgat gtcaattcaa tcgtccctgt cagtagagtc atctttctcc agggagaaca 780
agaaacggtt gagttcaaag tgacgtggga gaggcaacgc tggacgaagc agaactatgt 840
ttggccgagt gtttgccggc gtgacgtcag catgaatgaa aagggaatcc cttgaagatg 900
cagacaccag atgagagaaa aaaatccaga tgaaacccac cagcaactga gacaggacac 960
tgtcagaccc attcatcgta ctgtagaaga gtttgcgact cagttccagc accattgacc 1020
ctcccttccc cagagggaag tggtccagca gctggcaaga ccttaaatac acccagctgg 1080
atgcaggctg tctagactgc tccgacctca gcatcggagt aatatacacc ctcctgcatg 1140
cccttcctgc agcgctggtc gatcgacagc cactcagcat ccggctatcc ccagcgatcc 1200
cccatagtcc atgcgggatc catattgcag tcgctcctta tccccgaaga cgcgctgctg 1260
ctcgcctcgt ctggaccggg gtaagtggaa cgtggcgggg gttccgtacc gtatttctac 1320
ctcacgacga ccctcgtctg ctttcttccc ccagttcttt ttttctccct cttaatcttc 1380
ctcttcctct tcttctactc ttcattcttc tcttcttttc ccctctccac tcttcttcct 1440
ccctctcctg atcagcctgt ccttccttcc tcttctcatc cctctacctc cctcacaatg 1500

<210> 28
<211> 1500
<212> DNA
<213> Aspergillus niger

<400> 28
ggcctttagg atggatcgcg gccgtcaagc atcaacacca ctagctgact agtgatgctt 60
cacaccttga accaatagtg tatcgactct atccctcaaa atcggctaat cattcgattc 120
tttgcttatt ccgtgcctga cgccattgct ctgctgaaac aaacacaaat cagcgaataa 180
aggcccggct ctcgtcggtg gtcgctgcct acagcccttt ctccgcccga cagtgataat 240
ggtggctttg acgccacgca actcccaaac gtcagacggc acaaaaatat cccacccccg 300
ggaaagggct gggtagaagg aaagggaatc ccttcgaaag ggggatatgc caatttgcca 360
agcagtagaa acctcccttc tatgtagctc gtctacagat tctgcatctt tgctgcagaa 420
cggtacactt gacgagaggt caactatggt tgcggtgtgg ttcttagggc acagaaaata 480
tcagcattac atgaggggaa caagtgcatg gcgcccagac gaggggaatg ccttcgagtc 540
tggactgcag tggggggcgc gctgtgggcg ttcatcccgg ccagatggtg aactaacagc 600
cactaaacta gtgctgccct aaaggcgcgg tctggccgct gccagacggt tggccgacat 660
ggcccatcac agaaccggat tgtcgagggc gattctgtcg agagaaaaga ctgcacaaaa 720
gtacgagata aaaatgatgt tgttgggtag acatacacct ccattaaagt agggggaata 780
agtcggatac atccactgga ccgatcaact gcaggtatcc gcaccgctgc aggaacaacc 840
accgcaaggt tacccccgga cgcttgctgt ccagtcactg ccaaccgcca ggcacacggg 900
ctgaataatg ggcgtcaata tttctctgtc ccactgtccc tgagcgacac acggtacccg 960
cccgatgacg ttccatgggt cggccgcggt gaggatgcag gggggtcagg aacgctccga 1020
cgcaggcaat cagagggggt cgtcgaacaa tggaaaaagc aacgattagt gactagttcg 1080
actttactca tgcaagagca aataagaacc ttcctccttg tggagacctg attggtcgga 1140
accaaattgg cgcctagaaa aagcacccag ccctaacttg gttctgcaac tgccactccc 1200
cgttgttggg cgtctatata accgccctct ttcccctccc tgtctcctct tcgaaactct 1260
tcttcctcac ctagatcttc ctctctcacc tccccagcct ttccttcttt gcacctgtgc 1320
cgtgcacggt cgagccattc cttcattctt tgaacatatt gcctgactcc gagtagtcta 1380
gcatccactc cttgcaagag cactttgaga gaaccggtct tctcatactc aaaagttata 1440
catacacaac acttctctcc gaacaaaacc gaacaaaatt cgcagaacac atacacaatg 1500

<210> 29
<211> 1500
<212> DNA
<213> Aspergillus niger

<400> 29
gtggacttct tccggtcgga aggcttggac tcggcgctgg ttttctcgtt tcgtgtatag 60
atggtttcgg gcactgaagg gtgagagaaa aaaagcaatt agtaaagcta gaccccaaag 120
tgccacagat aaacagagtt aaacttacca aagaagatct gggcaacgaa ttgtgtggcg 180

tacaggatgg ctgcgatgta catggaccac cgccatccga ggctcttatt gtatgcaatc 240
gagccaccag cgacaccgcc cacatacgga ctaataacga gcaaaatgct atttacaccc 300
atccgcgacc ccttctcatg caagaaaaac atatcagaaa tagcagcggg tccgatggac 360
aacccaacgc tgccacctac attcatgagc atgcgacaag ctaaacactg cgcatatgtc 420
tgggatcgag ccaccccgat cgcgctgacc aaggtcatca gattgccaat gagaagtact 480
ggccgtcgac ctattcggtg gctcatcggg atccagaaga aggaggttat tgcgtttaag 540
atggaaggta cagatccgat gtacgtcgcg gttgtctcgg ggacattgaa ttcttcagcc 600
agctccaatg catcaggagc ctaagggatc tattaggaga ggagataaaa agagtaggaa 660
agttcagtag acctacaatc aacgtggccg agaatttgac caggaatgat tcgaacgcga 720
ggaccataag agctgcaatt ttggtgaatt ggctccaatt ctataggaaa tccctctact 780
gtcaggacat cattgatcag tagtttctgt gaggacagcg aacgaaccaa gggatcctct 840
ggatcatcac tgggctgggg gaccagaggg atgccttgtg cggtggtctt gattctagca 900
ttggcctccg ggtcccccga tgacatggac ttttcttgga gaacttccac agattgaagc 960
tcctttacgt ccgccatttt gacaccaagc aagcgctctt gctggataat tggggaagaa 1020
gaagacaggg gggcattctt cagaaattta tcgtcacggc aagcgccgag acgctatcgc 1080
gggggagaat attgacgcgg agctccagag gcaaaatccg gagtcaagtc gcgagtagaa 1140
gtagtagtag taatagataa gagatcttcg gaagctgtca gaactgaaaa gcgaacgtgc 1200
agactggacg gcatgcatgc ttgccgccag ttttgtgcgt gggcttaatc tcgagcccgc 1260
gctatctgga ccggtcgttc acgctaaggc cgtggctgca tatcgccagc aatttgtagc 1320
tcacgccgtc cgttatagtc tgttataccc ctgtcaagct cgaaaatagt cacccacctc 1380
cgagaataca taatcaatcc ctccggaaca caccggctac atgcagaccg caactcctcc 1440
ttctcctcca ttcagccctc ccggaggatc tcccggttga tcaatccgca accaaagatg 1500

<210> 30
<211> 1500
<212> DNA
<213> Aspergillus niger

<400> 30
gagctcctcg aatccggcaa cgtcggcaac aaggtcagcg tacaggtcgt ccgagttcag 60
ggtggaccag aactgggtgt actgggcaga ctcgaggagg gtggagaggc gggcgagctt 120
ctggatcgac tcgacgaagt cggaggtctg ggcggcggcc tgggcctcag cgtcggaggc 180
ctggaagggc tgagtgtggg cggggagaag agcaaggcag agggagaaag cgggagaggg 240
gaagacagtc agggccttga cgaggacgtt ggtgacggtc tccgcctgga gaaggtgggg 300
gttgaactgg tacctgtcag agggcgtgaa ggggttagca tcgctattca ggtatgatgt 360
acatgcgata gaataaaagt tgagatcatg gttttgtttc aggagaattg gagtctcgac 420
atcccccaaa gtagggtgtc aggtaatagg aaacgtgata atgatataag attcttcatc 480
acataaaacc aaaatggttc cccgcgtcgg agctctttat gtttcggctg aaagtgtttt 540
gaactggata gaaaggcgca tgaactcaca acttcagcaa ggccaggttg gcgtagcagt 600
cgaaggtgcg gtcctcgcac tgctgggcga catagtcctg gaagacagtg gtggtctcgg 660
ggttgtagcg gtcgaggcca ttgaggatgg cgtcaatgtt ggccgggcgg gtctcgcact 720
tgtcgaaggt gacacccatc ttgacggaga tacggagaat ggacgagtcg gggggaagaa 780
gaggggaaag cgtgggtgga ggaggaggag agggagggaa gcagaacgag gggataggaa 840
gagagcagcg gaatggatgg actcgaggtt tggttggccg gacttgcttt tggtgggatt 900
tttcgattgt ctcgctgtgc ggctgcccgg ccgtcggagc caaccccaac tcggttaatt 960
tgtccgatgc atctgcattg gcattatttt tattttattc ctttctattc tagtcagtca 1020
gtcagtcagt catcactctt tactaaactt cactcaatac tactctaaat taggcactag 1080
tggctgatct ggtaaatcgt atgatatact ttgtaacttt gagatgaacc aaattccacg 1140
tgcccccgga atcggtctcg ccgggggcaa aggatctccc tgtcccgtgg tgggatcatg 1200
tcggtgctgg tagggatagc ccgtgtcgcc agtgtatttg gttagtaggg actcgccccc 1260
gtgtaggttt accccgtggt aggtatatcc cgcggtaggg attcgccgca tatataagat 1320
gacggggata acaactccaa cacagcagta gcttctgcgc ctgacttcta tacattcaat 1380
aaattgaccg cgtatttccc ttagcctttg tccgaagcac gcagttctga tcgcaaccgg 1440
gatcccctca acaaccctaa cgatatcccc gggctatact atacacctta aatcgcgatg 1500

<210>  31
<211>  1500
<212>  DNA
<213>  Aspergillus niger

<400>  31
tgatcgcgac aatgcattgg ccacgcttga atttgagggt ggtaagatgg ctgctttgta      60
ctgcacgcgc atgatggcgg cgggacagga ggatattact gaacttatct gtgaaagggg     120
ttcaattcag gtgaattttc agccgcgaaa gaatcatgtt gagattcatg acgctgcagg     180
cgctagacgg ctgcttccac agcactatta tgagcggttt agggaggctt tcgttactga     240
ggctgctgag tttactgagt gttgtttgga gaataaggct ccaccgatca gccttcggag     300
ctcggttagg gcggtcgctg ttgggcaggc tttgcagcgc agtttgatta gtggagagaa     360
gatatttttc gagggtgaga ccttaaagaa ctagatagaa tgaaagtgac actcacgatt     420
caagttgttt tgttgaggtg tactgagccg acctgattat ggataatgat gatcgaatat     480
gacaaatagg tactgagaga tagttacgag gtccgtagag tcaaacgaag cagtatggct     540
cattgaacaa ctagaaacaa gacttatatt aatacttgat aatagtagaa aacaggaacc     600
agggatatac tctagagatt acagaaataa tcaagctata aacagaagaa ggtatcctgc     660
ctttttacta tattataaga ttatttccag aaagaggatt aggacttatt ctattatata     720
atttatcttg ccgtgaataa tcttgaaaat atattagata aaatcagaaa taaactgagc     780
atgcctatat tttataataa actcagttag gatagagcaa catactccgt atgcctctat     840
aatattcagt gcatgattaa ataatttata gaagtaactg ttgcatgtgc aagaccacgc     900
tgactaagtg gattagtgga ttataacgta acccacggtt aaacggccca cacggttagg     960
cggcccagct ggtaatacta tacaaaagac cctttttgaa cacaagatat ctcaaccatt    1020
ataagactag aagactcaat attgtataaa tagattataa attatataaa taactatcca    1080
atacatctca aaaatctata taaatatgaa tagtatctta tcatattaca aaatctgtaa    1140
tttatctgta attttggata tattgatata tttttcataa aagaaattct taaaaatcta    1200
gaaaaattat atattattta ttctactgta tattatctat atattaagtt agaactgatt    1260
atattcagta ttggttgaga tatgaagatt taaaaaaaca tcttttatat gggataacca    1320
gatgggccgc ctaaccgtgt gggccgctta accgtgggtt acgttatgat aatctctcat    1380
ggaagttcct ggaggtaaag aatgttttgt ttccttgtgg cctatcttta agatatttta    1440
gtcaagtgta gcacagttct ttgtattctt gactgttgtc gagcgcagtg ctttcacatg    1500

<210>  32
<211>  1500
<212>  DNA
<213>  Aspergillus niger

<400>  32
aatctacctg acaaatatgt ttaaatcctt gctaaagcag aacatgtgag attttcaaat      60
tatttattat ggaagatttc aaggcccaat ttgacaggtt ttatgtactg ataaacaatc     120
acagcttgaa ttgacactga actccaataa atgtgacaca tagtctgaac agcagatgct     180
ggcagaaaaa ttatacaatg tacatatgca atgcaggatt gaatcgcttt aaccaatgaa     240
caatgcattt atgacaaatc aacgccaaga taccctcgct ccagtacgta atgcaggtat     300
taacacatcc ttctccccaa acccttagga ggtgaaccac atgaatctgg gataagcaga     360
attagcggaa atcgtaaagc tcaagtcaag cagataccta catctcatag gccatggagg     420
gaatatcttt gcaaagtgag acaccagatc gaataaggga tatggttgca cccgtcagca     480
cgaggatggc caatggacga aacgccagaa accaggcata tctccacacg acccctatgg     540
cggatctgtt taaaactggt cagcacgcta gttgaattta gttgaccatt gtgctgcagc     600
ttacatgaga gtggaggtgg aagacatctc gtttagtgat gggcgttttt ttctctcaac     660
gaacaaagag tccttgtggt actattgagg aaaggttttg aaacttcaag gatgagcaaa     720
tcgattcaga catgatcctt gaaagtggga aacaagccaa ggctcttatg tcaaggttat     780
atcccatttg cttgcgccat tgccaaggtg gcagcctgct atcgtcattc ccaacgggtc     840
tcgtccgagc cacagcagcg ggggtaaacg ttgcaccgcg ccaaagacat tggatcttta     900
gatagaatcc tctaatccca ttcaggaaag cctgtggtaa aaatgctgcc tgaacccgat     960
aatcctacca aatcctgcct tccgactttc cgaaagttgt ccacggggtc ctcgggagct    1020
ggccaaccag aagagctggc tgggtcaatt cgcgccagat gcgaagagtg actgggcagg    1080

gcagccggaa gactttgctg ggggtctcca ctattggctc ctcgggaggg gaaaggggcc 1140
cgctccctca gccctacaag gctgccgctg cacttttgtg aaatatcagt tgacccctgg 1200
ccttgcgcaa cgtggatgtc ctttttctta ctgctttctc tttaaatcca aagtcactcg 1260
ctctctgtct accttggccc agccgtttac ggatgaaaac cgtgtcgacg atctatgacg 1320
ttttgcttgt tcgatcagtc aacaagcgtt tatatcatag aatgggctga gtgtacgcct 1380
ttcctgccct tctctggctt tgtgcgtttt cactgtggtt agatacgctt gtgtgtgaat 1440
aatttttact gtcgctcttt cgctccctga ggtggctgct cagtctggat tcacaagatg 1500

<210> 33
<211> 1500
<212> DNA
<213> Aspergillus niger

<400> 33
cacggctaag gctggtgtcg tcagcccgga agaggagcaa ctgcgcttct gggtccgggg 60
agagaagggc agcttcaaga aggtgagaca acggcctttc accctaggat gtcggttagt 120
aacaaaatac tgatgcgtac acgctcgata gttccatctc gacgtgcagg aagagcagct 180
caaggctggt gtgaagccag gtgacagcgg atatggccga gagccttctg agagatacgg 240
taagtcaagg attgccgccg ttggactttc gcaacaggct aacagtctct gttaggtact 300
ctcacgacta ttcaggacgg caagcccgtc aaggaagtca tgcccacagt agagcccccg 360
acctggacgg aatactaccg caagctggcg cgggctcttg gtggagaggg cgacctccct 420
gccagcggcg ccgaggctcg tgaagtgatc cggctgatcg agttggctca ggagagctcg 480
aaacagggaa agaccctgga tgtataagtc tcgtgtaaag agtgtgccta tcatgggtca 540
tgatttaatg atggatagaa ttgttttcgc attgtacgag tggtagtgtg aagtttttag 600
attgtttctc tttgaaggaa tagatacagc agacctgctc aatgccacta acatattagt 660
gtgctccacc tgctgcaggc gagtgttcga ctgttgtacg taggctctag ttactccaag 720
gacagacatc cctatacagt ctggtcccaa cagcgtcccc aaatcccccc tcatttcccc 780
tcacgacggg ctttacgagt gagaggcgcg agggttcggg gggcgccatg tttttgggga 840
accccagatt gatcgacgat tgctcaagtc tgggcgggaa tgaggttagt tacgcggtct 900
gaaatcccga taccagcagg agcccatgaa acaggggtat ttggggggaa aagacagccg 960
ggttcgggta acagtcggcg taatattaca ggccagtgac ctcatcgtca gctgccccga 1020
cgctaaaccg gaagcagagt ttccattacc caacgcgaat atcatgagtg gcagaaagat 1080
gtcttggacc agccagcaaa agggcgtgtt tcccttcttt ccagggcatc aatggccatc 1140
tccaactcag cgctgcattc ctgccctctg cccgctctct tccgcggaga tcctcgtggt 1200
tacaatgtat gagtcctcgt tttgcgtgcc gacttttcca ccgtcttctc agtgctggcc 1260
ggatggcccc attctagtat tttaatctcg aggaacccca taatggtcct gtccactcgc 1320
tcttcgtctg tttcagacct ccaatgggat tctttggttt atatttagct tctctctccc 1380
ctcctgtcaa tccacgtagt tcaaggtagg ttctgtcctc gcgggccccc ctgatatgga 1440
cccgtcgggg ctccatttaa tcaaactgat gcctggtcgc ttgatcggtg tcaatgcatg 1500

<210> 34
<211> 1500
<212> DNA
<213> Aspergillus niger

<400> 34
ttttccaatt ttagcccttg accaccaatc acgttggttt catgcggtat ttttcacact 60
tgcctcattc gcactaagat ggtccgcgat gccgaaaaag gaaactgaga cccactcaac 120
ttggcatcgt gaatcgagtc atccgccgtg gagccgttgc acaggcgggc cagtatcaat 180
gagggaaagg gaaagggaaa aagagggaaa agaagggaac atacatggga ataaggcaag 240
gagaaaatag atttttcatg gatcaaaggt agcactttat atacacagtt tgattacccc 300
tcgtacaagc cgcatcgaag ctgtactacg tagtatctag ccaccgtggt atcttgtgta 360
ggaacaaaac atcaacttga cagttggaga caacaacggc aacgtggaga cccatgcaca 420
agggcgggcc atccatgatc ctcaggcaga aacagccagg ccgtccaacg acttgccccg 480
gaccgggccc cgttcctggc cgatcgcata gcaccggctt tctgggtttg gctggctggc 540

cgatagtatt tggcttccca aagaagtagt attgcaggta tgacttaata atggctgtgc 600
cattcggatt aatctcattt tgggaaattt cgatttttat ttttcatttt tttttttttc 660
cccttcgatg tgataataag aaatgtttat tatttaatta ttattatttg ttttaccttt 720
ggggaaatac gattcctcgt ctggcttcag ctccagtttg tataaagagc cagcatctcc 780
cgggtggatt tgcacctgat tccactgctt tgttcgtgtc catcacattg aatatcctcc 840
ctgtgattta atttgacctg atctgatttg aattcgcatc cttctttcct tcttggataa 900
attgggattc tctccgctat ttttccctct tctttcccac cttcccctct tcatctgtgt 960
ttcttccatc tttgaatccc aaactctccc aaactagcct ctttgtgcat tccattggtg 1020
acgtgtcggt tgatggctgg taccgacaag tgaaccccgt ttcagtctgt ttggctcgtt 1080
tctcttgctc tccccgataa gcttcgagct ttcgtcattg ccctggcccg tagcaacgcc 1140
agatcaccaa ttttcctttg catcaatcga cgcttccatt gaggtaggtt tgtcgctagt 1200
ggtctagaat tcccatactt tcttttctgg cttccttcta tatctccctg cgccggcgcc 1260
atctcctgca catcttcatg cagctgtgtg gctgccttat ctttcccagc ttcagcttcc 1320
catcctcttc ccccctccat tctcgcgcac gaaccagttt cttttattgc tagcagcttg 1380
gctcctgcct tgttactcat gcatggctca ccaagtaaca gatataaacc cactagtgcc 1440
gcctcttttc tgagttcaat tgagcctctc aactcctctt gccactccac tcccatcatg 1500

<210> 35
<211> 1500
<212> DNA
<213> Aspergillus niger

<400> 35
cttggcctcg cgcgatccgc ttggtgagga acggtgttat tgacttgaag aagcttgtga 60
cacaccggtt cctcttggag gatgcgatca aggccttcga aacggctgcc aaccccaaga 120
cgggagccat caaggttcaa atcatgagct ccgaagacga tgtgaaggct gcttccgctg 180
gtcagaagat ttaaacagtc gtacattcgt gagcacatgc ctccactgct ttatattggg 240
tgactggtca ccttggatac ctttcgcatt catgacacat atttttcatg acattttgat 300
ggatggttta tcgtgtaacg ttcccctttt ttatgacgtt gttagagatc cctgctggtt 360
ggaggcatag agatgcacgt agaattctat tccttcattt ttgactctca caacatctgc 420
atccgacata tcgcaggtag agaagctgtc tctcatcgca ttgacaagcc tttgcataga 480
atgaagcggc agagtaaccc agagctgcag cttgactgaa cactttgtct taacgcatct 540
gcacacttcc cccaggtccc caagcttctg ccagggtacc cggcctgctg gaggagtgaa 600
tatgcacacc tcgagtgcca cattaattag tacgatgtgg tcaatacctt atgccacctc 660
attgcttaat gcttgcagca ctggcagtaa gcgaccgcct cttgacaggt cagagccacc 720
tatcagctac aactacgtat cagcagaccg gatccgttgc atccgacgct ttgtctgact 780
cttgtgcggt ttttgagtga ccagaatatt tactttggtc catgttcttt gagtgagatc 840
tgatcctgat cctttgaatg tctcagtttg tttgtttgcg gggcgtcagg cggggcacgt 900
ggtggggaga gtgaggagag ccaccatcac tgtcacttcc tcgattccat ctccatacta 960
ctattcgcta ccaaaagctt acttgcataa aatttggagc caatcatcca gggcattacc 1020
gagagatata ccgattagac gtgcccagag tctaggctgc tgcacgagat tcaatgaacg 1080
agaatcggtg taagtgacct gatgttattt ggtccctgca tcagccccaa gccgatagcg 1140
gcgaagatcc ccgataattg accgagatgg gacgacctta gactcacatt gtcttcttta 1200
ggcaatcgtc tccacgtttc tcggcttttc tatcctataa atattgcttt ttgtttttcc 1260
tcatagaact gctcggctca tcccgcctct ttgtcagata cattccttgg cttcgctgat 1320
tgaatctgcg gggtccggtc ataccgcgca acgccacatt atgcacttcg gccaacgcgc 1380
catgcattca atgtcatcag tccgtgccca aacacatata agccgctggg accacccagc 1440
tgggatatga agtcacggct tgctgtaatc cggggtgatc ccagagccaa catcataatg 1500

<210> 36
<211> 1500
<212> DNA
<213> Aspergillus niger

<400> 36

tcaggtcttc ctgaagttta tctcttcttt cgcggctact gacgtcagtc tccttgcgaa 60
gcgactcgag ctcaatttgg atggtggtaa tatcgcggtc gagctctctg atcacatcgg 120
gcttcgattc ttgctgtagt cgcaaggcgg acgccgcctc gtctaccaga tcaatcgcct 180
tatcgggaag gaaccgatcc gtgatgtacc ggttgctgta agttgccgcc gccaccaaag 240
caccatccgt gattcggaca ccatggtgca cttcatactt gttcttgata ccacgcaaga 300
tggatatagt agcagcaacc gagggctccc cgacttggat aggctggaag cgtctagcca 360
aagccacgtc cttctcaatc aacctgtact cgttcaaagt cgtggcaccg caacattgca 420
gctcaccgcg agacaaagcc ggtttcagaa ggttgctggc gtcgatactg ccttccgcct 480
ttcccaagcc aagtaaggta tgaagctcat caatgaagag gattacaccc ccttgcgcat 540
cctcgacctc cttaagaaca gatttcaacc gctcttcaaa atcacctctg aacttagcac 600
cagcaatgag agaacccaga tccagcgcaa taacacgttt gtccttgatg ctctctggaa 660
cgtctccctg aacaatcctc tgtgccaaac cctccaagac cgcagttttt ccggtacccg 720
cagctccaat cagaacgggg ttgtttttcg ttcgtctgga gagaacctga atggtgcgat 780
ggatttcggc atcacgtcca atgaccgggt ccagcttgcc tgccttagcc ctggctgtaa 840
ggtcgacacc gaactgttcc aaggctgact tttctggttc accaccaagg ttcatgcgat 900
gtgtgcctcc gggaggatga gggcggccat tggcataagt gcgtataggc aagacggacg 960
ggtgccgggc gagattcgcg atccgggtgc gcgcaagtag acgcaatgct tgagaaggtt 1020
gaagaacgcg gttcgatgac ggtagggagg atggccggag aagccgaccc cgagcgagtt 1080
gtaaccttga cgacatggtc aaaacaccga ccgtatgcac agttgggtgt gaaaacaaat 1140
ggcacaacac tgcagatctt cagtcgagct cccgatatag ctgggggaaa cctatatacc 1200
tcgccgggac aggcggccag ccgattgcct ctcaggcgtc gcgaggcttc tcgaaggctc 1260
caccgcacgg cggacggtga gtggcggaag ctcccccaag agggccagct agctccgtca 1320
ccgtggggtc acatgaccgg ccggaatgag gggctcactt ggtgccgccg aaaaatttca 1380
ctactcggac cctccgctga aaaaatactt cagaacacct ttcccctcct atttattcct 1440
ccttttcttc ttcttttctt ctactcacca cacacatccc ccaaacacac attcacaatg 1500

<210> 37
<211> 1500
<212> DNA
<213> Aspergillus niger

<400> 37
cagccttgtg ggtccggcat cacaggtcca ttgttgagtc acgaagccta ctctgtacaa 60
gccatgccat ctgaaagaaa agacagagag ggagggatgg ataaccacgg aaagggagga 120
gcaggtgact gctgaaatcc gaggagggga gggatgatct catcctgccg accgttccgg 180
gatgcgagga tctgcagagt tgcaaagcac ggcggaagat ttagtctcac tgtagctcga 240
agcttctcga gagagggagg gagtgcattg actgattatt gatcccgttg gaacccgccg 300
ggttgcacga tgggacgtgg cgtggtcgcc attggtctgt gccgaagctg gtggaagctg 360
cttgatgacc agcgctgagt acggtgctga gtcacggtga aatcatagtt agttaacatt 420
cctggagata attattcagt atgagtgaaa agactagatg cttctgatga tggaatagat 480
gatagagatg ggatggacag tgagtagtga tgatggaaaa ttgtagaaag gtagccagaa 540
ttgaagaatg aagataagac cgaaggaaat ccgacgagat ccagaccggg cgatcccccg 600
tgacgccaag ccatgacgat ggaacttgag tggcttcacg gcttcaattc tggcttcttc 660
caccctccaa tccttcaatc accgtccagt cacgcaacct ggcaatcatg agttgaccaa 720
caacaccctc tctcccttgc ctggtccgtt cccgactctg tccatctggc tccatccatc 780
agtccatggt ccacgctgat cctcacacta gccatgccgg acctgatccc caccccaact 840
gtgatgcaga cccagctgcg ttgttgcggt tgttgatgtt gatgctgctg tgatggccat 900
tgccttccac ctcggttggg cacccctccc atcctaggaa cagtatgatg ctgtccccta 960
ccttagtacc cctctgttga tgtggtcttc ctgcacaact acggcaatct ggtcagtgca 1020
atcacacaac cgacacaaca actggtccgc ctcggaactg acttacttca tgcagaggaa 1080
attatgacgc atctggggaa aagcgccaaa aaacccgcct aagctggccc tccttccgtt 1140
ggcgtccctc agttttccct ggacaggcca ctactactga gggccagaaa aactttcggc 1200
tcaagcttaa gggggaaaaa aaaaaaatac aacaacctct gacgtaaaag atacgccccg 1260
ttaattcggt cggccatgcg cccttccgtc ctatccattc aagccgacca ggcgacatat 1320
aaagaacctt gtcctggcat tccttcccct cctcatcatc cattcatcag agtccgacga 1380
accagtctgt cagtggaact gagcttctct ttgcgcgtcg cccaataaac tactagctgc 1440

cctactatct cactttcttc ccatctctat acactttcag tccagtcaat cgcagccatg 1500

<210> 38
<211> 1500
<212> DNA
<213> Aspergillus niger

<400> 38
ttggatggag gctgaccatg ctgcagagat ggatgaactt ttaccgattt aatgacggtg 60
tttaattggt cacattggac aattgcaatg attgatttaa tctaaacata attcttatga 120
cagacatgac atgagccttc catctgctta ctcccatcca tcactctgct ttctgctctc 180
atctactccg tagtagaaat atcaaacgta caatgacaga tcctaaaccc ttggtcagct 240
accacacttc cagtgcagaa cgatacctac tgctgatcat cacagaatcc tcctgtccac 300
ccaccaccct gttcaatgca ctaacccttg aattagtctc tctccatgac actggacaaa 360
agcaacaagc agcctaccgt attccgacag gcaagattgg caatgctatc cccgccacgg 420
atctggcacg aggtggaatt gctcagcatg atttccatga gggcagccag ggcgggcaga 480
cccaagccga ggcaggagta gagggtgact agggcggttt tcatcgtggt gtagatagct 540
tctagttggt ggctaagaac tagtggatgt tggcggtggt ggatgtctac cagagagtga 600
gagccccttg ctacgatagg cctactaggt aagttctggg agggaccttg gttgactggc 660
tagcaggatg gctgcattct gtgagggatc tgcagagggg attacttgga ttgaagctcg 720
tgcatggctg cggatggtcc ggtgctacac agcacgatct gctatcgata tggcgaggga 780
tactcgggat ttgacttgtt gatctttcaa tctttggatg ccttaattgc tcgtgcatta 840
tcttcatgcc caaaagactc tgttcctgtt tattcgatca aagtcactag agttatggct 900
gcttcgacgt ggcatccctt gctgacgggg ccttaggtaa gtaaagaggg gagggggcca 960
gatgcacctt gaggaatggg tgggaggaat ctcattctcc gaaatccgat ggtcggcaaa 1020
cccccaagat tggggcagag tgggatccgc cggtcgcgat ggataccgga aaacaatagt 1080
catcctccag actgcggtta gtgaatagtt gactatggag gagatggtta gattagttag 1140
ttatttacat gggttaagtt ccgagaccga gggggatgga ccaacagcag caatcaggag 1200
aggcaccgtc agcacgccgc ccggaagcac agaaagtccg accgcatcag ccccgccggc 1260
cgagctttgc tccgtttgaa tccttcactc tcttcccctc tcattctctc tccgcccttt 1320
tatttctctt cttccccctc tacttttatt cccttccact ctccatcttt cctcacttat 1380
cctcctcccc ccttttctct gtaagccatc tctctctctc tatttttctc actcttctca 1440
ctgtcagttt ggctgtgtct aaccattgca tctctagtat cttcaccaca cacaaccatg 1500

<210> 39
<211> 1500
<212> DNA
<213> Aspergillus niger

<400> 39
ttttcggtag aatccacttc gtcttcagga agcacttcga ggaatcttcg tgtcagcgtc 60
tgtcataggt tctcattgga agagacatga cttgattgta tggcacattc tcgcccgcta 120
ttttgaagga tgggtagccc ttgcatgatg ctgatggttg cacatgataa gcatgtgtct 180
atggttacat ctgtctgttg cctcttaaag aggggttgaa ggtcaataag gaacagacaa 240
ccgacgccac tggattgggg aagtcatacg acgctaccac ctgtgacacg cggctccaag 300
acgtagaagt caaggttcga tatgtgaggg gctattagtg attcttcact aacttcatag 360
gtgacacttc aatgtccaat catggcaacc acgggatttc gacagtcata ataccaaacg 420
gttggtggaa ggacatgctg gacagagtaa ccgtaaagga gtcttaactg ggctgcgtga 480
tgtgaaccga gtcaagtctt tcattgaacc aagatgccat acgtggaggt cacccgccaa 540
cagattcttc gaccaggcta gcattgtacg tctcaggata atgcagtcag gatcctcgcg 600
taagcgggcg tatccttatg ctcgtgataa ccttctacga ccatctgcaa ggggtaggac 660
gcaccccgct cttcgccagc ggggttggaa tgcatcaaag gttgtttctg ttgccctcgc 720
atagtgcttt tgtccgaaat atttcggtat gactcacatg aagagtatgg tgaggctgtg 780
tatttggcga gatgaaagta ctgactagtg aacgggtagt gaaaatgtaa aggactgcaa 840
atggatgtca aatacttgcc atagatgtgt taagatcttg cttggaatgt ccataccacc 900
ttatacatat tcatttgcaa gttgctatat catgctaaac agcctgattc acgcttgcca 960

tgggtgtatg tctgtccttc agactcttcc aaaacatcta ccgtgataat agactagtct 1020
atattcgttc tgcaagagat caagattttg aaggtagcac aaggtagggt ttgcaaatag 1080
tgtctgtagg ccatcctgca tgaagagcgc ttcagagcaa tctagacctt cttcgtagtc 1140
ttaaaaacct ccgagcatag tcgaagttga gtcagttgac aaaatacatg gcaatagcca 1200
acgcaagtat aggctgcacc aagccaatgt tttgcaggtt atcctccaca tcccgcctgt 1260
aatgagaggc tagatggtct atcggattac tggatcaaca ttacgtctca agtatgcaaa 1320
tagggcgtat atcccaatta gcaagtcgaa agaattatct aaattgggac cgaactcaag 1380
ggtgaaaggg atatataaac catcgaaagt cccattcttc ctcctgaagt tttctatcat 1440
cccaacacac tgcacattat cacatctcaa gaaaaaaaaa aattcagacc cgtcacaatg 1500

<210> 40
<211> 1500
<212> DNA
<213> Aspergillus niger

<400> 40
aaaccgttgt cgcacaaatt ccatccatcc atgatgagag ctaatcatag cgattaagtt 60
cggacgtgca tttctggaaa catggtggaa tcggtccgtg ggtggtaaac gatgcgcata 120
tcttggggca tgagtctgca ggcctcgtcg tcaaggtcca cccttctgtc acgacactcg 180
cagtgggaga tcgcgtcgcg atcgaacctc acattgtatg caaggcatgc gaaccgtgtc 240
ttaccggacg ctacaatggg tgcaagaatc tgcaattccg ctcaagtcca ccatcgcatg 300
gtctcctgcg gacctacgtg aaccatccag ccatctggtg ccacacaatc ggcgatctat 360
catatcagaa aggcgccttg ttggagccac taagtgtcgc actcaccgca gttactcgat 420
ctggggtcag aattggagat ccggttttga tttgtggcgc gggaccgatt gggttggtgg 480
tgctgcaatg ctgccgcgct gctggggcgt atcccactgt gatcagcgat gttaacccgg 540
cgcggttgca atttgcgcga cagttcgtgc ctgctgctcg aacactgcag gttcggccgg 600
aggagaccga caaggaattt gcagctcggg tggtgcagtt gatgggtggg gaggattgcg 660
agcccgtggt tgccctggaa tgcacgggag tcgaaagttc gatcgccggt gctattcaga 720
gtgtcaagtt tgggggcaaa gtgtttgtgg ttggcgtggg caaggataag cttcagtttc 780
cattcatgcg actgagcgag cgcgagatcg atctgcagtt ccagcagcgc tatgtcaaca 840
tgtggccccg cgccatccgg gtgatgtcca gcggagccat cgatctagac cagatgatca 900
ctcatgtcta tgcctttgaa gacgctctca aggcgtttca gacggcgtca gatccgagta 960
gcggctcaat taaggtgctg attgaggggc cacaaggatg agagatggcg gatattggac 1020
agtgaagcca tgactgttat aaggtatagc agaacagaac atgtatcttg tcaacgaatt 1080
gattgatttc agcacggaaa tgttcaaggc atggaacaat tgcttctgac ccggaaccgc 1140
ggattgcaag gatggatatg tggatcagat ggtgtgaaga agatctgtct gttagcatac 1200
ttcataagtt ccaaggagga gtaaatgtgg agtcataaga gtaatatcga atatcataga 1260
atggggttgt cttgctaacg gtgaatcgga ctccgtcggc tctgccagcg atcggcaggt 1320
cacgcgtctg cgtaacttgg tacttatctt cccagtcacc taagcactgt tactccactc 1380
ttctctcttt gacgataaga agatagcaga gactcttgat aaaaagggtt gggagcatgc 1440
tttgaatttc ttcactccga gagctttctg tccccatctg gtctcttgtt gtccccgatg 1500

<210> 41
<211> 1500
<212> DNA
<213> Aspergillus niger

<400> 41
tttgccctaa ctattctcta tatggagtgc gccctccacc gattatcata taaataatga 60
aagtgaataa atgaaagtga tcaattgatt tataatgggt ccttccattt tatctcgtag 120
cggcaccact aaggatatag gcaacctcat cgctcttttc ggccggcccg aactcctcag 180
ctgggtgacg gtcagtactc gtacaccaat aaattcaatc ctgataaaat cacatagaag 240
ggaaactatc ttcaatcgaa ccagaacgat tgttagcccc gtcttaccag ctagatcaca 300
aactaccatg caagctctgt caggagtaaa tatgcatgac ccatggatct agtctgcagc 360
aaatcaagga caatcaggag tcaaaaattc tccagctcaa acagttgcgc ttctaactct 420

acgaagtgaa ttcaattgag accggtttaa tcctcggcga cggcgtggaa aagtccaatt 480
agcttgcaag gatgaagatg gcatatgcat acgctgagat actaacagaa agaattggca 540
gtaatgatca caaataacat taggaagcga ggtctaaaac agactatctg gcactgaggg 600
aatggctcca gaaagctgaa actgggttgg gtagctgtta atacatgcag atgggatatt 660
tacactctgc tcgacgatgg cgcgccatgt gttcttcccc tatctcagtc ccataacaac 720
ttccagggcg gccgctgaat tcaataaagc cgctaattgc tcagaaatgg agaaggtgct 780
cgggatggat aagttggctc taatgaatga tgagtttgaa tggcaaatga tcgataaaga 840
ggactctttg ctatcagact agctggcacg gaatagatac tcgtctccac tagggttggg 900
aacagtgagc gaaatgtggg ccgctaatgt gccacggcgt ggataaacca atcgtcctac 960
atccccaatg gaagccaaca ccaaaaccgg ctaaacccta caataccata ctgctggaaa 1020
tcaaactata attcacacgg gtaacccagc aagtttaaac aaggatgata ccccgcagac 1080
gacttctccc aggctatcgg aagtgggata gtgtgcgttc gccccaagtg gaacggaagc 1140
cgacatggac ttttcggatt tgcattccgg atcggcaatc caagaagggc aaccgaaacc 1200
ccatcgaaac ctggcagcga aaccatgaca ccttcgaagg catgggcttc cgttctctgg 1260
cggatctggc ggctaatcgg ttgacctgtg tacatggacc acatcggtag taccgacctg 1320
cacaggtgac cgactgatgc gtctggctct aacgtaccgt ccgtgccaca tatcatatat 1380
cgccattata cctcatgacc atgacaaaat atcttgttgc gctgctcatt gtcgtcggca 1440
cttttcacat attaatcaat tatcgcgtta cacgtctcta tcgctcgcct gctcaccatg 1500

<210> 42
<211> 1500
<212> DNA
<213> Aspergillus niger

<400> 42
   tccatgctat atcctacact gcgccacgtg gtagcggcct aaggtcgctg cgaacgcgac 60
   tcagacaact atatgaacta tcgtctgtat tactacgtct gtcgtttcgg ttatatgtgc 120
   tcatggatcc ctggcgggtt ggtaggtacc cgtacggcat ccgaaaaagc aaaatgagga 180
   agtctaggca ccaacaactt ccggagaaat atcggtgctt tacccagctt cctcacgcgg 240
   aggaagcccg aagaagggcc caaacaaagc gggattgaac acggttagcc gagaactcaa 300
   ctgactttac agaagcctag caagtggggc cccgaagtag gtacgagaga gattacgacg 360
   caagttagac ccttcgccac ctctcccccc ggggaagtac cgtcgctgcg agattaatcg 420
   cgcatagggt gctgagacac cccgtggaga agggttgtct ggtccggggc aatgggctgc 480
   tggcgatacg ggccgaatta cgtattattg cctcgcggac atattgacgc tctccgcaag 540
   aatgaaattg agctgctgtg gaatgaactc acgcgggatt ctttgcaaca acgtcatact 600
   cgagatccct tttgctaatc taaatcttcg cttttctcta ggcctaggga tcttcgagaa 660
   acttgaatac gaccgagaaa cgcccagtcg ttttgacccc atgggaggca cacctaatgt 720
   acgaaggtat cgcctagtat ttctcaaatg cttcgagtag tctaaccatt acatattgtg 780
   gcagagtacg gagtacttag tgtgctacca gttccgaccg ggcagtctcc ggagaaaatt 840
   actcgggtcc cagtcaaccc cggtcctgct gagccgtccg acttggcaat tgggtgtgag 900
   cccgtaaaac tttgcccgtg cacgttcctt ccgagcgggg aagggcatgg aagtttcttt 960
   gctaaacgga ctcagagtat tgggggcgtc gctcacttgc tactactcaa tcaagttagg 1020
   catgtttggc cttgagggtt tccgggggtc ctgtatttct aacgacccga cttctgttag 1080
   ctgttcaaga acccttgacg tcgagccttt cacgctcgct gcgattaaaa gaaccttttc 1140
   tccatggcgt gggttggatt tgatagtcgt taggcgcgtt gcttttctta ttcacatcac 1200
   cccataataa tacaaacctg atacaccttg atgcaaccgc gtttgggtcc agaagagaaa 1260
   gaaaagactt gattgtcgga tactctgggg acttgtgggg tcgaggaagt ggggtctggg 1320
   acatggaggc cggggattgg agagagggac aataatggta cggagtacaa taatccgcag 1380
   acaaccttcc cggctaaaca ggcattatta tatagagggc attggggtcg tctgactatt 1440
   tttcttccac cctgaccaca cataatcctt ttatcatatt gattcgacaa actcgaaatg 1500

<210> 43
<211> 1500
<212> DNA
<213> Aspergillus niger

<400> 43
gacacaagcc gaggatgacc acatcatagt aatctaccta agtggcaagg cgggatatgg 60
tattcctatc cacggtagca atgtacgaaa atgtttctct ctggctgaac caaacccatt 120
tcctgaaaag agagtttggg taggaaaatc ttcggggaca tgtagactat agtggcggtc 180
accacgcaca ccaaggtccc tgaggatatt tgcaggtgga tgggttacgt attggcttac 240
ttggcagagg aagagcaggg cggtgatgtg tggagaccag tgattggctt gatattggcc 300
tagatggggc tgttttctcg tgattcggga ttttagacca ataggaggcg agtcttggtg 360
ctgggatgga ggggcactgc atgtatgcct gaggtgtcga ggtgggatgg tttcctcagt 420
atcatcttta aaaggcagag ataagcttgt tggcggtgat ataacggagg gaaggggaac 480
tgggctgttg atacagctac acattaagga ttcggagaat tgagtggcaa accccgttat 540
tcggagaaag aaaagcagtt ctgtgtccat ttcggattta cggctgacct ctcctttggt 600
tctgcgaata ggcttgatat gcagtattga ttgattctta gttgccaatg gctatcccct 660
aagcatattg atagaaaatc catctgaatt gggaaccagg ttggaggtgg agtggcgtca 720
ggatcagggg ttgtacgaga agtaaccaag ctttggttgt accgatgcag actcggacga 780
agcgccggct tggttggatt gactgtctct gataactaga tctgccagtt gataccacgc 840
cgcaagagga taatgagcct cgggaattga tactctactg atagcagtgc tacttggctg 900
cagaattgat gcccagaagt catgagcgtg atttgtgatg gcccagagat gcaagccaga 960
tagacccatg attgaaccct gcagagagag ccgacctagt acgggatatc tgatccggaa 1020
acccattcca ggaagaagca gcaagacatg aacatcgcct cgagggccga tttgtcaacg 1080
gggacggtct agatatggtg gccagcggac gggaatggcg caggcccgaa aaaggaatgc 1140
gtggtggggt cagtcagtct cgggcggtct tcttgataat acagttcccc ctccaagccc 1200
taccaatcga agagtttcgg tatccttctg cttcggatca cctcatggcg ccgtgggcgg 1260
gccgtcgccg catcattcgt tgcccttgac acaggaacca ttcgctgaac tgaagctaag 1320
gtcgaattac ccgccacagg ctcgttgccc cgttccgtct ccccttcttc cctatataac 1380
caaccttcca ttcccttccc aaacttcttc actctcacaa ctcattacta ctactactac 1440
tactcaatca ctcttaacct ttctagtaca cgttactacc aactaatctc attcacaatg 1500

<210> 44
<211> 1500
<212> DNA
<213> Aspergillus niger

<400> 44
gctgctcgac cggaggctct gtgacataag tcgtgtctta atcacccaaa ttggattcgt 60
cgcaagggtc gagcaagcgc ccgccgttat ggaggcgtaa cctcgtgaga gccaccaact 120
atctgttcca ctgtcagcct caccatcacc cattctccgt agcatcactc ctcacctgtt 180
gtttcgtaga aatactcccg agaccgatcg tagaccgcca aatagacggc ccacgtcggc 240
aggtatccca ggagcatggg acccaatcct tgataaaggc ctcgaatacc atcctcccgc 300
catattactc gtccagttcc tagcatgccc cggtacagtg ttttggcctc gaccgccttg 360
ccacgtcgtc gcgcgaaccc accctgagcc tgcagcttgg tcttgatcac atcgagaggg 420
caggtgacaa ttcccgaggc tacgcccgca ctggccccgc agaacggcgt gatgtaataa 480
tccggcacac gtgtagcgag catttcgaag cgatcgagaa gagtgggctg ggatggggac 540
tcggccgata catgaagcgt cgacgaaggc gaactgtccg aagttgtacc ctgctgctct 600
tgagagccgc tggcagcccc cgaagggcca tggccttcag agaacatctt tccagccagc 660
actcaaagca gtatgcaatc cgtctactat gtcaccgagg gttcccggat ccgagcatat 720
cggtgctcgc tccggaaccc aagaataacc cgtgtgtcaa atcgtggtga gaggggagga 780
aagcgtgacg tcgttcgggg cgctggtaga acaggaaaaa ctaccccgag acaatgaatt 840
gaagggtttt ttctttcgca gagaggagcc gatgctgccg ggaggagtgg gcgtaataag 900
gttggcctat ctgaagcaca gacggggcgg ggtttgtagg agcaacgcag ctcccctatc 960
ggagtgaaag ggaatcgaac taagaaaaat gttcaacgga tagataataa gcggcatgcc 1020
cagtgagtac aaaccaaagt atcagaaaca aagaccgtcg ggtgaagtcc gaacagatg 1080
ggagcgccgg tgttaagagg aggcagacgg aacaggacgg agcaatgagg acgggatgaa 1140
ttgagctgga gggattgtct tagtcgtatt ccagtcagtc aggtggagtg gagtcgggaa 1200
tgagtgagtg aatcttgtcc gcctccaagc gcgccgctct tctggcctga ggcaaccatg 1260
gcgcaagctg agccggtcac gtggaaacca gtcttacaaa gtaacttctc ggaatgattc 1320

ctcctgcctc aggcaccaac atcggatgtt tttcggaaag tcacccgccg aacctccgtt 1380
ttgcacaatc tccaacgtcg ggccatctcc tgctcagctt gactgacatt tcgcgatcct 1440
ccccctccac accaccccgt caattggacc cgaacaaccc cgctgggatc aatcaccatg 1500

<210> 45
<211> 1500
<212> DNA
<213> Aspergillus niger

<400> 45
ggactgatgg ctccagtata agaagctccg ccctatgcac ctattggtgc ctgaggtctc 60
acatactttt aagcgaactg aacaaactag gattccgtat gaatataaga aatagctgga 120
aggctatctc atcttgttca aaggcacgtt cgattagcac gatctatccg tcctagatat 180
ggcagggctt gctcgaaaag ctcgggatcg cccggctcgg cttgtgccaa gatgacccga 240
gcctgcttat ttaaggcttg cgatttgata ctattggtgc tcaagagtat gttgatactc 300
aaaagtttgg ctcaaatata aaagaatcat taaaataagg ctcaatattt tataaggacc 360
aggataatga tggacaaccc ggctggacag aagtacaata cttgatattg ttagtgcaca 420
gtttatttag atcacatagc tagattatgt aagcttagac aagcgctcag aataacagtc 480
actttctcta atatattctc ctggggcaat ccaagatcat tcagggattg ctcaaaacat 540
atacttttgt acttctctta tactccaggc cactcagtga acaagaagca gcgccacgtt 600
caacctaaag tggaaagggg aagtcactca attttcgcag aaatccaatt cagtagcagt 660
acggctagtt gcaatatata taccaagcag cacaaccatt gagaacaagt tcttactact 720
gaagctatcg gacaatccag cagtatttcc agctatagtg acggcatcaa accatttact 780
tattctatac ttcatctaag caattgttct tattagcaat tggctatctt tggaacggcg 840
gtgatggcat cggagttcgc agcgagagat gggctgctta tcgcccaccc ctgccactga 900
taagcgatgc agatctgata tgttctcaac acattgacca gtcgcatctt atggctattg 960
ccactccacc gtcatcggca gccggcggaa gaggcaagca gattgcatca acagagaaca 1020
tcttcatctg aatgattgga ttatggaatt cctattatca ttgggaatta tcatgctgaa 1080
cggcgactct ccggatgggt tggcaagtcg ccgagcacgg ggccaacacg atcgctgatt 1140
ggtgcaaaac gccgaagcag ctgtacggcg cggggcagct tccaatccat cgcgagctct 1200
cctttaacag ctgccccctc gcttccattt aaaagccctt cgcgcggcca tttttctcgt 1260
gctttatctt ctctccccat agtcatctca attgcaccag ttgaagctat aatcggtgct 1320
ggtttgctgc ttgtctggtt gagcatcgat cgaggagctc cttatcaatc atcattgcgt 1380
tatcgtcatc gtatccttcc ccacatccct gatttccagc tgggtatttc tgtgcccctg 1440
gccaccccaa ctcattcctt tttctcagac ccatctcctc tccattaatt catcacaatg 1500

<210> 46
<211> 1500
<212> DNA
<213> Aspergillus niger

<400> 46
ccaggagggt tctgactaag ttgttgcatg gctgcgggag gcagggcgat agtaatatta 60
gactgcgtta atattatgca ggcgtggctt tttactgcac ccggccatca gtgaatcaag 120
ggagggtgga tcagccaatg tcgcccccct ccttccaaac aaacaaatca ttcacgttgt 180
caagatgctt ccccttcctt ctgtgacacg gaatggccgg agacagcgat gttttagcca 240
ctgggattgc agtatgtggt ggttccgggt aacatcgaca tgtcagtgaa tcaatcaatg 300
atgcaaaccg ttttggtcgt acgtaaattg tttgtctggt gacgggaatc ggtctgccaa 360
cccgacgaca gccgcatcat agttataatt attttagatt atgattcggt tgaaggagga 420
ctcggtacca ttcatcatcg tcatcatcag caatcatcat caaagatcaa tgctctcctt 480
cgggatcagt gtcacggtca cactgtggca aaacattctg tgtagccgat ctggagattc 540
ctactctata caaggataga tgaaatcgat ggacaataac aacaacaaca acatcatcat 600
catcatcatc atcatcatca caaaggagct gtatacaaag agtatagtat gcatgtatgc 660
atgtatgtat gtatttacgt gggatacaga atgtgtagag aatcagttag gattcggaaa 720
gtcacgactc aagaatcctc cgaaagaggc aagaatgttg gtaacaaaat aacagcaaaa 780

aaagaaagaa ggcaaaaaga gcaggtgatg ccctcgtgac ctgcccgcat ccatcatccc 840
aaatcctccc gtcaggaact ccagcttcat ccactccatt ctttcttagc gctactccat 900
ccaatctcca gtctctctcc tccttccctc catctcggtt cgtctccatc cgctttttct 960
ccgccggcga aaacggattc cccccgattc aggtactcac cgtccacatc tccccgtttg 1020
tcgtagcctg ctccggctct ctccagacag tctctccact ctctccccac tccaagacac 1080
gctctcgaag catccaatga actctccgcc ccctagcttc cgaatctccc acctcgttcg 1140
cctgtcttcg ggcccttgag atctgggccc ccgttttttc ttgccatggt cctaccccga 1200
ggatggggtc ctccaatccc tgggatggag gcgaatcgcc gacggatgga gcgtcaaatt 1260
ctttcacttc cattttagtg gcattcactc gttgacaccg tcatacttac cgagttcatc 1320
tttgtgtgcg gcccccctcc ccccccctct ccccctctgc ccggacccca tatatatccc 1380
tccatctccc ccgggtctgt cagccctttg gatgtctttc tctcccatca atcttctctt 1440
tttcccatct tcccatccct ggcagaggat tccgcgttgt atgaatccac cggcaacatg 1500

<210> 47
<211> 1500
<212> DNA
<213> Aspergillus niger

<400> 47
gaatatacct caaaccagag gtgccatatg gtccgggcga gacctgtccc aaccagatga 60
aagtggcacg acgtgttcaa cggagttgtc atggccccgg tccataattt agattcaatg 120
ctactggggc catctgataa cgtgggggtc cagtgagatt gatcccttgt ctggggaatg 180
atttcaagac ttgcggtgtg tgggacgccc ttgcttggat gggttggcat gtcccaacca 240
agcttggatc atgtggttgc ctgaggcgat aagagtcaat gggaggaatg taacatgggt 300
cacgtggtgc accgttgtgg aaagtgttgg gggtgaatgc atgcctgaat catgtggcga 360
cgatcatcag gttttcattc tccggactat tcaaaggagc atcaacggag cacccggaaa 420
caatttgtgc cgaggagctt gcggctcgca gtcatgccaa aacggaattt gatgtttcaa 480
actcggccgg tggcaataat gtccactcat agggctgccc tgtgcggaaa agtctgatct 540
gagtggacca atgactgatc caaggcatgc tgattatagc actagaatgc gttagtgtgg 600
ccacagcata agcaatgact ccaatgtttc aattctccag aattaggatc aactctggaa 660
gaaataggga ctattgctcg agtcgcaagc tagatggagt ggtgctactc cgagcaggca 720
atactttgat gcggaaaagg aaaccgtccc gcaatcccaa tcgggatgga tagccacagt 780
caagccaccc gagcaatgac accagccaca cagagcgatc aaggggcaaa aaacgttggg 840
gattcaacga atggttgaac tgttctgatt ggtggtccgc tcccgacctt acccaaagcg 900
gcagcttctg gccgagcagc gccatcgaat cagagggagc ccaacaagct tagttggagg 960
aacaggcggc gctgtatggt tggagatact ccggccattt gccatcgcgg atacactctg 1020
ccatccggac accttccaga cgtgcctgga taatactgtg gtagtagtcc ccttcctcac 1080
gctccctttc ttgtgtctgt tgaaccgtcg gccactttgg gacctcggga tctcatgatt 1140
acttcactga tctaccagtg aacttgccgt caggccaccc ttcttaactt attccatgcg 1200
ggtgtcctca tagtcgcatc attatcattg attgtccgcc ttgcttttcc ccaaccatca 1260
tccgccggtg gaccctggta gagttcaact gcctccgaat tttccctcct tcactggctc 1320
agatctgccg cttacttctt cggcctttcg attgcattcc ccaccctttt tctccgtctc 1380
ttttttatcg ctcgcctccg gctcttctct ttaaacccac cctctcccgc acgcatcctt 1440
ctcctcctca tccgaaccat actgcagggg tcgacaatac acacgaagga catcaccatg 1500

<210> 48
<211> 1500
<212> DNA
<213> Aspergillus niger

<400> 48
ggaggggctc ctcctcgcga tctgtccaag ctcggcatgg tcgatctcat gcaagaaaag 60
gaacgcatcg aagaagaact ctcggctctg agtagcttcc ttggctcggt gagttgaact 120
gatattggat aagccttgcc actctgtgag atgtcgactg acgatgcttt ctgtctagca 180
tggcgttaat atgaacacat ctctcactac tttcgatggc ttcccacgag acgatatcga 240

tgttgcccaa agtgagtcac tactttttgc ctccgcagtg cgtctgctaa acagtagtta 300
gtacgcacca ctcgagcccg gattattcga ctgcggaatg accataaaga cgtgatgtca 360
catctggaga aaggaattca caaccacttt gccaatctgc agcgggcgca aactgccgca 420
cagtcgggtg gcttggggtc gcaacctagt gtgactggga ataatacctc cggcactggg 480
gcatctgggt tacccttcgc aaaggtcaat agtgtggtgc ctggaagtcc cgcagaccag 540
gcaggtctaa gggttggaga taccgttcgg gagttcggaa gtgccaactg gctcaaccat 600
gaacgtctct ccagggtggc ggagatagtg cagcagagtg aaggggtaag taggcgatgt 660
gcttccaacc tcttagtcgt ctagtccagg gacatgcact cgggagaacg ctctaggcta 720
acgtcttcta gcgcactgtg gcggtcaaag tcgtgaggaa agatccgagt tcttccagca 780
gcatcgactt gtccctgcag cttgtccctc gccgtgattg gggcggtcgg ggcctgttgg 840
gctgtcatct cgtgccgctg tagtggactg gcatcgggtt aggcacgagg atgacggccg 900
tttactgtac tctgggatag tccctagaat actaggactt agccgggtac gatctacttt 960
cgtttatgat ttctcccttt tgcttgctat tggactttat gaggaatgtg acatgtattc 1020
atgatcgaac gcgacgtgtt tattcttgca gggaaatacc tagggaagca ttcagggtgg 1080
cgtttcagtt taggttcaag agatgaaatt gcacctgttt atatggacat tgtctatcag 1140
ttccgtacag cgtagagcgt tcactcgcga agaaccgcgt ggatcgacag cgaggcatgc 1200
aataaatgca gtttgcgcga tcctgcgggg cacgtggctg cctgaggccc aaaaagatca 1260
cgtggccgtc aggcttgggc gaccgactca cggcaaaaat tccatcaccg gtagaaataa 1320
ccttcccact ctaactcctc cctgtccaac cccctcgttc tatcgaactc ccctcggtcg 1380
cttcgtccat cgagtgtcgc atctcactgt cgacttttaa tttccgtcga cttctatcgc 1440
agactctgta tcgcttctct tcatctccaa cccctcacat cacatccaac cttcatcatg 1500

<210> 49
<211> 1500
<212> DNA
<213> Aspergillus niger

<400> 49
ctatagagcc gctcatcctg ctggcacgaa gctttcggga aggcatcatt cttttgtctg 60
ccgatcactc ctggatctga ctcaggtttt tgtctcccag caaacccatt gcaagttatg 120
cgacacggag ttcattccat catgcagcgg acggtgtctc tgaaaagaag ccctaaatga 180
gcacgtcatg gctcctcatg actgggatat ggtaccagat aaatttgtgc tggggtctga 240
cgttggagcg aaggcatcgt ccgttgagcc gtacatgaca ttctatgact ggttgccata 300
gttgttagtc caggtatttg atcatacatg gaaactcggc aaacacaaga tagaaactgg 360
caacttgtat gataagatgc gtttccccag cgggcatcca tggcaggttt agaatgaggc 420
gtatatctac ggtccctcca taaagtgcgg ataaacaggc ctgtcacaga gctcctcttc 480
cacgaatggc ctattagatc cagcaaaatg agccaagaat agtagtgtcc tcctccaact 540
ctcttagatt gggattatca acattgggca tactggagtc gctgtcggcc gtgatttggg 600
cctggcgggt aatgcgacat gttagattgg cagcgatgca ggtggagcag gaagagacac 660
ggttgcattt agtctacgag agtgaaattc ggttagaatg caaatgcacc taaggttcat 720
ggggttaaga cgagtgtctc accttgcgac tccggcactg gtcacacaga acaccatcat 780
caacaaatcg atcaaatgaa tcggcaatgg tcaatgaggt aaggcatgct aagggatgag 840
acgatgagag tccaactcac agccggtctt ttgctgtttc cacgggtgcc gataccgacc 900
tccatagcgc gggacattgg ctggtctaca cagtacgctc tgtggttgca tgtgggggag 960
ggacaatcgg tgcaactcgc ggcggaaccg gagctgccag gcagttattc cagccccatc 1020
cagtagatag tagtagttgg tagtcagatc catgcagacc aaccgagtca acgctagcag 1080
tgtgagagac tgcctgaggc aatcattctc aatatgctct tccctctttg ggagggaatg 1140
taggagttgc ctcgccatcg ggatgccaac tagaaaggcc gagattgagg ttagcgcggg 1200
acccgcgcta acccgaatct cggtcggcac atttccccat cgggcagtcc tcggattcgg 1260
agagggtaac acacaaagta ctatgcccaa catgcttgcc gaccagaaac accacggtcc 1320
gtgaccgggg actagggaga gacccatcca agatgatgcc gaacactcgc aattccattc 1380
gcataaggtg gtagagaggt ataaagtcac cgggagaagt ccatggtctc atagtcaaag 1440
cattccaaac ccttccgacc aagtcccttg aacttcctcc ccattcccca catcacaatg 1500

<210> 50

<211>  1500
<212>  DNA
<213>  Aspergillus niger

<400>  50
cgcccttatc tcatggaatt tcctttttgt gggacccttg cacctgtcac ccaaacattg      60
aacaatagca ataacaacaa acgagcccat cgaacccatg agcactatcc gaagcgaccc     120
tactaatgaa acctccagat aaaaatggta cgtatcgagg gcaagattag ttccaggtgg     180
gaaaaaagag agacaggggg cacaagtggg tccagcagcc aaccagccat tgggtcaaaa     240
gcggggaaga gagagccaca gcgggatagt ggccagagcg atgagtggcc aacggcatca     300
ggaacagcct gcaggcccca ctgaccccaa cactcattgg acagcaggga gaaacatgaa     360
acaaagtcta agttggcatt caacgtcctg tggctcagta aagcgaggtt tctgccccct      420
gaacttattt ccttttgatt atcgggggag gtggatgatg atgacatcct aatttttgga     480
cgggttacaa gtagtcagtt tagttgatcc cgcttttacc gttgggaccg agtggtgaat     540
gttactgcct ggtagactaa agtatgtact agtaagtaaa gtaccagtag tagtagtggt     600
gatgtactac gctgtagtga cgggacgatg tgcctgactg atgcaggtac ggaatcccgc     660
gcggttcatc aaagtaaata attgctatct actattgctt gaaatccaaa aaagttcgaa     720
cctaaaaatc cagtagggga accatcgatg acccaactaa gtatctaccg accccattaa     780
ctcccccctc cccccctgag ccgaagagtt agtcgactgt tcttccggtc gaaaagagca     840
atggagcgac cgatcaacag cacttgtctc gcaacgcatg gacagcttgg acagcatgga     900
cagtaattca gggccaggga cgaaagtcac ttggcggcac tagttggtct agtcaaactg     960
gcgaaggggc tggttggacc aaccaagagc tgtgggtgag acgggcgcag aacccacgag    1020
gcgcgaaaac gaaaaaaaac gagcgaaaga agtgagaagt gagaggggcc atcgcgggtt    1080
gccccaaact tgcccatgaa ggtgctctca caccgcatcg gcgctctgat tgattcgggg    1140
gccatcaact ccacccgcgt gcagttgcgc tctccccttt tcgtccctga gacgatctcc    1200
aggttaatct cccggctcat acaaagggcc cccggttccc ccctctgcct cccagctcgt    1260
tcctggttct ctctgttctt tctctttcaa gaccagactt ctttgactgc ttgaagcatc    1320
ccattgactt cggtcagcca ctcttttaac caaacccccc aagcaaaacc ccaactagcc    1380
acactctctt ttatctaaac gcagctccct gtgttttaca tacaaaacaa cccacacata    1440
aaaatcaaat tatcatcgac cttgacggtt ttattctttc ccaacacaac attcaagatg    1500

<210>  51
<211>  1500
<212>  DNA
<213>  Aspergillus niger

<400>  51
gaagaagcga ccggggagga cagaacccga atgcgattgg ggagtctctg gtctgaaaac      60
aaaagccgca cagaagagga cacatcgaca acggccatta ttcatctctc actcggtcca     120
gatgcgttag ctcgcagtca gatatgccgc tgagctataa gctagttaag ctgccagcgg     180
gacaaatgta agccaacaga caccgaaacc tctggttttg gcctggtcgc ttgcgagatt     240
catggcctat gatacagatc aagatcgaat ccagtgctac cttgcgcaaa gcaaagtggg     300
cattccgatg caagggaagg ccaagcaaac aataaggcca cgcaagaggg gtaatgtgtt     360
cttccgccgg ggctacggtg aaagacattt cgccccataa acaccgcgcc aagaggattc     420
gccaggtcaa acagtctcca tagcggggtt ttcctgggat aagccacacc acaggtttcg     480
atctgctgat ggtagagcgt tgccagccaa cggtctcccg tacactcttt tcccggaatt     540
attctgccta aggcactcag ccctgtcagg ggcatttcta acttcttggc cagcaagaca     600
agagattcga aacacagcca ctccgcccgg aatcgctcag cctagaaaac gggaatgttg     660
tcaatcagcc ttatgtcatt atctcggctc tgtctcactc gccaagggat tatcccgtgg     720
ggcgtcgttg cgcccagtga ctgtcagccc aacagaagcc catggatgca tctgcgacca     780
gccggatcca acagcagccg caaatgctca tcgatgacaa gcatgtcgcc attccagatt     840
caacgcacag cgggccatga catgcaccaa gcgccaatac caagactgct gcctaggacg     900
atagggaaac ggacgactga ccatggagat ggagccctgc ctgtggctga gatgaagggg     960
gagggaccct ttcagggtcc accgctgacc tgtctgccaa gaagcctcat tgagcacgcg    1020
gagtgggggg atcactggcc ccagagacca ccatgaaaca ataattgtca attcctatgg    1080
agcaatcagc cacaatagtg ggaattcagg cgcctgtctg gggctagaaa cggtgaaccg     1140

tcaaatcacc gttcatggtg cttcactcca taccggggtt gttgcattgc attcccctat 1200
cccaagaagc ccccacgaaa atagcctacc tactcttacc aggttgtcga caatcaatca 1260
atgaatgctc aagccactgg caaatgacct gaggccaagt gtatgatatc catgctcagg 1320
atctcttggt gactgcgaag agccgactcc actgaggcat aacgaggtat ataaaccccc 1380
ccttcatccc ctcttgccct ccatcaacaa acccaaccac aaccacaaag caatcaatct 1440
cattatacaa ccacttttcc tccattccct cataacctct ttcacaactc catcacaatg 1500

<210> 52
<211> 1500
<212> DNA
<213> Aspergillus niger

<400> 52
aagcgggcag ctgagtcagc caccgcggaa cggaggaatt gccggccgac attcgtcaaa 60
cccacaaaaa aagagtgggt gataggtttt cttttaccat cccaatctca aaagacgagg 120
ccggagcgca cttctctcaa caaccacatt aaacctttag gtatgtggta ttctcccgca 180
agccacggct gattgcccgt tattttgatc atgttcaaaa aacatttttt gtcatggcca 240
aaaaagcggt agcattcagt agtgggctct gaaatagcgg agtgaacaaa tgctgattaa 300
aattgatcat gcagaagttt agtcttcagc gtaacgatgg cccccaagag caagaagacc 360
ggtgacacca tcaactcgcg cctggcgctt gtgatgaagt ccggaaaggg tatgttatac 420
cagaatttgc cagttcagac attttagaca gtctctaatc gagcccatct agtcaccctc 480
ggctacaagt ccaccatcaa gaccctccgc gtgagtatac cggataattc aggcaatagc 540
cgtgcagaac atccgtgaat ggtcgactaa taacgtcaac agtccggcaa ggccaagctg 600
gtcatcatcg ccgccaacac tcctcctctt cgcaagagtg agcttgagta ctacgccatg 660
ctcgccaagg cccccgtcca ccacttctcc ggcaacaacg tgagtgactg cgatcagatc 720
tttggcatct ctatggagtg ggtccggacc tagttctggc atagactact tccgtagtga 780
tcgcaacgta aaactggagt aaacgctaac attatggtga acagatcgag ctcggtaccg 840
cctgcggtaa gctcttccgc acctccacca tggctatcct cgatgctggt gactccgaca 900
tcctgtccag ccagtaggtg taaagcctag tcaatggatt catgttatgt ctgaacggcg 960
gtatgccatg atgcacaacg tcacggcacg atttagataa tgacaatgaa ctttacatct 1020
atattgggtt tcgtttctaa acgctgtagg ataagactgt aggagtgatc tggaagatag 1080
cacggtcgca tcactaaaga accggaggga ggagtggcag ctcaactcca ctttatgagt 1140
cacgtgatta aaagtcaggc atttagacaa ttataaagca gcgaaaagtc atcacattcc 1200
attccgcagt aactaaaaca tgtttcattc acggcttaga aagaactgat atatcagtct 1260
gaacaagaat gtcggagttg ccgtagtctt acaaatgcgt catagcggct acagaatgtg 1320
attggcggag agggtagggt ttagggctgt gccactcggt agcgggcgct tgcttggaaa 1380
tcaacgtgga aagtgcctgc gcccataatc ccccttccct ttcgcctccc gacaccacct 1440
ttttcgacaa cctcaccgtt cgcaggaagt gcagccgccc catttccagc agtcaagatg 1500

<210> 53
<211> 1500
<212> DNA
<213> Aspergillus niger

<400> 53
tcgcccccct taggggtggg taataattac accattgacc atcttgattg atatcatata 60
ttggaaaacg gagtgatggc aaaaaaaaaa gaaaacaaaa agggtaaggg gatgactgaa 120
aacagagggg agatacgtag attttccacg ttactaaaaa gtaaataacg actccagctt 180
gtgtggccca tcatccatgg tatctgaggc acctcgaatc cttgacacga catctggtag 240
gtcagtttgg taggcagcat gagccacctc aggcggcagt aagccaatca ccgaggaccc 300
tgccaagtaa tccgcaattc attcgaccga caagtcaaag aatctccagg cccgaattgg 360
tgagggctga ggcgtcgggg ggttgtggtt gttgtggaca gagttggacg gcgaaaagtg 420
ggtaacgata cgcgcgtttt ggtactgcgt gcggtaatac cgatggtaac ccccgcccgg 480
tgcaggtggg atgaatgggg gagggtgaag cgggcttgtg ataggctgca ggggcgccca 540
tctgcttacc atatttacac cacccgggcc tttttaggaa taatccagta tcgcatacag 600

gtacaggact gggcgcgcaa gtaaattagc agatccagat cccaaaataa ccaccagcgc 660
cgccccccct ctccccttct tcccaggtcc tactagggag tagcgccagt cctatcttcc 720
ctgcttgggt ggtcaatcaa ttcccttttc accacttacc gtccaactca agcctcctcc 780
tccctccccg tccgtctctt agttccttcc ttccttcttt tcgttctttt gatcttcgac 840
ctctttttcc tttcggttcc tgttcgctct ttttttcgtc gacacttcgt ttcccccgac 900
tcatcttctg caacactagc gaccttccct ccctcccttc cttctttctt gctttcgttt 960
gaggaaagaa gagggaggcc tttcttggct gagacaggtt cagccaacgg cagtcgggag 1020
gcttttgctt ttgaaatccc accccctagt cgtgttttgt tctttacttc acttcaccct 1080
tccattcgtt tatcgaacgt ccctccagcg acgaccaacc ggccccgccc tggaagtcga 1140
cctacagagc aagagaaaaa aataagcaga cgatcgtttc gagttgagat tgttgtttag 1200
ttttacgccc ttgaaacaca ctccggcact ggtgccttca gagaaaaaga acgctgcgcc 1260
agcatcaaaa gtcattcttc gacgcgttct gaacatacac atccattgcg ccagaagtgc 1320
tatctgttta cgcttatttc actggaaatc ggatcagagt aatttttaat gggccactgt 1380
taatgtaagt tgcaggatct tgcattcaaa acgccatccg accccttagt aaggccatat 1440
tgatactcat ggtttagaag aagatattaa acggttcggt gattgtggct attcaacatg 1500

<210> 54
<211> 1500
<212> DNA
<213> Aspergillus niger

<400> 54
gtaaataaaa tacgacaaag caaacccaga cccatgatac ttgtaataaa aagatcaaga 60
acaccccaac gtcccagtca taccatgaac caacccgaaa aagcaacccg tcgcgtcact 120
gctgcggaag gacaactgac gcgccgaccg cgcaggatgg ctaaaaagaa acaagcagga 180
aaataaaaaa ggcgtttaac caccgaaacc gtagagggta cctgaggaat tccatgttag 240
acgcgttgga cacacaaaag ccaaaacgtg tggtacttac ggccctgtct cttaagagca 300
tagacgacgt cgagggaggt gacggtcttg cgcttggcgt gctcagtgta ggtgacagcg 360
tcacggatga caccctcaag gaaggtcttg aggacaccac gggtctcctc gtagatcatg 420
gcagagatac gcttgacacc accacgacga gcgagacgac ggatagcggg cttagtgata 480
ccctggatgt tgtcacgcaa gatcttgcgg tgacgcttgg cgccaccctt gccgagaccc 540
tttccaccct ttccacctgt tgagacgcgt caagtcagtc actgttgatc atggagtaga 600
cggcagtgga caatctatcg agatgataga tagcccatga tgggaagtga tgaacttacg 660
gccagtcatt gtgaaggttg tctaaaaagt aaacggaaat agagttgaat taaagttgag 720
ggtgaagatg gtggagataa gttaagcgtt ttagagtgag gatggatgta aagggaagag 780
ggcagagagc atccatggga cgggagatat ttgtatagca gaaggcgcgc cgcggggagg 840
gcgttggaat cacacgctag agcgcggatc gaccaatcag agcgcaggat cccggccggc 900
ggactgatct ggcggcaacg tgaccgggca gggatgattc cgctgggatg agactgattc 960
ccacgggaat gctcccaacc acccagatca gcaatcagag ggtgtcaggg acattcctgt 1020
cgatggccat gagcatctgc aacaggttga taggatcaca ttccgggcct gaggttggga 1080
tcaatgtgag gcaggggcag cttctagatc cgtgatcctg gcttcctatt ggctggcggg 1140
cattagggcg ctcgcttagc gctccgagca ggcagaaagg ggactgaccg cctttgggca 1200
cttttgattt tcccagccaa aagtgagccc cgaaatcacg gcttatggtg tgctgctttc 1260
cgaacacttt ttcgatcaaa ggaacggcac gctaaggccc cctccctttg ctggcaaacc 1320
cagatcacca cctacccttt cgcttactac aaatacctct ccccacccgc ctcctccatc 1380
cttcttccat ctcctcatcc cgtctaccct tatctatcaa cctccatcaa tctttcatcc 1440
tcaacgtcat ctttccatct ctaaaacaac taaatccatc aaacttaatc cactaagatg 1500

<210> 55
<211> 1500
<212> DNA
<213> Aspergillus niger

<400> 55
tgcgcattga tattcggaga tcgtagggct cgaatttgag agaccaggta gtccctgatt 60

cgttccactg cctacgtagt ttgtcagtcg gtttgtaaag tgaatctata tcggaagcag 120
tacctttttc ttgatatcac ccagtaaagg ccgcacatct tcgatagact tgctactatt 180
ggtgcctgag gccttggcct cgatgctagc cgtccgagtt tcaatctcat tcaaggcctt 240
gacccagttc tcatcaatag gcccctccgc cactaaccgg acggtcttcg gagacaaact 300
gatttcttcc acggcaggac caagcagctg ttcaacattc cgccgattct cgagcatcgc 360
attcagctgt atagaccgag attgcaaact ctcaatttct gccgatacag ctcccaattc 420
attctgaaag tcattcaaat acatttcaac tgacttggag acgtcatcgc agccagtgat 480
tgccgagtgc aggtcctgga atttgtccct ttctttctca aattgctgga tggtctgggc 540
accgacatcg ctttgccaag ctcttcgcgg ttggtcttct tccgcgataa agtcctccag 600
gctgagtcca tgaaaatcaa tgtcttccac gagctccggg ggtttcagtt ccgcgacgga 660
cgctggagag ctgtctcttg ttttgccttg tttcccaatg atcccattca agacttcgag 720
cggatcagcg acatcggatg ggcggggtct tgtagcactc ggttttggcg aggatccttc 780
gcctcgcgca ggcgcagaga gggaggtaga cgaatcattc ggggtcgaca agagagacaa 840
cgctgaacct tggcgactag acgcggggcg attgttctga ggggttggag ccaagcgaga 900
agaggtcctt cgaggcaagg gagagctgct gcgactatcg aattggggac cggagggcgt 960
cgcatggccc gaaatgcggt ctagccacat cttgggaggc cggcacggag aagcgtgcgg 1020
atagcggaga gctccccggg ttcatcgagg ggtgaggtag aggggtgagg gaagttctcc 1080
cagaacacag atgggtactg ggagatgtta cagggcggcg aacattcgag gagatggaga 1140
tcaagaccga gaccaggctg atattgtcga caggagataa ccagtagata actactgcta 1200
ctataatggt taggagtata cgtggaatag caggccgaga tgctacggcg aactgcagca 1260
accgcggacg tggtttgtcc gagactaggc tggactggga ctagcgacca caaaaccccg 1320
ccacaaccag tgcctttttt tcgcagtcca tttgtcgatt tcttttcgcc tgtgtccccc 1380
tcttcgtcct tccttcgctt ttttctcttc cttttcctct aatctcctcc tcaattccaa 1440
catcctcctc acctctccct ttcacttttt acctcatacc ccctaatatc catcaccatg 1500

## Description

### Field of the invention

The present invention relates to a recombinant host cell for the production of a compound of interest. The present invention also relates to isolated fungal promoter DNA sequences, to DNA constructs, vectors, and host cells comprising these promoters in operative association with coding sequences encoding a compound of interest. The present invention also relates to methods for expressing a gene of interest and/or producing compounds of interest using a promoter according to the invention.

### Background of the invention

Production of a recombinant polypeptide in a host cell is usually accomplished by constructing an expression cassette in which the DNA coding for the polypeptide is placed under the expression control of a promoter, suitable for the host cell. The expression cassette may be introduced into the host cell, by plasmid- or vector-mediated transformation. Production of the polypeptide may then be achieved by culturing the transformed host cell under inducing conditions necessary for the proper functioning of the promoter contained in the expression cassette.

For each host cell, expression of a coding sequence which has been introduced into the host by transformation and production of recombinant polypeptides encoded by this coding sequence requires the availability of functional promoters. Numerous promoters are already known to be functional in hosts cells, e.g. from fungal host cells. There are examples of cross-species use of promoters: the promoter of the *Aspergillus nidulans (A. nidulans* gpdA gene is known to be functional in *Aspergillus niger (A. niger)* (J Biotechnol. 1991 Jan;17(1):19-33. Intracellular and extracellular production of proteins in *Aspergillus* under the control of expression signals of the highly expressed *A. nidulans* gpdA gene. Punt PJ, Zegers ND, Busscher M, Pouwels PH, van den Hondel CA.) Another example is the *A. niger* beta-xylosidase xlnD promoter used in *A. niger and A. nidulans* (Transcriptional regulation of the xylanolytic enzyme system of Aspergillus, van Peij, NNME, PhD-thesis Landbouwuniversiteit Wageningen, the Netherlands, ISBN 90-5808-154-0) and the expression of the *Escherichia coli* beta-glucuronidase gene in *A. niger, A. nidulans* and *Cladosporium fulvum* as described in Curr Genet. 1989 Mar;15(3):177-80: Roberts IN, Oliver RP, Punt PJ, van den Hondel CA. "Expression of the *Escherichia coli* beta-glucuronidase gene in industrial and phytopathogenic filamentous fungi".

However, there is still a need for improvement of the recombinant expression and production of compounds of interest. For example, a known problem to be associated with the production of a compound of interest in a fungal host cell is that only part of the mycelium is involved in the production of the compound. This also applies to other host cells in which expression is not constitutive in all phases of the cell-cycle. It is therefore an objective of the invention to provide improved expression and production systems.

### Description of the Figures

Figure 1 depicts the plasmid map of pGBTOPGLA, which is an integrative glucoamylase expression vector.
Figure 2 depicts the plasmid map of pGBTOPGLA-2, which is an integrative glucoamylase expression vector with a multiple cloning site.
Figure 3 depicts the plasmid map of pGBTOPGLA-16, which is an integrative expression vector containing a promoter according to the invention in operative association with the glucoamylase coding sequence.
Figure 4 depicts glucoamylase activity in culture broth for *A. niger* strains expressing glaA constructs, all under control of distinct promoters. Construct are described in Table 2. Glucoamylase activities are depicted in relative units, with the average of the WT1 cultures at day 4 set at 100%. The two transformants per type indicated were independently isolated and cultivated transformants.
Figure 5 depicts the plasmid map of pGBDEL-PGLAA, which is a replacement vector.
Figure 6 depicts a schematic representation of a promoter replacement.
Figure 7 depicts a schematic representation of integration through homologous recombination.
Figure 8 depicts spatial activity of laccase in 6 day (upper panel) and 10 day (lower panel) old sandwiched colonies of *P. cinnabarinus* recombinant strains G13 (A), S1 (B) and L12-8 (C) (see Table 4 for explanation of the strains. Laccase activity is detected as a grey or black staining. Black arrow indicates edge of the colony.
Figure 9 depicts Laccase activity of strain GS8 (A) and the parental G14 strain (B). Laccase activity is detected as a grey or black staining. In G-S8 the largest part of the mycelium is devoted to secretion.

### Detailed description of the invention

It is an objective of the invention to provide improved expression systems based on recombinant hosts cells comprising multiple distinct promoter sequences. These promoters may e.g. demonstrate distinct activity during different phases of the cell cycle, or in different parts of a fungal cell or fungal mycelium. They may also be inducible by a specific convenient substrate or compound. Several distinct functional promoters are also advantageous when one envisages to simultaneously overexpress various genes in a single host. To prevent squelching (titration of specific transcription factors), it is preferable to use multiple distinct promoters, e.g. one specific promoter for each gene to be expressed.

Accordingly, the present invention relates to a recombinant host cell comprising at least two DNA constructs, each DNA construct comprising a coding sequence in operative association with a promoter DNA sequence, wherein the at least two DNA constructs comprise at least two distinct promoter DNA sequences and wherein the coding sequences comprised in said DNA constructs encode related polypeptides. Said recombinant host cell will herein be referred to as the recombinant host cell according to the invention. The recombinant host cell according to the invention is advantageously used for the recombinant production of at least one compound of interest. Optionally, said two DNA constructs, are comprised in a single construct. According to an aspect of the invention, the recombinant host comprises at least one DNA construct that is introduced into the host cell by recombinant techniques, i.e. the parental host from which the recombinant host is derived may already contain a native DNA construct comprising a coding sequence encoding a compound of interest.

The compound of interest may for instance be RNA, a polypeptide, a metabolite, or may be the entire host cell or a part thereof. (i.e. biomass or processed biomass, e.g. an extract of biomass).

The term "distinct promoter DNA sequences" is herein defined as promoter DNA sequences that are not both obtained from a single gene. The distinct promoter DNA sequences will be referred to as the promoter sequences according to the invention. According to the invention, a promoter DNA sequence may be native or foreign to the coding sequence and a promoter DNA sequence may be native or foreign to the host cell.

The term, "related polypeptides" is defined herein to encompass polypeptides involved in the production of a single compound of interest. One or more polypeptides may be the actual compound(s) of interest; another polypeptide may optionally be a regulator, e.g. a transcriptional activator of the polypeptide of interest. If the compound of interest is a metabolite, the related polypeptides may be enzymes involved in the production of a metabolite.

Otherwise, the related polypeptides may share a substantial match percentage. According to an embodiment of the invention, the related polypeptides share a match percentage of at least 50%. More preferably, the related polypeptides at least 60%, even more preferably at least 70%, even more preferably at least 80%, even more preferably at least 90%, even more preferably at least 99%. Most preferably, the related polypeptides are identical polypeptides.

For purposes of the present invention, the degree of identity, i.e. the match percentage, between two polypeptides, respectively two nucleic acid sequences is preferably determined using ClustalW as defined in: Thompson JD, Higgins DG, and Gibson TJ (1994) ClustalW: improving the sensitivity of progressive multiple sequence alignment through sequence weighting, positions-specific gap penalties and weight matrix choice. Nucleic Acids Research 22:4673-4680.

According to a preferred embodiment, the at least two distinct promoter DNA sequences possess distinct expression characteristics.

According to another preferred embodiment, at least one of the at least two promoter DNA sequences is selected from the group consisting of:
(a) a DNA sequence comprising a nucleotide sequence selected from the set consisting of: SEQ ID NO's:1 to 4 and 13 to 55 and the promoter DNA sequences of the genes listed in Table 1,
(b) a DNA sequence capable of hybridizing with the DNA sequence of (a),
(c) a DNA sequence sharing at least 80% homology with the DNA sequence of (a),
(d) a variant of any of the DNA sequences of (a) to (c), and
(e) a subsequence of any of the DNA sequences of (a) to (d),
wherein preferably at least two distinct promoter DNA sequences possess distinct expression characteristics.

The promoter DNA sequences of the genes depicted in Table 1 are for the purpose according to the invention defined as the 1500bp nucleotide sequence immediately upstream of the startcodon (ATG) of the respective gene. According to a more preferred embodiment of the invention, at least one of the at least two distinct promoter DNA sequences is selected from the promoter DNA sequences of the genes depicted in Table 1, wherein preferably at least two distinct promoter DNA sequences possess distinct expression characteristics, i.e. are selected from different columns (e.g. a promoter DNA sequence of a gene listed in column I combined with a promoter DNA sequence of gene listed in column II of Table 1).

Preferred promoter combinations are: the promoter DNA sequence of An03g06550 (e.g. glaA promoter with optimized translation initiation site as provided in SEQ ID NO: 16) combined with at least one of the promoter DNA sequences of An12g06930 or An05g02100 (e.g. amyB promoters as mentioned in WO 2006/092396 with optimized translation initiation site (as detailed in WO 2006/077258); SEQ ID NO: 17 represents promoter DNA sequence An12g06930 with optimized translation initiation site).

Other preferred promoter combinations are: at least one of the promoter DNA sequences of the set of genes An03g06550 (e.g. glaA promoter with optimized translation initiation site as provided in SEQ ID NO: 16), An12g06930 and/or An05g02100 (e.g. amyB promoters as mentioned in WO 2006/092396; SEQ ID NO: 17 represents promoter DNA sequence An12g06930 with optimized translation initiation site) combined with at least one of the promoter DNA sequences of An16g01830 (gpdA promoter as provided in SEQ ID NO: 18), promoters as mentioned in WO 2005/100573 (e.g. promoters as provided in SEQ ID NO: 13, 14, 15, 19 or 20).

Other preferred promoter combinations are: at least one of the promoter DNA sequences of the set of genes An03g06550 (e.g. glaA promoter with optimized translation initiation site as provided in SEQ ID NO: 16), An12g06930 and/or An05g02100 (e.g. amyB promoters as mentioned in WO 2006/092396; SEQ ID NO: 17 represents promoter DNA sequence An12g06930 with optimized translation initiation site), An16g01830 (gpdA promoter as provided in SEQ ID NO: 18), promoters as mentioned in WO 2005/100573 (e.g. promoters as provided in SEQ ID NO: 13, 14, 15, 19 or 20), combined with at least one of the promoter DNA sequences of the genes listed in column II of Table 1.

**Table 1. Aspergillus niger gene sequences. Columns I, II and III represent differentially expressed genes, i.e. disctinct expression characteristics of the promoter of the gene, wherein "distinct expression characteristics" are as defined below. The gene numbers refer to the sequences published by Pel et al., Genome sequencing and analysis of the versatile cell factory Aspergillus niger CBS 513.88, Nature Biotechnology 25, 221 - 231 (2007), said sequences are herein incorporated by reference. The annotated genome of Aspergillus niger CBS513.88 has been deposited at the EMBL database.**

| I | II | III |
|---|---|---|
| An03g06550 | An15g01590 | An15g07090 |
| An15g07700 | An14g00420 | An04g06510 |
| An11g01630 | An01g03090 | An14g03080 |
| An08g05640 | An02g07020 | An11g10490 |
| An02g10320 | An04g08150 | An08g03490 |
| An01g06860 | An16g09070 | An18g05640 |
| An04g08190 | An07g00070 | An01g03480 |
| An04g06380 | An04g09490 | An08g01960 |
| An15g03940 | An18g03380 | An09g06790 |
| An04g03360 | An15g01580 | An07g03880 |
| An04g00990 | An08g09880 | An14g00010 |
| An11g03340 | An03g02400 | An08g10060 |
| An04g06920 | An03g02360 | An01g05960 |
| An16g03330 | An01g11660 | An12g04870 |
| An16g07110 | An01g01950 | An04g02260 |
| An11g01660 | An17g00230 | An06g01550 |
| An01g07140 | An08g08370 | An08g06960 |
| An16g02930 | An18g05480 | An15g01700 |
| An12g00710 | An02g14590 | An01g02900 |
| An16g05930 | An12g09270 | An16g05920 |
| An16g05920 | An16g01880 | An08g07290 |
| An13g00320 | An11g02540 | An07g09990 |
| An12g06930 | An02g00090 | An17g00550 |
| An05g02100 | An09g00840 | An16g01830 |
| | An01g10930 | An18g04840 |
| | | An18g04220 |

The term "host cell" encompasses all suitable eukaryotic and prokaryotic host cells. The choice of a host cell will to a large extent depend upon the gene encoding the compound of interest and its source. The skilled person knows how to select appropriate host cell.

According to one embodiment, the recombinant host cell according to the invention is used for the production of a polypeptide.

According to another embodiment, the recombinant host cell according to the invention is used for the production of specific primary or secondary metabolites, said metabolites being the compound of interest, such as (beta-lactam) antibiotics, vitamins or carotenoids.

The term "DNA construct" is defined herein as a nucleic acid molecule, either single or double-stranded, which is isolated from a naturally occurring gene or which has been modified to contain segments of nucleic acid combined and juxtaposed in a manner that would not otherwise exist in nature.

The term "coding sequence" is defined herein as a nucleic acid sequence that is transcribed into mRNA, which is translated into a polypeptide when placed under the control of the appropriate control sequences. The boundaries of the coding sequence are generally determined by the ATG start codon, which is normally the start of the open reading frame at the 5' end of the mRNA and a transcription terminator sequence located just downstream of the open reading frame at the 3' end of the mRNA. A coding sequence can include, but is not limited to, genomic DNA, cDNA, semisynthetic, synthetic, and recombinant nucleic acid sequences.

In the context of this invention, a promoter DNA sequence is a DNA sequence, which is capable of controlling the expression of a coding sequence, when this promoter DNA sequence is in operative association with this coding sequence. A promoter DNA sequence can include, but is not limited to, genomic DNA, semisynthetic, synthetic, and recombinant nucleic acid sequences.

The term "promoter" or "promoter sequence", which terms are used interchangeably, is defined herein as a DNA sequence that binds the RNA polymerase and directs the polymerase to the correct transcriptional start site of a coding sequence to initiate transcription. RNA polymerase effectively catalyzes the assembly of messenger RNA complementary to the appropriate DNA strand of the coding region. The term "promoter" or "promoter sequence" will also be understood to include the 5' non-coding region (between promoter and translation start) for translation after transcription into mRNA, cis-acting transcription control elements such as enhancers, and other nucleotide sequences capable of interacting with transcription factors.

The term "in operative association" is defined herein as a configuration in which a promoter DNA sequence is appropriately placed at a position relative to a coding sequence such that the promoter DNA sequence directs the production of a polypeptide encoded by the coding sequence.

The term "distinct expression characteristics" is herein defined as a difference in expression level between at least two promoters when assayed under similar conditions, which difference occurs in at least one occasion. Examples of such occasions are listed below, but are depicted for illustrative purposes and are not to be construed as an exhaustive listing.

Distinct expression characteristics comprise expression differences which are the result of cellular differentiation. Cellular differentiation is a concept from developmental biology describing the process by which cells acquire a "type". The genetic material of a cell or organism remains the same, with few exceptions, but for example morphology may change dramatically during differentiation. Differentiation can involve changes in numerous aspects of cell physiology; size, shape, polarity, metabolic activity, responsiveness to signals or gene expression profiles can all change during differentiation. As such, a fungus can be differentiated between different parts of a hypha (such as hyphal tip versus the subapical part of the hypha) or between different parts of the mycelium (such as between the centre and the periphery of a vegetative mycelium, or between the vegetative mycelium and a reproductive structure such as a fruiting body, or a conidiophore).

Differentiation can occur for example as a result of age, growth condition, nutrient composition, stress (environmental condition), temperature, radiation, pressure, morphology, light, growth speed, fermentation type (batch, fed-batch, continuous growth condition, submerged, surface or solid state). An example of nutrient composition mediated differentiation is the induction of the transcription factor *Xln*R in *Aspergillus* by the nutrient xylose. The transcription factor *Xln*R induces the transcription of various genes encoding extracellular enzymes, whereas transcription of other genes is not induced (de Vries and Visser, Microb. Mol. Biol. Rev. 65: 497-522).

Differentiation, which may involve changes in numerous aspects of cell physiology, may find its basis at the transcription, translation, protein expression or enzyme activity level. The differentiation may start at the DNA, the RNA, mRNA, protein or enzyme activity or metabolite levels. Differentiation and thus distinct expression characteristics can be identified by measuring and comparing the DNA, the RNA, mRNA, protein or enzyme activity or metabolite levels between (potentially) differentiated cells. The (metabolic, protein of RNA) differences identified can be related to the genes involved. These genes are candidate genes possessing distinct expression characteristics. The expression profile and possible distinct expression characteristic can be investigated by measuring the mRNA levels of the genes between (potentially) differentiated cells under investigation. Differentially expressed genes contain a promoter with distinct expression characteristics. Differentially expressed genes differ preferably at least 2-fold in expression level, more preferably at least 3-fold, even more preferably at least 5-fold, even more preferably at least 10-fold, even more preferably at least 20-fold, even more preferably at least 50-fold and most preferably at least 100-fold. Preferably, one promoter results in undetectable expression compared to another promoter resulting in high level expression when assayed under similar conditions.

For application of promoter DNA sequences possessing distinct expression characteristics, the distinct expression characteristics may relate to industrial relevant conditions and processes. These conditions of differentiation relate to but are not limited to spatial, temporal, environmental or nutritional differences between cells occurring during industrial growth and production of biomass and product.

Distinct expression characteristics may be reflected during different phases of the cell cycle, or in different parts of the cell.

### Recombinant host cells

According to the invention, the recombinant host cell according to the invention may be any suitable eukaryotic and prokaryotic host cell.

According to an embodiment, the recombinant host cell is a prokaryotic cell. The prokaryotic host cell may be any prokaryotic host cell useful in the methods of the present invention. Preferably, the prokaryotic host cell is bacterial cell. The term "bacterial cell" includes both Gram-negative and Gram-positive microorganisms. Suitable bacteria may be selected from *e.g. Escherichia, Anabaena, Caulobacter, Gluconobacter, Rhodobacter, Pseudomonas, Paracoccus, Bacillus, Brevibacterium, Corynebacterium, Rhizobium (Sinorhizobium), Flavobacterium, Klebsiella, Enterobacter, Lactobacillus, Lactococcus, Methylobacterium, Propionibacterium, Staphylococcus* or *Streptomyces.* Preferably, the bacterial cell is selected from the group consisting of B. *subtilis, B. amyloliquefaciens, B. licheniformis, B. puntis, B. megaterium, B. halodurans, B. pumilus, G. oxydans, Caulobactert crescentus CB 15, Methylobacterium extorquens, Rhodobacter sphaeroides, Pseudomonas zeaxanthinifaciens, Paracoccus denitrificans, E. coli, C. glutamicum, Staphylococcus carnosus, Streptomyces lividans, Sinorhizobium melioti* and *Rhizobium radiobacter.*

According to an embodiment, the recombinant host cell is a eukaryotic cell. The eukaryotic host cell may be any eukaryotic host cell useful in the methods of the present invention. Preferably, the eukaryotic cell is a mammalian, insect, plant, fungal, or algal cell. Preferred mammalian cells include e.g. Chinese hamster ovary (CHO) cells, COS cells, 293 cells, PerC6 cells, and hybridomas. Preferred insect cells include e.g. Sf9 and Sf21 cells and derivatives thereof. More preferably, the recombinant host cell is a fungal host cell. The fungal host cell may be any fungal cell useful in the methods of the present invention. "Fungi" as used herein includes the phyla Ascomycota, Basidiomycota, Chytridiomycota, and Zygomycota (as defined by Hawksworth et al., In, Ainsworth and Bisby's Dictionary of The Fungi, 8th edition, 1995, CAB International, University Press, Cambridge, UK) as well as the Oomycota (as cited in Hawksworth et al., *supra)* and all mitosporic fungi (Hawksworth et al., *supra).*

According to preferred embodiment, the fungal host cell is a yeast cell. "Yeast" as used herein includes ascosporogenous yeast (Endomycetales), basidiosporogenous yeast, and yeast belonging to the Fungi Imperfecti (Blastomycetes). Since the classification of yeast may change in the future, for the purposes of this invention, yeast shall be defined as described in Biology and Activities of Yeast (Skinner, F. A., Passmore, S. M., and Davenport, R. R., eds, Soc. App. Bacteriol. Symposium Series No. 9, 1980).

According to a more preferred embodiment, the yeast host cell is a *Candida, Hansenula, Kluyveromyces, Pichia, Saccharomyces, Schizosaccharomyces,* or *Yarrowia* cell.

According to an even more preferred embodiment, the yeast host cell is a *Saccharomyces carlsbergensis, Saccharomyces cerevisiae, Saccharomyces diastaticus, Saccharomyces douglasii, Saccharomyces kluyveri, Saccharomyces norbensis* or *Saccharomyces oviformis* cell. According to another even more preferred embodiment, the yeast host cell is a *Kluyveromyces lactis* cell. According to another even more preferred embodiment, the yeast host cell is a *Yarrowia lipolytica* cell.

According to another preferred embodiment, the fungal host cell is a filamentous fungal cell. "Filamentous fungi" include all filamentous forms of the subdivision Eumycota and Oomycota (as defined by Hawksworth et al., 1995, supra). The filamentous fungi are characterized by a mycelial wall composed of chitin, cellulose, glucan, chitosan, mannan, and other complex polysaccharides. Vegetative growth is by hyphal elongation and carbon catabolism is mostly obligatory aerobic. In contrast, vegetative growth by yeasts such as *Saccharomyces cerevisiae* is by budding of a unicellular thallus and carbon catabolism may be fermentative.

According to a more preferred embodiment, the filamentous fungal host cell is a cell of a species of, but not limited to, *Acremonium, Agraricus, Aspergillus, Aureobasidium, Chrysosporum, Coprinus, Cryptococcus, Filibasidium, Flammulina, Fusarium, Humicola, Magnaporthe, Mucor, Myceliophthora, Neocallimastix, Neurospora, Paecilomyces, Penicillium, Phanerochaete, Piromyces, Pleurotus, Schizophyllum, Shiitake, Talaromyces, Thermoascus, Thielavia, Tolypocladium, Trametes,* or *Trichoderma* strain.

According to an even more preferred embodiment, the filamentous fungal host cell is an *Aspergillus awamori, Aspergillus aculeatus, Aspergillus foetidus, Aspergillus japonicus, A. nidulans, Asprgillus niger, Aspergillus sojae, Aspergillus tubigenis, Aspergillus vadensis* or *Aspergillus oryzae* cell. According to another even more preferred embodiment, the filamentous fungal host cell is a *Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum. Fusarium reticulatun. Fusarium roseum, Fusarium sambucinum, Fusarium sarcochroum, Fusarium sporotrichioides, Fusarium sulphureum, Fusarium torulosum, Fusarium trichothecioides,* or *Fusarium venenatum* cell. According to another even more preferred embodiment, the filamentous fungal host cell is a *Agraricus bisprorus, Chrysosporium lucknowense, Humicola insolens, Humicola lanuginosa, Mucor miehei, Myceliophthora thermophila, Neurospora crassa, Penicillium purpurogenum, Penicillium chrysogenum, Pycnoporus cinnabarinus, Thielavia terrestris, Trichoderma harzianum, Trichoderma koningii, Trichoderma longibrachiatum, Trichoderma reesei,* or *Trichoderma viride* cell.

Several strains of filamentous fungi are readily accessible to the public in a number of culture collections, such as the American Type Culture Collection (ATCC), Deutsche Sammlung von Mikroorganismen und Zellkulturen GmbH (DSM), Centraalbureau Voor Schimmelcultures (CBS), Banque de Resources Fongiques de Marseille, France, and Agricultural Research Service Patent Culture Collection, Northern Regional Research Center (NRRL) *Aspergillus niger* CBS 513.88, *Aspergillus oryzae* ATCC 20423, IFO 4177, ATCC 1011, ATCC 9576, ATCC14488-14491, ATCC 11601, ATCC12892, *P. chrysogenum* CBS 455.95, *Penicillium citrinum* ATCC 38065, *Penicillium chrysogenum* P2, *Acremonium chrysogenum* ATCC 36225 or ATCC 48272, *Trichoderma reesei* ATCC 26921 or ATCC 56765 or ATCC 26921, *Aspergillus sojae* ATCC11906, *Chrysosporium lucknowense* ATCC44006, *Pycnoporus cinnabarinus* BRFM44, and derivatives thereof. Optionally, the filamentous fungal host cell comprises an elevated unfolded protein response (UPR) compared to the wild type cell to enhance production abilities of a compound of interest. UPR may be increased by techniques described in US2004/0186070A1 and/or US2001/0034045A1 and/or WO01/72783A2. More specifically, the protein level of HAC1 and/or IRE1 and/or PTC2 has been modulated in order to obtain a host cell having an elevated UPR.

Alternatively, or in combination with an elevated UPR, the filamentous fungal host cell may comprise a specific one-way mutation of the sec61 translocation channel between ER and cytoplasm as described in WO2005/123763. Such mutation confers a phenotype wherein de novo synthesised polypeptides can enter the ER through sec61, however, retrograde transport through sec61 is impaired in this one-way mutant.

Alternatively, or in combination with an elevated UPR and/or one-way mutation of the sec61 translocation channel, the filamentous fungal host cell is genetically modified to obtain a phenotype displaying lower protease expression and/or protease secretion compared to the wild type cell in order to enhance production abilities of a compound of interest. Such phenotype may be obtained by deletion and/or modification and/or inactivation of a transcriptional regulator of expression of proteases. Such a transcriptional regulator is e.g. prtT. Lowering expression of proteases by modulation of prtT is preferable performed by techniques described in US2004/0191864A1, WO2006/04312 and WO2007/062936.

Alternatively, or in combination with an elevated UPR and/or and/or one-way mutation of the sec61 translocation channel, a phenotype displaying lower protease expression and/or protease secretion, the filamentous fungal host cell displays an oxalate deficient phenotype in order to enhance the yield of production of a compound of interest. An oxalate deficient phenotype is preferable obtained by techniques described in WO2004/070022, which is herein enclosed by reference.

Alternatively, or in combination with an elevated UPR and/or and/or one-way mutation of the sec61 translocation channel, a phenotype displaying lower protease expression and/or protease secretion and/or oxalate deficiency, the filamentous fungal host cell displays a combination of phenotypic differences compared to the wild type cell to enhance the yield of production of the compound of interest. These differences may include, but are not limited to, lowered expression of glucoamylase and/or neutral alpha-amylase A and/or neutral alpha-amylase B, protease, and oxalic acid hydrolase. Said phenotypic differences displayed by the filamentous fungal host cell may be obtained by genetic modification according to the techniques described in US2004/0191864A1.

### Promoter DNA sequences

Promoter activity is preferably determined by measuring the concentration of the protein(s) coded by the coding sequence(s), which is (are) in operative association with the promoter. Alternatively the promoter activity is determined by measuring the enzymatic activity of the protein(s) coded by the coding sequence(s), which is (are) in operative association with the promoter. Most preferably, the promoter activity (and its strength) is determined by measuring the expression of the coding sequence of the *lac*Z reporter gene (Luo (Gene 163 (1995) 127-131). According to another preferred method, the promoter activity is determined by using the green fluorescent protein as coding sequence (Microbiology. 1999 Mar;145 ( Pt 3):729-34. Santerre Henriksen AL, Even S, Muller C, Punt PJ, van den Hondel CA, Nielsen J.Study)

Additionally, promoter activity can be determined by measuring the mRNA levels of the transcript generated under control of the promoter. The mRNA levels can, for example, be measured by Northern blot or real time quantitative PCR (J. Sambrook, 2000, Molecular Cloning, A Laboratory Manual, 3d edition, Cold Spring Harbor, N.Y.).

According to an aspect of the invention, the promoter DNA sequence according to the invention is a DNA sequence capable of hybridizing with a DNA sequence comprising a nucleotide sequence selected from the set consisting of: SEQ ID NO's:1 to 4 and 13 to 55 and the promoter DNA sequences of the genes listed in Table 1. According to a preferred embodiment, the promoter DNA sequence according to the invention is a DNA sequence comprising a nucleotide sequence selected from the set consisting of: SEQ ID NO's:1 to 4 and 13 to 55 and the promoter DNA sequences of the genes listed in Table 1.The present invention encompasses (isolated) promoter DNA sequences that retain promoter activity and hybridize under very low stringency conditions, preferably low stringency conditions, more preferably medium stringency conditions, more preferably medium-high stringency conditions, even more preferably high stringency conditions, and most preferably very high stringency conditions with a nucleic acid probe that corresponds to:
(i) nucleotides 1 to 1500 of: a sequence selected from the set of SEQ ID NO's:1 to 4 and 13 to 55 and the promoter DNA sequences of the genes listed in Table 1, preferably nucleotides 100 to 1490, more preferably 200 to 1480, even more preferably 300 to 1470, even more preferably 350 to 1450 and most preferably 360 to 1400
(ii) is a subsequence of (i), or
(iii) is a complementary strand of (i), (ii), (J. Sambrook et al., *supra).*

The subsequence of a sequence selected from the set of SEQ ID NO's:1 to 4 and 13 to 55 and the promoter DNA sequences of the genes listed in Table 1, may be at least 100 nucleotides, preferably at least 200 nucleotides, more preferably at least 300 nucleotides, even more preferably at least 400 nucleotides and most preferably at least 500 nucleotides. Hybridization conditions are as defined further in the description.

The nucleic acid sequence of a sequence selected from the set of SEQ ID NO's:1 to 4 and 13 to 55 and the promoter DNA sequences of the genes listed in Table 1, or a subsequence thereof may be used to design a nucleic acid probe to identify and clone DNA promoters from strains of different genera or species according to methods well known in the art. In particular, such probes can be used for hybridization with the genomic or cDNA of the genus or species of interest, following standard Southern blotting procedures; in order to identify and isolate the corresponding gene therein. Such probes can be considerably shorter than the entire sequence, but should be at least 15, preferably at least 25, and more preferably at least 35 nucleotides in length. Additionally, such probes can be used to amplify DNA promoters by PCR. Longer probes can also be used. DNA, RNA and Peptide Nucleid Acid (PNA) probes can be used. The probes are typically labelled for detecting the corresponding gene (for example, with 32P, 33P 3H, 35S, biotin, or avidin or a fluorescent marker). Such probes are encompassed by the present invention.

Thus, a genomic DNA or cDNA library prepared from such other organisms may be screened for DNA, which hybridizes with the probes described above and which encodes a polypeptide. Genomic or other DNA from such other organisms may be separated by agarose or polyacrylamide gel electrophoresis, or other separation techniques. DNA from the libraries or the separated DNA may be transferred to and immobilized on nitrocellulose or other suitable carrier material. In order to identify a clone or DNA that shares homology with a sequence selected from the set of SEQ ID NO's:1 to 4 and 13 to 55 and the promoter DNA sequences of the genes listed in Table 1, or a subsequence thereof, the carrier material may be used in a Southern blot.

For purposes of the present invention, hybridization indicates that the nucleic acid sequence hybridizes to a labeled nucleic acid probe corresponding to a nucleic acid sequence selected from the set of SEQ ID NO's:1 to 4 and 13 to 55, the complementary strand, or subsequence thereof or corresponding to a promoter DNA sequence of one of the genes listed in Table 1, the complementary strand, or subsequence thereof, under very low to very high stringency conditions. Molecules to which the nucleic acid probe hybridizes under these conditions are detected using for example a X-ray film. Other hybridisation techniques also can be used, such as techniques using fluorescence for detection and glass sides and/or DNA microarrays as support. An example of DNA microarray hybridisation detection is given in FEMS Yeast Res. 2003 Dec;4(3):259-69 (Daran-Lapujade P, Daran JM, Kotter P, Petit T, Piper MD, Pronk JT. "Comparative genotyping of the Saccharomyces cerevisiae laboratory strains S288C and CEN.PK113-7D using oligonucleotide microarrays". Additionally, the use of PNA microarrays for hybridization is described in Nucleic Acids Res. 2003 Oct 1;31(19): 119 (Brandt O, Feldner J, Stephan A, Schroder M, Schnolzer M, Arlinghaus HF, Hoheisel JD, Jacob A. PNA microarrays for hybridisation of unlabelled DNA samples.)

Preferably, the nucleic acid probe is a nucleic acid sequence selected from the set of SEQ ID NO's:1 to 4 and 13 to 55 or of a promoter DNA sequence of one of the genes listed in Table 1. More preferably, the nucleic acid probe is the sequence having nucleotides 20 to 1480 of a sequence selected from the set of SEQ ID NO's:1 to 4 and 13 to 55 or of a promoter DNA sequence of one of the genes listed in Table 1, more preferably nucleotides 500 to 1450, even more preferably nucleotides 800 to 1420, and most preferably nucleotides 900 to 1400 of a sequence selected from the set of SEQ ID NO's:1 to 4 and 13 to 55 or of a promoter DNA sequence of one of the genes listed in Table 1. Another preferred probe is the part of the DNA sequence upstream of the transcription start site.

For long probes of at least 100 nucleotides in length, very low to very high stringency conditions are defined as prehybridization and hybridization at 42 DEG C. in 5 x SSPE, 0.3% SDS, 200 microgram/ml sheared and denatured salmon sperm DNA, and either 25% formamide for very low and low stringencies, 35% formamide for medium and medium-high stringencies, or 50% formamide for high and very high stringencies, following standard Southern blotting procedures.

For long probes of at least 100 nucleotides in length, the carrier material is finally washed three times each for 15 minutes using 2 x SSC, 0.2% SDS preferably at least at 45 DEG C. for very low stringency, more preferably at least at 50 DEG C. for low stringency, more preferably at least at 55 DEG C. for medium stringency, more preferably at least at 60 DEG C. for medium-high stringency, even more preferably at least at 65 DEG C. for high stringency, and most preferably at least at 70 DEG C. for very high stringency.

For short probes which are about 15 nucleotides to about 100 nucleotides in length, stringency conditions are defined as prehybridization, hybridization, and washing post-hybridization at 5 DEG C. to 10 DEG C. below the calculated Tm using the calculation according to Bolton and McCarthy (1962, Proceedings of the National Academy of Sciences USA 48:1390) in 0.9 M NaCl, 0.09 M Tris-HCl pH 7.6, 6 mM EDTA, 0.5% NP-40, 1 x Denhardt's solution, 1 mM sodium pyrophosphate, 1 mM sodium monobasic phosphate, 0.1 mM ATP, and 0.2 mg of yeast RNA per ml following standard Southern blotting procedures.

For short probes which are about 15 nucleotides to about 100 nucleotides in length, the carrier material is washed once in 6 x SCC plus 0.1% SDS for 15 minutes and twice each for 15 minutes using 6 x SSC at 5 DEG C. to 10 DEG C. below the calculated Tm.

According to another embodiment, a sequence selected from the set of SEQ ID NO's:1 to 4 and 13 to 55 or a promoter DNA sequence of one of the genes listed in Table 1 is first used to clone the native gene, coding sequence of said native gene or part of it, which is operatively associated with a sequence selected from the set of SEQ ID NO's:1 to 4 and 13 to 55 or a promoter DNA sequence of one of the genes listed in Table 1. This can be done starting with either a sequence selected from the set of SEQ ID NO's:1 to 4 and 13 to 55, a promoter DNA sequence of one of the genes listed in Table 1, or a subsequence thereof as earlier defined and using this sequence as a probe. The probe is hybridised to a cDNA or a genomic library of a given host, either *Aspergillus niger* or any other fungal host as defined in this application. Once the native gene or part of it is cloned, it can be subsequently used itself as a probe to clone genes that share homology to the native gene derived from other fungi by hybridisation experiments as described herein. Preferably, the gene shares at least 55% homology with the native gene, more preferably at least 60%, more preferably at least 65%, more preferably at least 70%, even more preferably at least 75% preferably about 80%, more preferably about 90%, even more preferably about 95%, and most preferably about 97% homology with the native gene. The sequence upstream of the coding sequence of the gene sharing homology with the native gene is a promoter encompassed by the present invention.

Alternatively, the sequence of the native gene, coding sequence or part of it, which is operatively associated with a promoter according to the invention can be identified by using a sequence selected from the set of SEQ ID NO's:1 to 4 and 13 to 55, or a subsequence thereof as earlier defined, or a gene sequence listed in Table 1, or a subsequence thereof, to search genomic databases using for example an alignment or BLAST algorithm as described herein. This resulting sequence can subsequently be used to identify orthologues or homologous genes in any other fungal host as defined in this application. The sequence upstream the coding sequence of the identified orthologue or homologous gene is a promoter encompassed by the present invention.

According to yet another embodiment, the promoter DNA sequence according to the invention is a(n) (isolated) DNA sequence, which shared at least 80% homology (identity) to a sequence selected from the set of SEQ ID NO's:1 to 4 and 13 to 55 or to a promoter DNA sequence of one of the genes listed in Table 1. Preferably, the DNA sequence shares at least preferably about 85%, more preferably about 90%, even more preferably about 95%, and most preferably about 97% homology with a sequence selected from the set of SEQ ID NO's:1 to 4 and 13 to 55 or with a promoter DNA sequence of one of the genes listed in Table 1.

For purposes of the invention, the terms "homology" and "identity" are used interchangeably.

The degree of homology (identity) between two nucleic acid sequences is preferably determined by the BLAST program. Software for performing BLAST analyses is publicly available through the National Center for Biotechnology Information (http://www.ncbi.nim.nih.gov/). The BLAST algorithm parameters W, T, and X determine the sensitivity and speed of the alignment. The BLAST program uses as defaults a wordlength (W) of 11, the BLOSUM62 scoring matrix (see Henikoff & Henikoff, Proc. Natl. Acad. Sci. USA 89: 10915 (1989)) alignments (B) of 50, expectation (E) of 10, M=5, N=-4, and a comparison of both strands.

According to yet another embodiment of the invention, the promoter DNA sequence is a variant of a sequence selected from the set of SEQ ID NO's:1 to 4 and 13 to 55 or of a promoter DNA sequence of one of the genes listed in Table 1.

The term "variant" or "variant promoter" is defined herein as a promoter having a nucleotide sequence comprising a substitution, deletion, and/or insertion of one or more nucleotides of a parent promoter, wherein the variant promoter has more or less promoter activity than the corresponding parent promoter. The term "variant promoter" will encompass natural variants and in vitro generated variants obtained using methods well known in the art such as classical mutagenesis, site-directed mutagenesis, and DNA shuffling. A variant promoter may have one or more mutations. Each mutation is an independent substitution, deletion, and/or insertion of a nucleotide.

According to a preferred embodiment, the variant promoter is a promoter, which has at least a modified regulatory site as compared to the promoter sequence first identified (e.g. a sequence selected from the set of SEQ ID NO's:1 to 4 and 13 to 55 or a promoter DNA sequence of one of the genes listed in Table 1).Such a regulatory site can be removed in its entirety or specifically mutated as explained above. The regulation of such promoter variant is thus modified so that for example it is no longer induced by glucose. Examples of such promoter variants and techniques on how to obtain these are described in EP 673 429 or in WO 94/04673.

The promoter variant can be an allelic variant. An allelic variant denotes any of two or more alternative forms of a gene occupying the same chromosomal locus. Allelic variation arises naturally through mutation, and may result in polymorphism within populations. The variant promoter may be obtained by (a) hybridizing a DNA under very low, low, medium, medium-high, high, or very high stringency conditions with (i) a sequence selected from the set of SEQ ID NO's:1 to 4 and 13 to 55 or a promoter DNA sequence of one of the genes listed in Table 1, (ii) a subsequence of (i) or (iii) a complementary strand of (i), (ii), and (b) isolating the variant promoter from the DNA. Stringency and wash conditions are as defined herein.

According to yet another embodiment, the promoter is a subsequence of a sequence selected from the set of SEQ ID NO's:1 to 4 and 13 to 55 or of a promoter DNA sequence of one of the genes listed in Table 1, said subsequence still having promoter activity. The subsequence preferably contains at least about 100 nucleotides, more preferably at least about 200 nucleotides, and most preferably at least about 300 nucleotides.

According to another preferred embodiment, a subsequence is a nucleic acid sequence encompassed by a sequence selected from the set of SEQ ID NO's:1 to 4 and 13 to 55 or by a promoter DNA sequence of one of the genes listed in Table 1, wherein one or more nucleotides from the 5' and/or 3' end are deleted, said nucleic acid sequence still having promoter activity.

According to another preferred embodiment, the promoter subsequence is a 'trimmed' promoter sequence, i.e. a sequence fragment which is upstream from translation start and/or from transcription start. An example of trimming a promoter and functionally analysing it is described in Gene. 1994 Aug 5;145(2):179-87: the effect of multiple copies of the upstream region on expression of the *Aspergillus niger* glucoamylase-encoding gene. Verdoes JC, Punt PJ, Stouthamer AH, van den Hondel CA).

The promoter according to the invention can be a promoter, whose sequence may be provided with linkers for the purpose of introducing specific restriction sites facilitating ligation of the promoter sequence with the coding region of the nucleic acid sequence encoding a polypeptide.

Unless otherwise indicated, all nucleotide sequences determined by sequencing a DNA molecule herein were determined using an automated DNA sequencer. Therefore, as is known in the art for any DNA sequence determined by this automated approach, any nucleotide sequence determined herein may contain some errors. Nucleotide sequences determined by automation are typically at least about 90% identical, more typically at least about 95% to at least about 99.9% identical to the actual nucleotide sequence of the sequenced DNA molecule. The actual sequence can be more precisely determined by other approaches including manual DNA sequencing methods well known in the art.

The person skilled in the art is capable of identifying such erroneously identified bases and knows how to correct for such errors.

The sequence information as provided herein should therefore not be so narrowly construed as to require inclusion of erroneously identified bases. The specific sequences disclosed herein can readily be used to isolate the original DNA sequence e.g. from a filamentous fungus, in particular *Aspergillus niger,* and be subjected to further sequence analyses thereby identifying sequencing errors.

The present invention encompasses functional promoter equivalents typically containing mutations that do not alter the biological function of the promoter it concerns. The term "functional equivalents" also encompasses orthologues of the *A. niger* DNA sequences. Orthologues of the *A. niger* DNA sequences are DNA sequences that can be isolated from other strains or species and possess a similar or identical biological activity.

The promoter sequences of the present invention may be obtained from microorganisms of any genus. For purposes of the present invention, the term "obtained from" as used herein in connection with a given source shall mean that the polypeptide is produced by the source or by a cell in which a gene from the source has been inserted.

According to an embodiment of the invention, the promoter sequences are obtained from a prokaryotic source, preferably from a species of *Escherichia, Anabaena, Caulobactert, Gluconobacter, Rhodobacter, Pseudomonas, Paracoccus, Bacillus, Brevibacterium, Corynebacterium, Rhizobium (Sinorhizobium), Flavobacterium, Klebsiella, Enterobacter, Lactobacillus, Lactococcus, Methylobacterium, Propionibacterium, Staphylococcus* or *Streptomyces.* More preferably, promoter sequences are obtained from *B. subtilis, B. amyloliquefaciens, B. licheniformis, B. puntis, B. megaterium, B. halodurans, B. pumilus, G. oxydans, Caulobactert crescentus CB 15, Methylobacterium extorquens, Rhodobacter sphaeroides, Pseudomonas zeaxanthinifaciens, Paracoccus denitrificans, E. coli, C. glutamicum, Staphylococcus carnosus, Streptomyces lividans, Sinorhizobium melioti* or *Rhizobium radiobacter.*

According to another embodiment, the promoter sequences are obtained from a fungal source, preferably from a yeast strain such as a *Candida, Hansenula, Kluyveromyces, Pichia, Saccharomyces, Schizosaccharomyces,* or *Yarrowia strain,* more preferably.from a *Saccharomyces carlsbergensis, Saccharomyces cerevisiae, Saccharomyces diastaticus, Saccharomyces douglasii, Saccharomyces kluyveri, Saccharomyces norbensis* or *Saccharomyces oviformis* strain. According to another more preferred embodiment, the promoter sequences are obtained from a *Kluyveromyces lactis* strain. According to another more preferred embodiment, the promoter sequences are obtained from a *Yarrowia lipolytica* strain.

According to yet another embodiment, the promoter sequences are obtained from a filamentous fungal strain such as an *Acremonium, Agraricus, Aspergillus, Aureobasidium, Chrysosporum, Coprinus, Cryptococcus, Filibasidium, Fusarium, Humicola, Magnaporthe, Mucor, Myceliophthora, Neocallimastix, Neurospora, Paecilomyces, Penicillium, Piromyces, Panerochaete, Pleurotus, Schizophyllum, Talaromyces, Thermoascus, Thielavia, Tolypocladium,* or *Trichoderma* strain, more preferably from an *Agraricus bisporus, Aspergillus aculeatus, Aspergillus awamori, Aspergillus foetidus, Aspergillus japonicus, Aspergillus nidulans, Aspergillus niger, Aspergillus sojae, Aspergillus tubigenis, Aspergillus oryzae, Aspergillus vadensis, Chrysosporum lucknowense, Humicola insolens, Humicola lanuginosa, Mucor miehei, Myceliophthora thermophila, Neurospora crassa, Penicillium purpurogenum, Penicillium chrysogenum, Pycnoporus cinnabarinus, Schizophyllum commune, Thielavia terrestris, Trichoderma harzianum, Trichoderma koningii, Trichoderma longibrachiatum, Trichoderma reesei,* or *Trichoderma viride* strain.

According to yet another preferred embodiment, the promoter sequences are obtained from a *Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, or Fusarium venenatum* strain.

It will be understood that for the aforementioned species, the invention encompasses the perfect and imperfect states, and other taxonomic equivalents, e.g., anamorphs, regardless of the species name by which they are known. Those skilled in the art will readily recognize the identity of appropriate equivalents. Strains of these species are readily accessible to the public in a number of culture collections, such as the American Type Culture Collection (ATCC), Deutsche Sammlung von Mikroorganismen und Zellkulturen GmbH (DSM), Centraalbureau Voor Schimmelcultures (CBS), and Agricultural Research Service Patent Culture Collection, Northern Regional Research Center (NRRL).

Furthermore, promoter sequences according to the invention may be identified and obtained from other sources including microorganisms isolated from nature (e.g, soil, composts, water, etc.) using the above-mentioned probes. Techniques for isolating microorganisms from natural habitats are well known in the art. The nucleic acid sequence may then be derived by similarly screening a genomic DNA library of another microorganism. Once a nucleic acid sequence encoding a promoter has been detected with the probe(s), the sequence may be isolated or cloned by utilizing techniques which are known to those of ordinary skill in the art (see, e.g., Sambrook et al., *supra).*

In the present invention, the promoter DNA sequence may also be a hybrid promoter comprising a portion of one or more promoters of the present invention; a portion of a promoter of the present invention and a portion of another known promoter, e.g., a leader sequence of one promoter and the transcription start site from the other promoter; or a portion of one or more promoters of the present invention and a portion of one or more other promoters. The other promoter may be any promoter sequence, which shows transcriptional activity in the fungal host cell of choice including a variant, truncated, and hybrid promoter, and may be obtained from genes encoding extracellular or intracellular polypeptides either homologous or heterologous to the host cell. The other promoter sequence may be native or foreign to the nucleic acid sequence encoding the polypeptide and native or foreign to the cell.

According to preferred a embodiment, important regulatory subsequences of the promoter identified can be fused to other 'basic' promoters to enhance their promoter activity (as for example described in Mol Microbiol. 1994 May;12(3):479-90. Regulation of the xylanase-encoding xlnA gene of Aspergillus tubigensis. de Graaff LH, van den Broeck HC, van Ooyen AJ, Visser J.).

Other examples of other promoters useful in the construction of hybrid promoters with the promoters of the present invention include the promoters obtained from the genes for *A. oryzae* TAKA amylase, *Rhizomucor miehei* aspartic proteinase, *A. niger* neutral alpha-amylase, *A. niger* acid stable alpha-amylase, *A. niger* or *Aspergillus awamori* glucoamylase (glaA), *A. niger* gpdA, *A. niger* glucose oxidase goxC, *Rhizomucor miehei* lipase, *A. oryzae* alkaline protease, *A. oryzae* triose phosphate isomerase, *A. nidulans* acetamidase, and *Fusarium oxysporum* trypsin-like protease (WO 96/00787), as well as the NA2-tpi promoter (a hybrid of the promoters from the genes for *A. niger* neutral alpha-amylase and *A. oryzae* triose phosphate isomerase), *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH2/GAP), and *Saccharomyces cerevisiae 3-*phosphoglycerate kinase, and mutant, truncated, and hybrid promoters thereof. Other useful promoters for yeast host cells are described by Romanoset al., 1992, Yeast 8: 423-488.

In the present invention, the promoter DNA sequence may or may not be a "tandem promoter". A "tandem promoter" is defined herein as two or more promoter sequences each of which is in operative association with a coding sequence and mediates the transcription of the coding sequence into mRNA.

The tandem promoter comprises two or more promoters of the present invention or alternatively one or more promoters of the present invention and one or more other known promoters, such as those exemplified above useful for the construction of hybrid promoters. The two or more promoter sequences of the tandem promoter may simultaneously promote the transcription of the nucleic acid sequence. Alternatively, one or more of the promoter sequences of the tandem promoter may promote the transcription of the nucleic acid sequence at different stages of growth of the cell or morphological different parts of the mycelia.

In the present invention, the promoter may be foreign to the coding sequence encoding a compound of interest and/or to the fungal host cell. A variant, hybrid, or tandem promoter of the present invention will be understood to be foreign to a coding sequence encoding a compound of interest, even if the wild-type promoter is native to the coding sequence or to the fungal host cell.

A variant, hybrid, or tandem promoter of the present invention has at least about 20%, preferably at least about 40%, more preferably at least about 60%, more preferably at least about 80%, more preferably at least about 90%, more preferably at least about 100%, even more preferably at least about 200%, most preferably at least about 300%, and most preferably at least about 400% of the promoter activity of a parental promoter, where the variant, hybrid or tandem promoter originates from.

### Coding sequences

In the present invention, the coding sequence in the DNA construct according to the invention may encode a polypeptide. The polypeptide may be any polypeptide having a biological activity of interest. The polypeptide may be homologous or heterologous to the host cell according to the invention. Preferably, the polypeptide is an enzyme.

The term "polypeptide" is not meant herein to refer to a specific length of the encoded product and, therefore, encompasses peptides, oligopeptides, and proteins.

The term "homologous gene" or "homologous polypeptide" is herein defined as a gene or polypeptide that is obtainable from a strain that belongs to the same species, including variants thereof, as does the strain actually containing the gene or polypeptide. Preferably, the donor and acceptor strain are the same. Fragments and mutants of genes or polypeptides are also considered homologous when the gene or polypeptide from which the mutants or fragments are derived is a homologous gene or polypeptide. Also non-native combinations of regulatory sequences and coding sequences are considered homologous as long as the coding sequence is homologous. It follows that the term heterologous herein refers to genes or polypeptides for which donor and acceptor strains do not belong to the same species or variants thereof.

The term "heterologous polypeptide" is defined herein as a polypeptide, which is not native to the fungal cell, a native polypeptide in which modifications have been made to alter the native sequence, or a native polypeptide whose expression is quantitatively altered as a result of a manipulation of the fungal cell by recombinant DNA techniques. For example, a native polypeptide may be recombinantly produced by, e.g., placing the coding sequence under the control of the promoter of the present invention to enhance expression of the polypeptide, to expedite export of a native polypeptide of interest outside the cell by use of a signal sequence, and to increase the copy number of a gene encoding the polypeptide normally produced by the cell.

According to a preferred aspect of the invention, the polypeptide is a peptide hormone or variant thereof, an enzyme, or an intracellular protein.

The intracellular polypeptide may be a protein involved in secretion process, a protein involved in a folding process, a peptide amino acid transporter, a glycosylation factor, a receptor or portion thereof, an antibody or portion thereof, or a reporter protein. Preferably, the intracellular protein is a chaperone or transcription factor. An example of this is described in Appl Microbiol Biotechnol. 1998 Oct;50(4):447-54 ("Analysis of the role of the gene bipA, encoding the major endoplasmic reticulum chaperone protein in the secretion of homologous and heterologous proteins in black Aspergilli. Punt PJ, van Gemeren IA, Drint-Kuijvenhoven J, Hessing JG, van Muijlwijk-Harteveld GM, Beijersbergen A, Verrips CT, van den Hondel CA). This can be used for example to improve the efficiency of a host cell as protein producer if this coding sequence, such as a chaperone or transcription factor, was known to be a limiting factor in protein production. Another preferred intracellular polypeptide is an intracellular enzyme, such as amadoriase, catalase, acyl-CoA oxidase, linoleate isomerase, trans-2-enoyl-ACP reductase, trichothecene 3-O-acetyltransferase, alcohol dehydrogenase, carnitine racemase, D-mandelate dehydrogenase, enoyl CoA hydratase, fructosyl amine oxygen oxidoreductase, 2-hydroxyhepta-2,4-diene-1,7-dioate isomerase, NADP-dependent malate dehydrogenase, oxidoreductase, quinone reductase. Other intracellular enzymes are ceramidases, epoxide hydrolases aminopeptidases, acylases, aldolase, hydroxylase, aminopeptidases.

According to another preferred aspect of the invention, the polypeptide is secreted extracellularly. Preferably, the extracellular polypeptide is an enzyme. Examples of extracellular enzymes are cellulases such as endoglucanases, β-glucanases, cellobiohydrolases or β-glucosidases; hemicellulases or pectinolytic enzymes such as xylanases, xylosidases, mannanases, galactanases, galactosidases, pectin methyl esterases, pectin lyases, pectate lyases, endo-polygalacturonases, exopolygalacturonases rhamnogalacturonases, arabanases, arabinofuranosidases, arabinoxylan hydrolases, galacturonases, lyases; amylolytic enzymes; phosphatases such as phytases, esterase such as lipases, proteolytic enzyme, such as proteases, peptidases, oxidoreductases such as oxidases, transferases, or isomerases; peroxidases such as ligninases.

The coding sequence comprised in the DNA construct according to the invention may also encode an enzyme involved in the synthesis of a primary or secondary metabolite, such as organic acids, carotenoids, (beta-lactam) antibiotics, and vitamins. Such metabolite may be considered as a biological compound according to the present invention.

The coding sequence encoding a polypeptide of interest may be obtained from any prokaryotic, eukaryotic, or other source. Preferably, the coding sequence and promoter associated with it are homologous to the host cell, resulting in a recombinant host cell being a self-clone.

According to an embodiment of the invention, the coding sequence in the DNA construct according to the invention may be a variant, optimized sequence comprising an optimized terminator sequence, such as for example described in WO2006 077258.

The coding sequence may be a partly synthetic nucleic acid sequence or an entirely synthetic nucleic acid sequence. The coding sequence may be optimized in its codon use, preferably according to the methods described in WO2006/077258 and/or WO2008/000632, which are herein incorporated by reference. WO2008/000632 addresses codon-pair optimization. Codon-pair optimisation is a method wherein the nucleotide sequences encoding a polypeptide have been modified with respect to their codon-usage, in particular the codon-pairs that are used, to obtain improved expression of the nucleotide sequence encoding the polypeptide and/or improved production of the encoded polypeptide. Codon pairs are defined as a set of two subsequent triplets (codons) in a coding sequence.

Alternatively, the coding sequence may code for the expression of an antisense RNA and/or an RNAi (RNA interference) construct. An example of expressing an antisense-RNA is shown in Appl Environ Microbiol. 2000 Feb;66(2):775-82. (Characterization of a foldase, protein disulfide isomerase A, in the protein secretory pathway of *Aspergillus niger.* Ngiam C, Jeenes DJ, Punt PJ, Van Den Hondel CA, Archer DB) or (Zrenner R, Willmitzer L, Sonnewald U. Analysis of the expression of potato uridinediphosphate-glucose pyrophosphorylase and its inhibition by antisense RNA. Planta. (1993);190(2):247-52.) Partial, near complete, or complete inactivation of the expression of a gene is useful for instance for the inactivation of genes controlling undesired side branches of metabolic pathways, for instance to increase the production of specific secondary metabolites such as (beta-lactam) antibiotics or carotenoids. Complete inactivation is also useful to reduce the production of toxic or unwanted compounds (chrysogenin in *Penicillium;* Aflatoxin in *Aspergillus:* MacDonald KD et al,: heterokaryon studies and the genetic control of penicillin and chrysogenin production in Penicillium chrysogenum. J Gen Microbiol. (1963) 33:375-83). Complete inactivation is also useful to alter the morphology of the organism in such a way that the fermentation process and down stream processing is improved.

Another embodiment of the invention relates to the extensive metabolic reprogramming or engineering of a fungal cell. Introduction of complete new pathways and/or modification of unwanted pathways will provide a cell specifically adapted for the production of a specific compound such as a protein or a metabolite.

In the methods of the present invention, when the coding sequence codes for a polypeptide, said polypeptide may also include a fused or hybrid polypeptide in which another polypeptide is fused at the N-terminus or the C-terminus of the polypeptide or fragment thereof. A fused polypeptide is produced by fusing a nucleic acid sequence (or a portion thereof) encoding one polypeptide to a nucleic acid sequence (or a portion thereof) encoding another polypeptide. Techniques for producing fusion polypeptides are known in the art, and include, ligating the coding sequences encoding the polypeptides so that they are in frame and expression of the fused polypeptide is under control of the same promoter(s) and terminator. The hybrid polypeptide may comprise a combination of partial or complete polypeptide sequences obtained from at least two different polypeptides wherein one or more may be heterologous to the fungal cell.

### Control sequences

The DNA constructs of the present invention may comprise one or more control sequences, in addition to the promoter DNA sequence, which direct the expression of the coding sequence in a suitable host cell under conditions compatible with the control sequences. Expression will be understood to include any step involved in the production of the polypeptide including, but not limited to, transcription, post-transcriptional modification, translation, post-translational modification, and secretion. One or more control sequences may be native to the coding sequence or to the host. Alternatively, one or more control sequences may be replaced with one or more control sequences foreign to the coding sequence for improving expression of the coding sequence in a host cell.

The term "control sequences" is defined herein to include all components, which are necessary or advantageous for the expression of a coding sequence, including the promoter according to the invention. Each control sequence may be native or foreign to the nucleic acid sequence encoding the polypeptide. Such control sequences include, but are not limited to, a leader, an optimal Kozak or translation initiation sequence (Kozak, 1991, J. Biol. Chem. 266:19867-19870) such as for example described in WO2006/077258, a polyadenylation sequence, a propeptide sequence, a signal peptide sequence, an upstream activating sequence, the promoter according to the invention including variants, fragments, and hybrid and tandem promoters derived thereof and a transcription terminator. At a minimum, the control sequences include transcriptional and translational stop signals and (part of) the promoter according to the invention. The control sequences may be provided with linkers for the purpose of introducing specific restriction sites facilitating ligation of the control sequences with the coding region of the coding sequence.

The control sequence may be a suitable transcription terminator sequence, i.e. a sequence recognized by a host cell to terminate transcription. The terminator sequence is in operative association with the 3' terminus of the coding sequence. Any terminator, which is functional in the host cell of choice may be used in the present invention.

Preferred terminators for filamentous fungal host cells are obtained from the genes for A. *oryzae* TAKA amylase, *A. niger* glucoamylase, *A. nidulans* anthranilate synthase, *A. niger* alpha-glucosidase, trpC gene, and *Fusarium oxysporum* trypsin-like protease.

Preferred terminators for yeast host cells are obtained from the genes for *Saccharomyces cerevisiae* enolase, *Saccharomyces cerevisiae* cytochrome C (CYC1), and *Saccharomyces cerevisiae* glyceraidehyde-3-phosphate dehydrogenase. Other useful terminators for yeast host cells are described by Romanos et al, 1992, supra.

The control sequence may also be a suitable leader sequence, i.e. a 5' untranslated region of a mRNA which is important for translation by the host cell. The leader sequence is in operative association with the 5' terminus of the nucleic acid sequence encoding the polypeptide. Any leader sequence that is functional in the host cell of choice may be used in the present invention.

Preferred leaders for filamentous fungal host cells are obtained from the genes for *A. oryzae* TAKA amylase, *A. nidulans* triose phosphateisomerase and *A. niger* glaA.

Suitable leaders for yeast host cells are obtained from the genes for *Saccharomyces cerevisiae* enolase (ENO-1), *Saccharomyces cerevisiae 3-*phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

The control sequence may also be a polyadenylation sequence, a sequence in operative association with the 3' terminus of the nucleic acid sequence and which, when transcribed, is recognized by the host cell as a signal to add polyadenosine residues to transcribed mRNA. Any polyadenylation sequence, which is functional in the host cell of choice may be used in the present invention.

Preferred polyadenylation sequences for filamentous fungal host cells are obtained from the genes for *A. oryzae* TAKA amylase, *A. niger* glucoamylase, *A. nidulans* anthranilate synthase, *Fusarium oxysporum* trypsin-like protease, and *A. niger* alpha-glucosidase.

Useful polyadenylation sequences for yeast host cells are described by Guo and Sherman, 1995, Molecular Cellular Biology 15: 5983-5990.

The control sequence may also be a signal peptide coding region that codes for an amino acid sequence linked to the amino terminus of a polypeptide and directs the encoded polypeptide into the cell's secretory pathway. The 5' end of the coding sequence of the nucleic acid sequence may inherently contain a signal peptide coding region naturally linked in translation reading frame with the segment of the coding region which encodes the secreted polypeptide. Alternatively, the 5' end of the coding sequence may contain a signal peptide coding region which is foreign to the coding sequence. The foreign signal peptide coding region may be required where the coding sequence does not naturally contain a signal peptide coding region. Alternatively, the foreign signal peptide coding region may simply replace the natural signal peptide coding region in order to enhance secretion of the polypeptide. However, any signal peptide coding region which directs the expressed polypeptide into the secretory pathway of a host cell of choice may be used in the present invention.

Examples of suitable signal peptide coding regions for filamentous fungal host cells are the signal peptide coding regions obtained from the genes for *A. oryzae* TAKA amylase, *A. niger* neutral amylase, *A. ficuum* phytase, *A. niger* glucoamylase, *A. niger* endoxylanase, *Rhizomucor miehei* aspartic proteinase, *Humicola insolens* cellulase, and *Humicola lanuginosa* lipase.

Useful signal peptides for yeast host cells are obtained from the genes for *Saccharomyces cerevisiae* alpha-factor and *Saccharomyces cerevisiae* invertase. Other useful signal peptide coding regions are described by Romanoset al., 1992, supra.

The control sequence may also be a propeptide coding region that codes for an amino acid sequence positioned at the amino terminus of a polypeptide. The resultant polypeptide is known as a proenzyme or propolypeptide (or a zymogen in some cases). A propolypeptide is generally inactive and can be converted to a mature active polypeptide by catalytic or autocatalytic cleavage of the propeptide from the propolypeptide. The propeptide coding region may be obtained from the genes for *Bacillus subtilis* alkaline protease (aprE), *Bacillus subtilis* neutral protease (nprT), *Saccharomyces cerevisiae* alpha-factor, *Rhizomucor miehei* aspartic proteinase, *Myceliophthora thermophila* laccase (WO 95/33836) and *A. niger* endoxylanase (endo1).

Where both signal peptide and propeptide regions are present at the amino terminus of a polypeptide, the propeptide region is positioned next to the amino terminus of a polypeptide and the signal peptide region is positioned next to the amino terminus of the propeptide region.

It may also be desirable to add regulatory sequences, which allow gene amplification. Examples of regulatory sequences are in eukaryotic systems, are the dihydrofolate reductase genes, which iare amplified in the presence of methotrexate, and the metallothionein genes, which are amplified with heavy metals.

Important can be removal of creA binding sites (carbon catabolite repression as described earlier in EP 673 429), change of pacC and areA (for pH and nitrogen regulation).

### Expression vectors

The present invention also relates to recombinant expression vectors comprising a DNA construct (comprising a coding sequence in operative association with a promoter DNA sequence according to the invention). Optionally, the at least two DNA constructs of the present invention (comprising a coding sequence in operative association with a promoter DNA sequence) are comprised in a single DNA construct, which single DNA construct is comprised in a recombinant expression vector.

Preferably at least one DNA construct of the present invention (comprising a coding sequence in operative association with a promoter DNA sequence) is present on a vector. The vector is introduced into a host cell so that it is maintained as a chromosomal integrant and/or as a self-replicating extra-chromosomal vector.

The recombinant expression vector may be any vector (e.g., a plasmid or virus), which can be conveniently subjected to recombinant DNA procedures and can bring about the expression of the coding sequence. The choice of the vector will typically depend on the compatibility of the vector with the host cell into which the vector is to be introduced. The vectors may be linear or closed circular plasmids. The skilled person knows, using general knowledge in the art, how to select a suitable and convenient vector.

The vector may be an autonomously replicating vector, i.e., a vector, which exists as an extrachromosomal entity, the replication of which is independent of chromosomal replication, e.g., a plasmid, an extrachromosomal element, a minichromosome, or an artificial chromosome. For autonomous replication, the vector may comprise an origin of replication enabling the vector to replicate autonomously in the host cell in question. Examples of origins of replication for use in a yeast host cell are the 2 micron origin of replication, ARS1, ARS4, the combination of ARS1 and CEN3, and the combination of ARS4 and CEN6. The origin of replication may be one having a mutation which makes its functioning temperature-sensitive in the host cell (see, e.g., Ehrlich, 1978, Proceedings of the National Academy of Sciences USA 75:1433). An example of an autonomously maintained cloning vector in a filamentous fungus is a cloning vector comprising the AMA1-sequence. AMA1 is a 6.0-kb genomic DNA fragment isolated from *A. nidulans,* which is capable of Autonomous Maintenance in *Aspergillus* (see e.g. Aleksenko and Clutterbuck (1997), Fungal Genet. Biol. 21: 373-397).

Altematively, the vector may be one which, when introduced into the host cell, is integrated into the genome and replicated together with the chromosome(s) into which it has been integrated. An example of such integrative system is described in EP0357127B1. Furthermore, a single vector or plasmid or two or more vectors or plasmids which together contain the total DNA to be introduced into the genome of the host cell, or a transposon may be used.

The vectors of the present invention preferably contain one or more selectable markers, which permit easy selection of transformed cells. The host may be co-transformed with at least two vectors, one comprising the selection marker. A selectable marker is a gene the product of which provides for biocide or viral resistance, resistance to heavy metals, prototrophy to auxotrophs, and the like. Suitable markers for yeast host cells are ADE2, HIS3, LEU2, LYS2, MET3, TRP1, and URA3. Selectable markers for use in a filamentous fungal host cell include, but are not limited to, amdS (acetamidase), argB (ornithine carbamoyltransferase), bar (phosphinothricin acetyltransferase), hygB (hygromycin phosphotransferase), niaD (nitrate reductase), pyrG (orotidine-5'-phosphate decarboxylase), sC (sulfate adenyltransferase), trpC (anthranilate synthase), as well as equivalents thereof. Marker conferring resistance against e.g. phleomycin, hygromycin B or G418 can also be used. Preferred for use in an *Aspergillus* cell are the amdS and pyrG genes of *A. nidulans* or *A. oryzae* and the bar gene of *Streptomyces hygroscopicus.* The amdS marker gene is preferably used applying the technique described in EP 635 574 or WO 97/0626, which enables the development of selection marker free recombinant hosts cells that can be re-transformed usinf the same selection marker gene. A preferred selection marker gene is the *A.nidulans amdS* coding sequence fused to the *A.nidulans gpdA* promoter (EP635 574). AmdS genes from other filamentous fungus may also be used (WO 97/06261).

For integration into the host cell genome, the vector may rely on the promoter sequence and/or coding sequence encoding the polypeptide or any other element of the vector for stable integration of the vector into the genome by homologous or non-homologous recombination. Alternatively, the vector may contain additional nucleic acid sequences for directing integration by homologous recombination into the genome of the host cell. The additional nucleic acid sequences enable the vector to be integrated into the host cell genome at a precise location(s) in the chromosome(s). To increase the likelihood of integration at a precise location, the integration elements should preferably contain a sufficient number of nucleic acids, preferably at least 30bp, preferably at least 50 bp, preferably at least 0.1kb, even preferably at least 0.2kb, more preferably at least 0.5 kb, even more preferably at least 1 kb, most preferably at least 2 kb, which share a high percentage of identity with the corresponding target sequence to enhance the probability of homologous recombination. Preferably, the efficiency of targeted integration into the genome of the host cell, i.e. integration in a predetermined target locus, is increased by augmented homologous recombination abilities of the host cell. Such phenotype of the cell preferably involves a deficient ku70 gene as described in WO2005/095624. WO2005/095624 discloses a preferred method to obtain a filamentous fungal cell comprising increased efficiency of targeted integration. The integration elements may be any sequence that is homologous with the target sequence in the genome of the host cell. Furthermore, the integration elements may be non-encoding or encoding nucleic acid sequences. In order to promote targeted integration, the cloning vector is preferably linearized prior to transformation of the host cell. Linearization is preferably performed such that at least one but preferably either end of the cloning vector is flanked by sequences homologous to the target locus.

Preferably, the integration elements in the cloning vector that are homologous to the target locus are derived from a highly expressed locus, meaning that they are derived from a gene which is capable of high expression level in the fungal host cell. A gene capable of high expression level, i.e. a highly expressed gene, is herein defined as a gene whose mRNA can make up at least 0.5% (w/w) of the total cellular mRNA, e.g. under induced conditions, or alternatively, a gene whose gene product can make up at least 1% (w/w) of the total cellular protein, or, in case of a secreted gene product, can be secreted to a level of at least 0.1 g/l (as described in EP 357 127 B1). A number of preferred highly expressed fungal genes are given by way of example: the amylase, glucoamylase, alcohol dehydrogenase, xylanase, glyceraldehyde-phosphate dehydrogenase or cellobiohydrolase genes from *Aspergilli* or *Trichoderma.* Most preferred highly expressed genes for these purposes are a glucoamylase gene, preferably an *A. niger* glucoamylase gene, an *A. oryzae* TAKA-amylase gene, an *A. nidulans gpdA* gene, the locus of a sequence selected from the set of SEQ ID NO's:1 to 4 and 13 to 55 or the locus of a gene listed in Table 1, the *A. niger* locus of a sequence selected from the set of SEQ ID NO's:13 to 55 or the *A. niger* locus of a gene listed in Table 1, or a *Trichoderma reesei* cellobiohydrolase gene.

Alternatively, the vector may be integrated into the genome of the host cell by non-homologous recombination.

More than one copy of a nucleic acid sequence encoding a polypeptide may be inserted into the host cell to increase production of the gene product. This can preferably be performed by integrating into its genome copies of the DNA sequence, more preferably by targeting the integration of the DNA sequence at a highly expressed locus, for example at a glucoamylase locus or at the locus of a sequence selected from the set of SEQ ID NO's:1 to 4 and 13 to 55 or at the locus of a gene listed in Table 1. Alternatively, this can be performed by including an amplifiable selectable marker gene with the nucleic acid sequence where cells containing amplified copies of the selectable marker gene, and thereby additional copies of the nucleic acid sequence, can be selected for by cultivating the cells in the presence of the appropriate selectable agent. To increase the number of copies of the DNA sequence to be over expressed even more, the technique of gene conversion as described in WO98/46772 can be used.

The procedures used to ligate the elements described above to construct the recombinant expression vectors of the present invention are well known to one skilled in the art (see, e.g., Sambrook et al., *supra).*

### Transformation

The introduction of an expression vector or a nucleic acid construct into a cell is performed using commonly known techniques. It may involve a process consisting of protoplast formation, transformation of the protoplasts, and regeneration of the cell wall in a manner known per se. Suitable procedures for transformation of *Aspergillus* cells are described in EP 238 023 and Yelton et al., 1984, Proceedings of the National Academy of Sciences USA 81 : 1470-1474. Suitable procedures for transformation of *Aspergillus* and other filamentous fungal host cells using *Agrobacterium tumefaciens* are described in e.g. Nat Biotechnol. 1998 Sep;16(9):839-42. Erratum in: Nat Biotechnol 1998 Nov;16(11):1074. *Agrobacterium* tumefaciens-mediated transformation of filamentous fungi. de Groot MJ, Bundock P, Hooykaas PJ, Beijersbergen AG. Unilever Research Laboratory Vlaardingen, The Netherlands. A suitable method of transforming *Fusarium* species is described by Malardier et al., 1989, Gene 78 : 147156 or in WO 96/00787. Other methods can be applied such as a method using biolistic transformation as described in: Biolistic transformation of the obligate plant pathogenic fungus, Erysiphe graminis f.sp. hordei. Christiansen SK, Knudsen S, Giese H. Curr Genet. 1995 Dec; 29(1):100-2. Yeast may be transformed using the procedures described by Becker and Guarente, In Abelson, J. N. and Simon, M. I., editors, Guide to Yeast Genetics and Molecular Biology, Methods in Enzymology, Volume 194, pp 182-187, Academic Press, Inc., New York; Ito et al., 1983, Journal of Bacteriology 153: 163; and Hinnen et al., 1978, Proceedings of the National Academy of Sciences USA 75: 1920.

The invention further relates to a method to prepare the recombinant host cell according to the invention, said method comprising:
(a) providing at least two DNA constructs, each DNA construct comprising a coding sequence in operative association with a promoter DNA sequence, wherein the at least two DNA constructs comprise at least two distinct promoter DNA sequences and wherein the coding sequences comprised in said DNA constructs encode related polypeptides,
(b) providing a suitable host cell, and
(c) transforming said host cell with said DNA constructs.

Optionally, said two DNA constructs, are comprised in a single construct. Optionally, at least one DNA construct is present on a vector.

According to a preferred embodiment, the transformation step is performed by at least two separate transformation events. A transformation event is herein defined as the procedure of transformation by introduction of a DNA construct in a parental host cell and isolation of transformed offspring of the parental cell.

### Expression

The invention further relates to a method for expression of a coding sequence in a suitable host cell. The method comprising the following steps:
(a) providing at least two DNA constructs, each DNA construct comprising a coding sequence in operative association with a promoter DNA sequence, wherein the at least two DNA constructs comprise at least two distinct promoter DNA sequences and wherein the coding sequences comprised in said DNA constructs encode related polypeptides,
(b) providing a suitable host cell,
(c) transforming said host cell with said DNA constructs,
(d) culturing said host cell under conditions conducive to expression of the coding sequence.

Optionally, said two DNA constructs, are comprised in a single construct. Optionally, at least one DNA construct is present on a vector.

According to a preferred embodiment, the transformation step is performed by at least two separate transformation events.

The invention further relates to a method for expression of coding sequence by culturing a recombinant host cell according to the invention under conditions conducive to expression of the coding sequence.

### Production

The invention further relates to a method for the production of a polypeptide, comprising:
(a) culturing a recombinant host cell according to the invention under conditions conducive to expression of the polypeptide,
(b) optionally recovering the polypeptide from the culture broth, and
(c) optionally purifying the polypeptide.

The invention also relates to a method for the production of a metabolite, comprising:
(a) culturing a recombinant host cell according to the invention under conditions conducive to production of the metabolite,
(b) optionally recovering the metabolite from the culture broth, and
(c) optionally purifying the metabolite.

In the production methods of the present invention, the cells are cultivated in a nutrient medium suitable for production of the polypeptide or metabolite using methods known in the art. Examples of cultivation methods which are not construed to be limitations of the invention are submerged fermentation, surface fermentation on solid state and surface fermentation on liquid substrate. For example, the cell may be cultivated by shake flask cultivation, small-scale or large-scale fermentation (including continuous, batch, fed-batch, or solid state fermentations) in laboratory or industrial fermentors performed in a suitable medium and under conditions allowing the coding sequence to be expressed and/or the polypeptide to be isolated. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection). If the polypeptide or metabolite is secreted into the nutrient medium, the polypeptide or metabolite can be recovered directly from the medium. If the polypeptide or metabolite is not secreted, it can be recovered from cell lysates.

The polypeptides may be detected using methods known in the art that are specific for the polypeptides. These detection methods may include use of specific antibodies, formation of an enzyme product, or disappearance of an enzyme substrate.

The resulting polypeptide or metabolite may be recovered by methods known in the art. For example, the polypeptide or metabolite may be recovered from the nutrient medium by conventional procedures including, but not limited to, centrifugation, filtration, extraction, spray-drying, evaporation, or precipitation.

Polypeptides may be purified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), SDS-PAGE, or extraction (see, e.g., Protein Purification, J.-C. Janson and Lars Ryden, editors, VCH Publishers, New York, 1989).

### Modulation of expression

The present invention also relates to nucleic acid constructs comprising a promoter DNA sequence comprising a nucleotide sequence selected from the set consisting of: SEQ ID NO's:1 to 4 and 13 to 55 and the promoter DNA sequences of the genes listed in Table 1, for altering the expression of a coding sequence encoding a compound of interest, which is endogenous to a fungal host cell. The constructs may contain the minimal number of components necessary for altering expression of the endogenous gene.

According to a preferred embodiment, the nucleic acid constructs contain (a) a targeting sequence, (b) a promoter DNA sequence comprising a nucleotide sequence selected from the set consisting of: a sequence of SEQ ID NO's:1 to 4 and 13 to 55 and the promoter DNA sequences of the genes listed in Table 1, (c) an exon, and (d) a splice-donor site. Upon introduction of the nucleic acid construct into a cell, the construct integrates by homologous recombination into the cellular genome at the endogenous gene site. The targeting sequence directs the integration of elements (a)-(d) into the endogenous gene such that elements (b)-(d) are in operative association with the endogenous gene.

According to another embodiment, the nucleic acid constructs contain (a) a targeting sequence, (b) a promoter DNA sequence comprising a nucleotide sequence selected from the set consisting of: a sequence of SEQ ID NO's:1 to 4 and 13 to 55 and the promoter DNA sequences of the genes listed in Table 1, (c) an exon, (d) a splice-donor site, (e) an intron, and (f) a splice-acceptor site, wherein the targeting sequence directs the integration of elements (a)-(f) such that elements (b)-(f) are in operative association with the endogenous gene. However, the constructs may contain additional components such as a selectable marker. The selectable markers that can be used are those described earlier herein.

In both embodiments, the introduction of these components results in production of a new transcription unit in which expression of the endogenous gene is altered. In essence, the new transcription unit is a fusion product of the sequences introduced by the targeting constructs and the endogenous gene. According to an embodiment in which the endogenous gene is altered, the gene is activated. According to this embodiment, homologous recombination is used to replace, disrupt, or disable the regulatory region normally associated with the endogenous gene of a parent cell through the insertion of a regulatory sequence, which causes the gene to be expressed at higher levels than evident in the corresponding parent cell.

The targeting sequence can be within the endogenous gene, immediately adjacent to the gene, within an upstream gene, or upstream of and at a distance from the endogenous gene. One or more targeting sequences can be used. For example, a circular plasmid or DNA fragment preferably employs a single targeting sequence, while a linear plasmid or DNA fragment preferably employs two targeting sequences.

The constructs further contain one or more exons of the endogenous gene. An exon is defined as a DNA sequence, which is copied into RNA and is present in a mature mRNA molecule such that the exon sequence is in-frame with the coding region of the endogenous gene. The exons can, optionally, contain DNA, which encodes one or more amino acids and/or partially encodes an amino acid. Alternatively, the exon contains DNA which corresponds to a 5' non-encoding region. Where the exogenous exon or exons encode one or more amino acids and/or a portion of an amino acid, the nucleic acid construct is designed such that, upon transcription and splicing, the reading frame is in-frame with the coding region of the endogenous gene so that the appropriate reading frame of the portion of the mRNA derived from the second exon is unchanged. The splice-donor site of the constructs directs the splicing of one exon to another exon. Typically, the first exon lies 5' of the second exon, and the splice-donor site overlapping and flanking the first exon on its 3' side recognizes a splice-acceptor site flanking the second exon on the 5' side of the second exon. A splice-acceptor site, like a splice-donor site, is a sequence, which directs the splicing of one exon to another exon. Acting in conjunction with a splice-donor site, the splicing apparatus uses a splice-acceptor site to effect the removal of an intron.

A preferred strategy for altering the expression of a given DNA sequence comprises the deletion of the given DNA sequence and/or replacement of the endogenous promoter sequence of the given DNA sequence by a modified promoter DNA sequence, such as a promoter according to the invention. The deletion and the replacement are preferably performed by the gene replacement technique described in EP 0 357 127. The specific deletion of a gene and/or promoter sequence is preferably performed using the amdS gene as selection marker gene as described in EP 635 574. By means of counterselection on fluoracetamide media as described in EP 635 574, the resulting strain is selection marker free and can be used for further gene modifications.

Alternatively or in combination with other mentioned techniques, a technique based on *in vivo* recombination of cosmids in E. *coli* can be used, as described in: A rapid method for efficient gene replacement in the filamentous fungus A. nidulans (2000) Chaveroche, M-K., Ghico, J-M. and d'Enfert C; Nucleic acids Research, vol 28, no 22. This technique is applicable to other filamentous fungi like for example *A. niger.*

The invention described and claimed herein is not to be limited in scope by the specific embodiments herein disclosed, since these embodiments are intended as illustrations of several aspects of the invention. Any equivalent embodiments and/or combinations of preferred aspects of the invention are intended to be within the scope of this invention. Indeed, various modifications of the invention in addition to those shown and described herein will become apparent to those skilled in the art from the foregoing description. Such modifications are also intended to fall within the scope of the appended claims. In the case of conflict, the present disclosure including definitions will control.

### Examples

### Experimental information

### Strains

WT 1: This *A. niger* strain is used as a wild-type strain. This strain is deposited at the CBS Institute under the deposit number CBS 513.88.

WT 2: This *A. niger* strain is a WT 1 strain comprising a deletion of the gene encoding glucoamylase (glaA). WT 2 was constructed by using the "MARKER-GENE FREE" approach as described in EP 0 635 574 B1. In this patent it is extensively described how to delete *gla*A specific DNA sequences in the genome of CBS 513.88. The procedure resulted in a MARKER-GENE FREE Δ*gla*A recombinant *A. niger* CBS513.88 strain, possessing finally no foreign DNA sequences at all.

BFRM 44: This *Pycnoporus cinnabarinus* strain is used in example 9 and is available from the Banque de Resources Fongiques de Marseille, Marseiile, France under deposit number BRFM44.

### Glucoamylase activity assay

The glucoamylase activity was determined using p-Nitrophenyl α-D-glucopyranoside (Sigma) as described in WO 98/46772.

### Example 1: Construction of a DNA construct comprising a promoter according to the invention in operative association with a coding sequence.

This example describes the construction of an expression construct under control of a promoter according to the invention. The coding sequence or reporter construct used here is the *gla*A gene encoding the *A. niger* CBS 513.88 glucoamylase enzyme. Glucoamylase is used as the reporter enzyme to be able to measure the activity of the promoter according to the invention.

### 1.1 Description of an integrative glucoamylase expression vector (pGBTOPGLA)

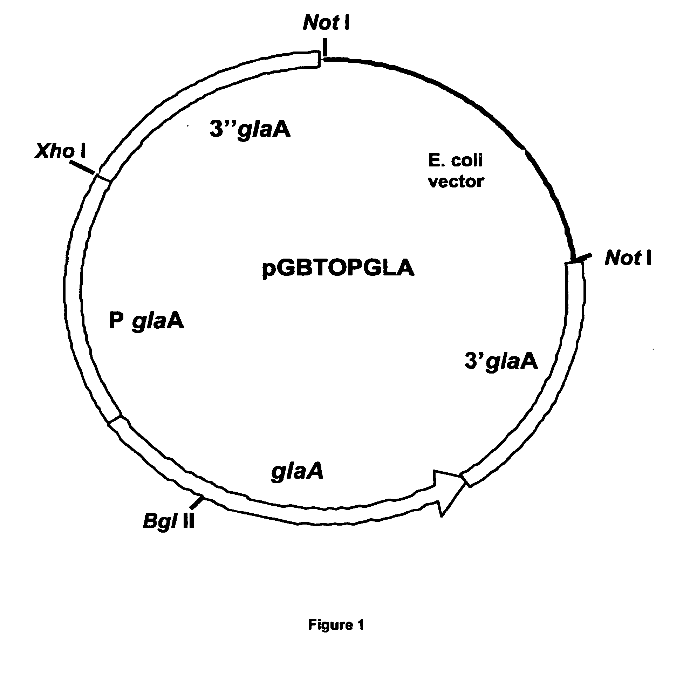

The glucoamylase promoter and the glucoamylase encoding gene *gla*A from A. *niger* were cloned into the expression vector pGBTOP-8, which is described in WO99/32617. The cloning was performed according known principles and to routine cloning techniques and yielded plasmid pGBTOPGLA (see Figure 1).In essence, this expression vector comprises the glucoamylase promoter, coding sequence and terminator region, flanked by the 3' and 3" *gla*A targeting sites in an E. *coli* vector.

### 1.2 Construction of an integrative glucoamylase expression vector with a multiple, cloning site MCS (pGBTOPGLA-2)

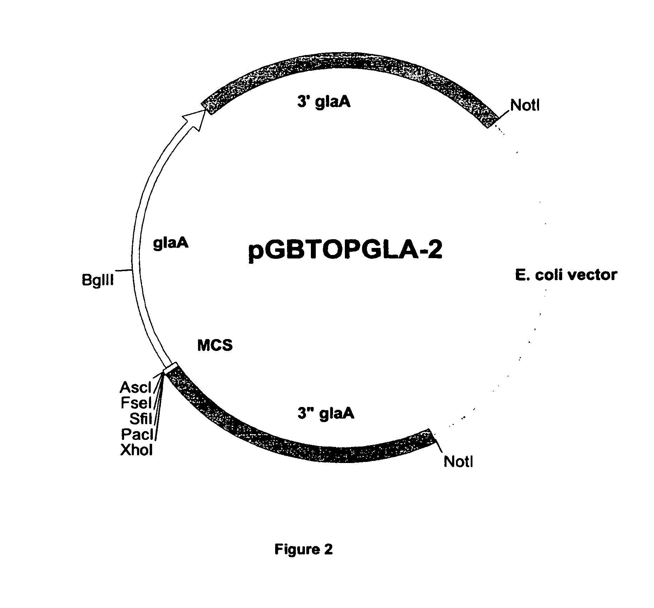

With PCR methods known to the skilled person (Sambrook et al., *supra),* using 1ng of pGBTOPGLA as template, a PCR fragment was generated containing part of the *gla*A coding sequence and flanked with *Xho*I and *Bgl*II restriction sites. This fragment was digested with *Xho*I and *Bgl*II and introduced in *Xho*I and *Bgl*II digested vector pGBTOPGLA, resulting in vector pGBTOPGLA-2 (see Figure 2). The sequence of the introduced PCR fragment comprising a MCS and part of the *gla*A coding sequence was confirmed by sequence analysis.

### 1.3 Construction of an integrative expression vector with the promoter according to the invention in operative association with the glucoamylase coding sequence (pGBTOPGLA-3)

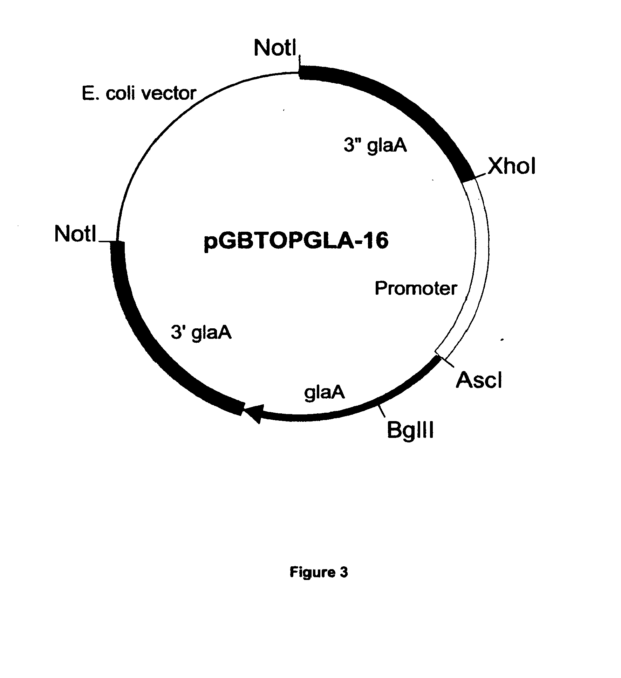

Genomic DNA of strain CBS513.88 was sequenced and analysed. With PCR methods known to the skilled person (Sambrook et al., *supra)* using:
- a 5'-PCR primer containing nucleotides 1 to 20 of a sequence selected from the set of: SEQ ID NO's:13 to 55 and the promoter DNA sequences of the genes listed in Table 1, flanked at the 5'- end with a Xhol restriction site, and
- a 3'-PCR primer containing nucleotides 1477 to 1497 (in some cases around 2000 nucleotides) of the sequence selected immediately here above, flanked at the 3'-end with an *Asc*I restriction site, and
- genomic DNA of strain CBS513.88 as template,
appropriate restriction sites were attached to the promoter according to the invention by PCR amplification. The resulting fragments of approximately 1.5-2 kb, comprising the sequence of one of SEQ ID NO's:13 to 55 or of one of the promoter DNA sequences of the genes listed in Table 1, were digested with *Asc*I and *Xho*I and introduced in an *Asc*I and Xhol digested vector pGBTOPGLA-2, resulting for example in vector pGBTOPGLA-16 (for general layout of vectors see Figure 3) and other vectors as for example listed in Table 2.

**Table 2: Expression constructs with a number of different promoters for glucoamylase glaA expression in A. niger**

| **Plasmid name** | **Promoter** | **SEQ ID NO:** | **Coding sequence** |
|---|---|---|---|
| pGBTOPGLA-1 | glaA | - | GlaA |
| pGBTOPGLA-16 | glaA with modified translation initiation site | 16 | GlaA |
| pGBTOPGLA-17 | amyB with modified translation initiation site | 17 | GlaA |
| pGBTOPGLA-18 | gpdA | 18 | GlaA |
| pGBTOPGLA-19 | An18g04220 | 19 | GlaA |

The sequences of the introduced PCR fragments comprising the promoters according to the invention were confirmed by sequence analysis.

### Example 2. Fungal host cell transformed with the DNA construct

In the following example, an expression construct is introduced in a fungal host cell by transformation.

In order to introduce additional pGBTOPGLA-1, and pGBTOPGLA-16 to -19 vectors in WT1, to increase the glaA copy number, a transformation and subsequent transformant selection was carried out as described in WO98/46772 and WO99/32617. In brief, linear DNA of the above mentioned pGBTOPGLA vectors was isolated and co-transformed with an amdS selectable marker-gene containing vector, which is designated pGBAAS-1 (constructed as described in EP 635574B1). Both types of vectors comprise two DNA domains homologous to the *gla*A locus of *A. niger* host strain to direct targeting to the 3'-3" terminator locus of *gla*A in WT 1. Transformants were selected on acetamide media and colony purified according standard procedures. Spores were plated on fluoro-acetamide media to select strains, which lost the *amd*S marker. Growing colonies were diagnosed for integration at the *gla*A locus and copy number. Transformants of pGBTOPGLA-1, -16, -17, -18 and -19 with an additional copy of glaA were selected.

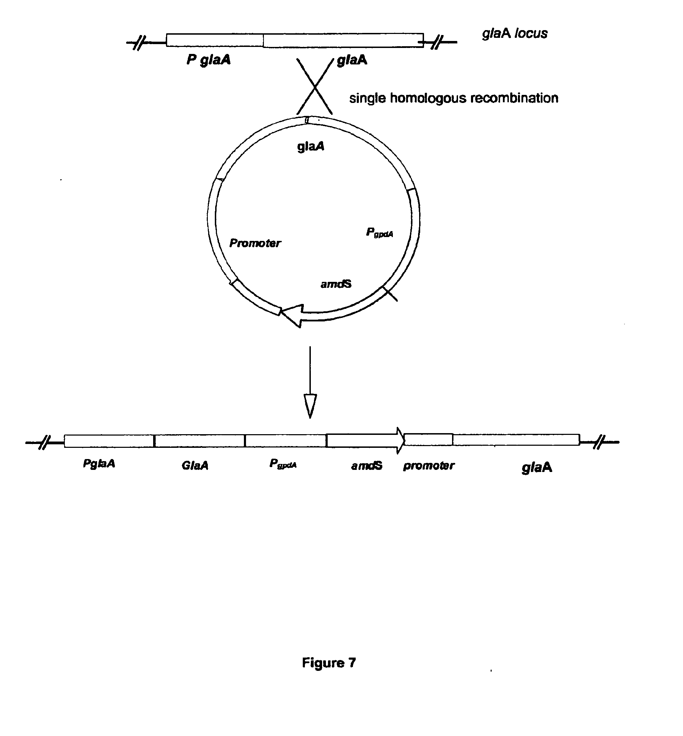

Alternatively, a circular construct as depicted in Figure 7 can be used to integrate into the genome at the *gla*A coding sequence of WT1. Additionally, the selectable marker gene and the gene of interest controlled by a promoter according to the invention can be on a single construct. Example of this vector and how to use in transformation can be found in WO99/32617.

### Example 3. Introduction of an additional glaA gene under control of a promoter according to the invention in the fungal host cell

To alter and increase the expression level of a given gene in a host cell, additional copies of glucoamylase operatively linked to a promoter according to the invention can be introduced in a given host cell. In this example, a promoter of the invention operatively linked with the glaA coding sequence is introduced next to endogenously present glucoamylase encoding *gla*A gene in a fungal host cell. In the following example, the activity of a promoter of the invention is measured by measuring the activity of the reporter (glucoamylase) in selected transformants. Therefore, the glucoamylase activity is determined in the culture broth.

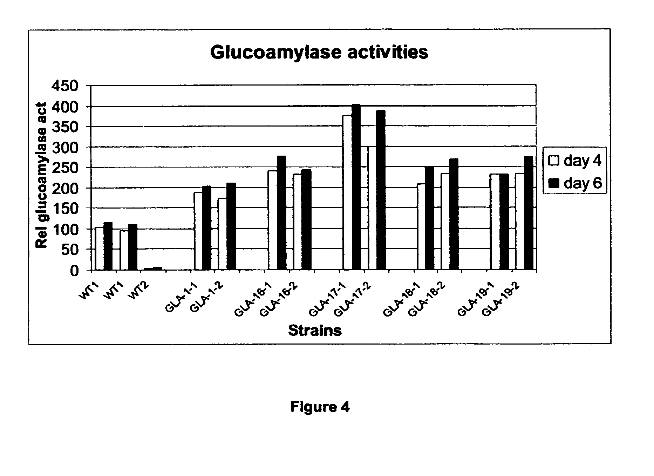

The selected pGBTOPGLA-1, 16, -17, -18, -19 transformants of WT 1 and both strains WT 1 and WT 2 were used to perform shake flask experiments in 100 ml of the medium as described in EP 635 574 B1 at 34°C and 170 rpm in an incubator shaker using a 500 ml baffled shake flask. After 4 and 6 days of fermentation, samples were taken to determine the glucoamylase activity, as described above. The glucoamylase activity in the selected pGBTOPGLA-1, -16, -17, -18, -19 transformants of WT1 was increased compared to WT 1 after either four or six days of culture (Figure 4). Surprisingly, it was shown that introducing a second *gla*A gene copy under the control of another promoter than *gla*A increased expression more than could be expected based on the copy number (more than 200%). Also using a modified *gla*A or *amy*B promoter, increases larger than expected based on copy number were identified.

Additionally, strains with multiple copies of the pGBTOPGLA constructs showed that the production per gene copy was constant until at least 5 copies for all vectors (data not shown).

### Example 4. Construction of a promoter replacement construct pGBDEL-PGLAA comprising a promoter according to the invention.

To alter the expression level of a given gene in a host cell, a promoter according to the invention can replace the endogenous promoter of said given gene. In this example, a promoter according to the invention replaces the promoter of the glucoamylase encoding glaA gene in a fungal host cell. Example 4, 5 and 6 describe a number of different steps in this process.

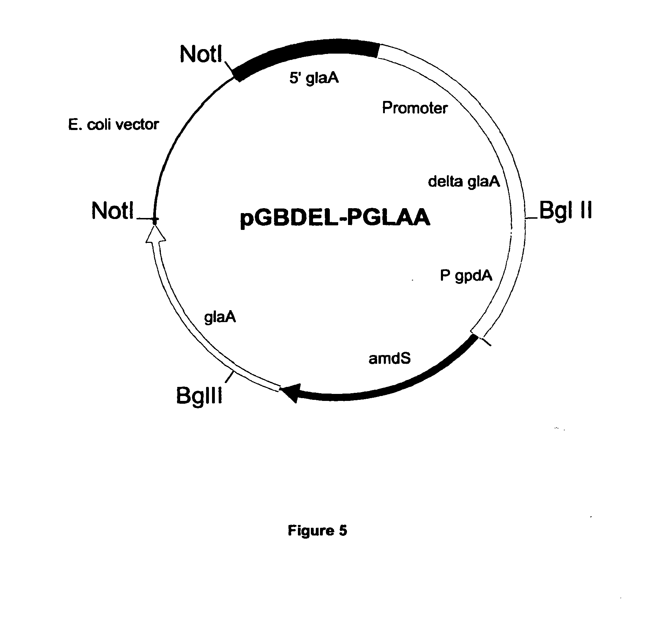

A replacement vector for the glucoamylase promoter was designed according to known principles and constructed according to routine cloning procedures (see Figure 5). In essence, the *gla*A promoter replacement vector pGBDEL-PGLAA comprises approximately 1000 bp flanking regions of the *gla*A promoter sequence to be replaced by a promoter according to the invention through homologous recombination at the predestined genomic locus. The flanking regions used here (see Figure 5) are a 5' upstream region of the *gla*A promoter and part of the *gla*A coding sequence. In addition, the replacement vector contains the *A. nidulans* bi-directional *amd*S selection marker, in-between direct repeats. The direct repeats used in this example are part of the *gla*A coding sequence. The general design of these deletion vectors were previously described in EP635574B1 and WO 98/46772.

### Example 5. Replacement of the glaA promoter by a promoter according to the invention in the fungal host cell.

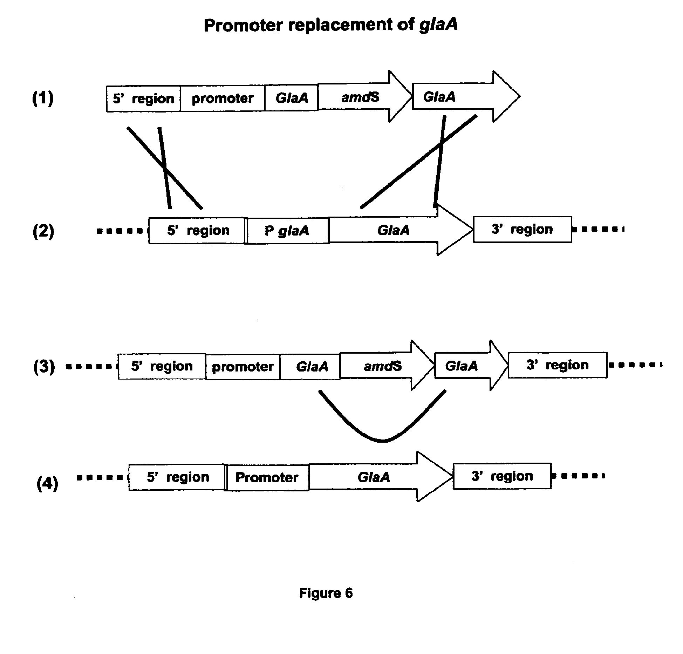

Linear DNA of *Not*I-digested deletion vector pGBDEL-PGLAA was isolated and used to transform WT 1 (CBS513.88). This linear DNA can integrate into the genome at the *gla*A locus, thus substituting the *gla*A promoter region with the construct containing *amd*S and a promoter according to the invention (see Figure 6). Transformants were selected on acetamide media and colony purified according to standard procedures. Growing colonies were diagnosed by PCR for integration at the *gla*A locus. Deletion of the *gla*A promoter was detectable by amplification of a band, with a size specific for the promoter according to the invention and loss of a band specific for the *gla*A promoter. Spores were plated on fluoro-acetamide media to select strains, which lost the *amd*S marker. Candidate strains were tested using Southern analysis for proper deletion of the glucoamylase promoter and replacement by a promoter according to the invention. Strains dPGLAA were selected as representative strains with the *gla*A promoter replaced by the promoter according to the invention and having a restored functional *gla*A coding sequence (see Figure 6).

### Example 6. Production of the glucoamylase polypeptide encoded by the glaA coding sequence under control of a replaced promoter according to the invention, in the fungal host cell.

The selected dPGLAA strains (proper pGBDEL-PGLAA transformants of WT 1, isolated in example 5) and strain WT 1 were used to perform shake flask experiments in 100 ml of the medium as described in EP 635 574 B1 at 34°C and 170 rpm in an incubator shaker using a 500 ml baffled shake flask. After 4 and 6 days of fermentation, samples were taken to determine the glucoamylase activity. The glucoamylase activity in the selected dpGLAA transformants of WT1 was altered compared to the one measured for WT 1 after either 4 or six days of fermentation.

### Example 7. Integration of glaA genes under control of multiple promoters according to the invention in the fungal host cell.

To alter and increase the expression level of a given gene in a host cell, multiple additional copies of a given gene operatively linked to various promoters according to the invention can be introduced in a given host cell. In this example, various promoters according to the invention operatively linked with the glaA coding sequence are introduced in a fungal host cell WT2. Example 7 and 8 describe a number of different steps in this process.

In order to introduce combinations of pGBTOPGLA-1, and pGBTOPGLA-16, -17 and -18 vectors in WT2, a cotransformation and subsequent transformant selection was carried out as described in WO98/46772 and WO99/32617. In principle, linear DNA of two of the above mentioned pGBTOPGLA vectors was isolated and co-transformed with an amdS selectable marker-gene containing vector, which is designated pGBAAS-1 (constructed as described in EP 635574B1). Both types of vectors comprise two DNA domains homologous to the *gla*A locus of A. *niger* host strain to direct targeting to the 3'-3" terminator locus of *gla*A in WT 2. Transformants were selected on acetamide media and colony purified according standard procedures. Cotransformants were identified using PCR techniques and colonies were diagnosed for glaA copy number and integration of two different pGBTOPGLA- contructs at the *gla*A locus. Transformants with 2 glaA copies and a combination of pGBTOPGLA-1/16, pGBTOPGLA-16/17 and pGBTOPGLA-17/18 were selected.

### Example 8. Production of the glucoamylase polypeptide encoded by the glaA coding sequences under control of multiple promoters of the invention in a fungal host cell.

The selected pGBTOPGLA-1/16, pGBTOPGLA-16/17 and pGBTOPGLA-17/18 strains, isolated in example 7, and strain WT 1 were used to perform shake flask experiments in 100 ml of the medium as described in EP 635 574 B1 at 34°C and 170 rpm in an incubator shaker using a 500 ml baffeled shake flask. After 4 and 6 days of fermentation, samples were taken to determine the glucoamylase activity. The glucoamylase activity in the selected pGBTOPGLA-16/17 and pGBTOPGLA-17/18 transformants of WT2 were increased compared to the pGBTOPGLA-1/16 transformants and WT 1, measured after either four or six days of fermentation (data not shown). This clearly shows that expression of the glaA gene under control of multiple and non-native promoters can provide increased expression of glaA.

### Example 9. Differential production of the laccase polypeptide encoded by the Pycnoporus cinnabarinus Icc3-1 gene in Pycnoporus cinnabarinus.

As depicted in detail below, it was demonstrated that SC3 promoter driven expression of *Icc3-1* results in the release of laccase at the periphery of colonies of P. *cinnabarinus.* In contrast, GPD promoter and laccase promoter driven expression result in the release of laccase in the centre and the middle of the colony, respectively.

By transforming strain BRFM 44 with two expression constructs, laccase was released at the periphery and the middle (SC3 and GPD driven expression of Icc3-1), the periphery and the centre (*SC3* and *GPD* driven expression) and the middle and the centre (Laccase promoter and GPD promoter driven expression) of the colony.

Accordingly, by using combinations of expression constructs multiple parts of the mycelium can be involved in secretion of laccase. This phenomenom is accompanied with an increase in laccase activity in liquid shaken cultures.

### 9.1 Materials and methods

### Cultivation of P. cinnabarinus

The monokaryotic laccase deficient *Pycnoporus cinnabarinus* strain BRFM 44 (Banque de Resources Fongiques de Marseille, Marseille, France) was routinely grown at 30 DEG C in liquid or solid (1.5 % agar) yeast malt medium (YM) containing per liter 10 g glucose, 5 g peptone, 3 g yeast extract, and 3 g malt extract. For laccase production, conditions were used that are optimal for laccase production in wild-type P. *cinnabarinus* (Lomascolo et al., 2003). Strains were grown in 250 ml minimal medium (MM) either or not containing filter sterilized ethanol in 1 L Erlenmeyer flasks at 250 rpm at 30 DEG C. MM contained per liter: 20 g maltose, 1 g yeast extract, 2.3 g C₄H₄O₆Na₂.2H₂O, 1.84 g (NH₄)2C₄H₄O₆, 1.33 g KH₂PO₄, 0.1 g CaCl₂.2H₂O, 0.5 g MgSO₄, 0.07 g FeSO₄.7H₂O 0.048 g ZnSO₄.7H₂O, 0.036 g MnSO₄.H₂O, 0.1 g CuSO₄ and 1 ml of a vitamin solution (Tatum et al., 1950).

### Transformation of P. cinnabarinus

*P. cinnabarinus* was transformed as described by Alves AM, Record E, Lomascolo A, Scholtmeijer K, Asther M, Wessels JG, and Wosten HA. Highly efficient production of laccase by the basidiomycete Pycnoporus cinnabarinus, Appl Environ Microbiol. 2004 Nov;70(11):6379-84. All steps in the transformation procedure were carried out at 30 DEG C unless stated otherwise. A 15 days-old colony (6-8 cm in diameter) was homogenized in 50 ml YM medium for 1 min in a Waring blendor. After adding the same volume of medium, the homogenate was grown for 24 h at 200 rpm. This culture was again homogenized, diluted twice in YM, and grown for 24 h at 200 rpm. The mycelium was protoplasted in 0.5 M MgSO₄ or 0.5 M sucrose with gentle shaking using 1 mg ml⁻¹ glucanex (Sigma-Aldrich). 1 E+7 protoplasts and 5 µg of plasmid DNA were incubated for 15 min on ice. After adding 1 volume of polyethyleneglycol 4000 the mixture was incubated for 5 minutes at room temperature. Protoplasts were regenerated overnight in 2.5 ml regeneration medium (Specht et al., 1988, Exp. Mycol. 12: 357-366). After adding three volumes of YM medium containing 5 µg ml⁻¹ phleomycin and 1 % low melting point agarose the mixture was spread on YM agar medium containing 5 µg ml⁻¹ of the antibiotic. Transformants in which a phleomycin resistance cassette was already introduced were retransformed with the same selection cassette by adding 500 µg ml⁻¹ caffeine to the medium in addition to the antibiotic.

### Construction of laccase expression vectors

To express the laccase *Icc3-1* gene from *P. cinnabarinus* behind the SC3 and GPD promoters of *S. commune,* its coding sequence was amplified by PCR using primers NcolPyc and BcllPyc (Table 3). This resulted in a fragment with an introduced Ncol site in the start codon and a *Bcl*I restriction site directly following the stop codon. To express the *Icc3-1* gene behind the laccase promoter, the coding sequence was amplified using primers PromoNCOforward and PromoLACreverse (Table 3), resulting in a fragment with an introduced Ncol site a the 5' end, and a Smal site immediately following the stop codon. The amplified coding sequences of *Icc3-1* were cloned in the expression vector pESC and its derivatives pEGP and pELP, resulting in plasmids pESCL1, pEGPL1, pELPL1, respectively. Plasmid pESC contains a phleomycin resistance cassette (Schuren and Wessels, 1994), in which the internal *Nco*I site has been deleted. Moreover, it contains the regulatory sequences of the SC3 gene in between which coding sequences can be cloned using Ncol and BamHI sites. The SC3 promoter is contained on a 1.2 kb *Hin*DIII/*Nco*I fragment, while its terminator consists of a 434 bp BamHI/EcoRI fragment. Plasmids pEGP and pELP are derivatives of pESC, in which the SC3 promoter is replaced for *Hin*DIII/*Nco*I promoter fragments of GPD (700 bp) (Harmsen et al. 1992) and laccase (2.5 kb), respectively. The laccase promoter was isolated as follows. *Bgl*II digested genomic DNA of *P. cinnabarinus* was circularized by self-ligation and used as a template for inverse PCR using the primers INVSE and INVASE (Table 3). The resulting 3.5 kb fragment was cloned in XL-TOPO (Invitrogen) resulting in plasmid pPL100. Sequencing confirmed that pPL100 contained a 2.5 kb promoter region. This region was amplified by PCR using primers promoLACforward and promoNCOrev (Table 3) introducing a HinDIII and a Ncol site at the 5' and 3' ends, respectively.

**Table 3. Primers used for construction of the laccase expression vectors, the respective primer sequences and the respective SEQ ID NO:'s.**

| **Primer** | **Sequence** | **SEQ ID NO:** |
|---|---|---|
| *Nco*IPyc | ttctgaccatggcgaggttccagtc | 5 |
| *Bcl*IPyc | acagtaactgattcagctcagaggtcgctg | 6 |
| PromoNCOforward | accccctctttctgaccatggcgaggttccagtc | 7 |
| PromoLACreverse | taacccgggcgctcagaggtcgctggggtcaagtgc | 8 |
| INVSE | tctgatcatgtcgaggttccagtcc | 9 |
| INVASE | gtcttcaaggacctgcggacagacatc | 10 |
| Promolacforward | accaagcttagatctccgaaccagaaatgc | 11 |
| PromoNCOrev | gactggaacctcgccatggtcagaaagaggggt | 12 |

### Laccase activity determination

Laccase activity of *P. cinnabarinus* strains was monitored on solid YM medium supplemented with 0.2 mM ABTS (2,2'-Azino-bis(3-ethylbenzothiazoline-6-sulfonic acid), Sigma-Aldrich) and 0.1 mM CuSO₄. Laccase activity in the culture medium was determined quantitatively by following the oxidation of 5 mM ABTS at 420 nm (extinction coefficient 36 000 mM⁻¹ cm⁻¹) in the presence of 50 mM Na-K-tartrate pH 4.0. Activity was expressed as nkat ml⁻¹. 1 nkat is defined as the amount of enzyme catalyzing the oxidation of 1 nmol of ABTS per second. Assays were carried out at 30 DEG C in triplicate. Standard deviation did not exceed 10 % of the average values.

### 9.2 Laccase production in Pycnoporus cinnabarinus

Previously, laccase production *in Pycnoporus cinnabarinus* was demonstrated according to Alves et al., *supra.*

The laccase deficient monokaryotic strain BRFM 44 (Banque de Resources Fongiques de Marseille, Marseille, France) of *Pycnoporous cinnabarinus* was transformed with the native *Icc3-1* laccase gene (SEQ ID NO: 4) placed under regulation of the laccase promoter (SEQ ID NO: 3) or that of the SC3 hydrophobin gene (SEQ ID NO: 2) or the glyceraldehyde-3-phosphate dehydrogenase (GPD) gene of *Schizophyllum commune* (SEQ ID NO: 1).SC3 driven expression resulted in a laccase activity of maximally 107 nkat ml-1 in liquid shaken cultures. This value was about 1.4 and 1.6 times higher in the case of the GPD and laccase promoters, respectively (Table 4). *Icc3-1* mRNA and laccase activity were strongly increased in the presence of 25 g L⁻¹ ethanol when *Icc3-1* was expressed behind either promoter (Table 3). Laccase production was further increased in transformants expressing the *Icc3-1* gene behind the laccase promoter or that of GPD by growing in the presence of 40 g L⁻¹ ethanol. In this case maximal activities were 3900 and 4660 nkat ml⁻¹, respectively, corresponding to 1 and 1.2 g of laccase per liter.

**Table 4. Laccase activity (nkat ml⁻¹) in media of 14-day old cultures of recombinant strains of P. cinnabarinus BRFM 44 expressing the laccase gene Icc3-1 behind the laccase promoter (L12-7 & L12-8), or that of the SC3 (S1 & S2) or GPD (G11 and G14) promoter of S. commune. Strains were grown in the presence or absence of 25 g L⁻¹ ethanol. n.d.: not detectable. Experiments were performed in triplicate. The standard deviation was less than 10%.**

| **Strain** | **promoter used** | **Activity -EtOH** | **Activity +EtOH** |
|---|---|---|---|
| BRFM 44 | - | n.d | n.d. |
| S1 | *SC3* | 107 | 431 |
| S2 | *SC3* | 49 | 138 |
| L12-7 | *Icc3-1* | 175 | 1223 |
| L12-8 | *Icc3-1* | 20 | 666 |
| G11 | *GPD* | 60 | 700 |
| G14 | *GPD* | 145 | 1393 |

### 9.3 Differential laccase production in Pycnoporus cinnabarinus

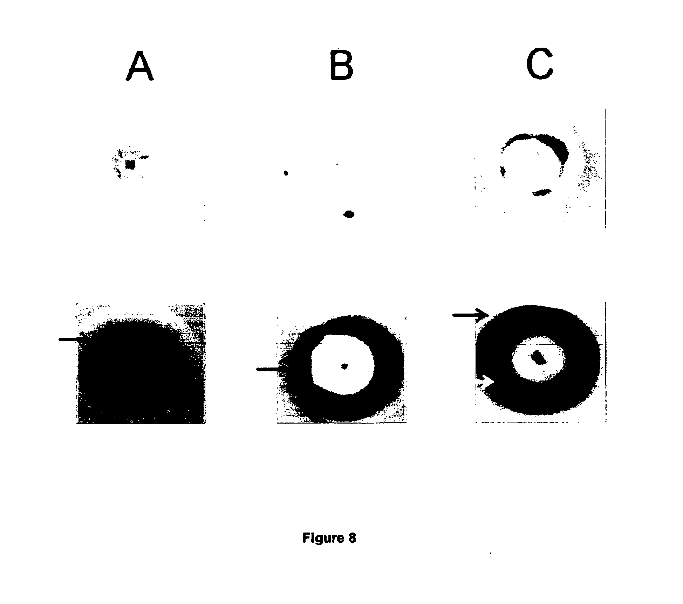

Constructs pESCL1, pEGPL1, pELPL1 expressing the laccase gene *Icc3-1* from the SC3, *GPD* and laccase promoter, respectively, were transformed to the laccase negative monokaryon BRFM 44 of *P. cinnabarinus* Alves et al., 2004, *supra).* Laccase producing transformants were selected by adding the substrate ABTS to the medium, which is converted in a green product by the enzyme. Spatial and temporal laccase activity was monitored, i.e. the laccase activity was monitored at various time intervals, and the locatlisation of the laccase activity in the colony was assessed. Two independent transformants of each construct were selected to exclude positioning effects. The presence of a PC membrane in between the agar medium and the colony did not affect the spatial and temporal activity of laccase on media containing ABTS. Colonies with introduced laccase gene(s) under regulation of the GPD promoter showed laccase activity exclusively in the centre of the colony starting at day 6 (Figure 8). After 10 days of growth activity had extended to the periphery and degradation of the substrate had increased. SC3 driven expression resulted in activity at the periphery of the colony from day 5 on. After 10 days of growth activity was still observed at the periphery and the intensity of the break down product had increased. Strains transformed with the laccase gene regulated by its own promoter showed activity in the middle of the colony. This activity was observed at day 4. After 10 days of growth the activity zone had extended outwards to a degree similar to that of the extension of the periphery of the colony. From these results we conclude that spatial and temporal activity of laccase in the medium depends on the promoter used.

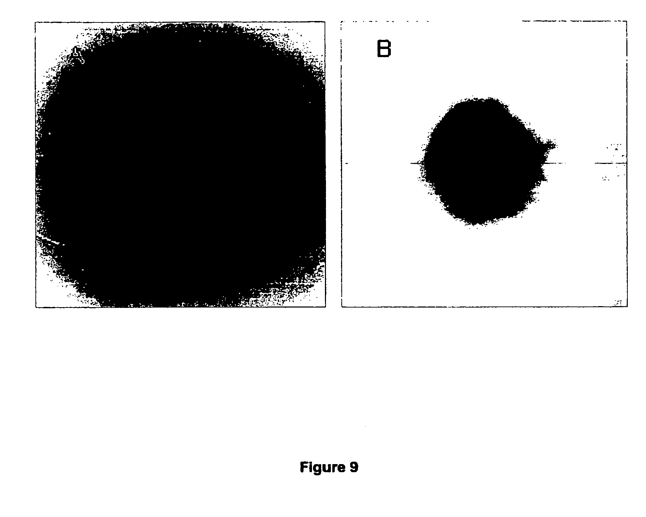

Recombinant strain G14 (transformed with pEGPL1) was re-transformed with pESCL1 using a selection with phleomycine in combination with 500 µg ml⁻¹ caffeine. This resulted in strains secreting laccase in two zones of the colony, corresponding to the zones of the single transformants. For instance strain G-S8 releases laccase both in the centre and at the peripheral part of the colony (Figure 9), the largest part of the mycelium being devoted to secretion.

Laccase activity was determined in liquid shaken cultures in the presence of 40 g L⁻¹ of ethanol. Strain G-S8 produced more laccase than its parent G14 (Table 5).

**Table 5. Laccase production in recombinant strains of P. cinnabarinus BMRF 44 (laccase negative background). S means that the strain has been transformed with a construct expressing Icc3-1 behind the SC3 promoter. Similarly, G and L mean that strains were transformed with a construct expressing the Icc3-1 gene from the GPD and laccase promoter, respectively. Strains are presented showing the highest activity out of 12 transformants that were selected on plate. Cultures were grown in the presence of 40 g L⁻¹ of ethanol.**

| **Transformant** | Activity (nkat/ml) | Production (g/L) |
|---|---|---|
| **S1** | 500 | 0.125 |
| **L12-7** | 3600 | 0.9 |
| **G14** | 4660 | 1.12 |
| **G-S8** | 5310 | 1.3 |

## Claims

1. A recombinant host cell comprising at least two DNA constructs, each DNA construct comprising a coding sequence in operative association with a promoter DNA sequence, wherein the at least two DNA constructs comprise at least two distinct promoter DNA sequences and wherein the coding sequences comprised in said DNA constructs encode related polypeptides.

2. The recombinant host cell according to claim 1, wherein the at least two distinct promoter DNA sequences possess distinct expression characteristics.

3. The recombinant host cell according to any one of claims 1 or 2, wherein at least one of the at least two promoter DNA sequences is selected from the group consisting of:
(a) a DNA sequence comprising a nucleotide sequence selected from the set consisting of: the promoter DNA sequences of the genes listed in Table 1 and SEQ ID NO's:1 to 4 and 13 to 55,
(b) a DNA sequence capable of hybridizing with the DNA sequence of (a),
(c) a DNA sequence sharing at least 80% homology with the DNA sequence of (a),
(d) a variant of any of the DNA sequences of (a) to (c), and
(e) a subsequence of any of the DNA sequences of (a) to (d).

4. The recombinant host cell of any one of claims 1 to 3, wherein said host cell is a fungal cell.

5. The recombinant host cell according to claim 4, wherein the host cell is an *Agaricus, Aspergillus, Penicillium, Pycnoporus* or *Trichoderma* species.

6. The recombinant host cell according to claim 5, wherein the *Aspergillus* is an *Aspergillus niger, Aspergillus sojae, Aspergillus oryzae* species.

7. The recombinant host cell according to any one of claims 1 to 6, wherein the coding sequence encodes an enzyme.

8. The recombinant host cell according to any one of claims 1 to 6, wherein the coding sequence encodes an enzyme involved in the production of a metabolite.

9. A method to prepare the recombinant cell of any one of claims 1 to 8, comprising:
(a) providing at least two DNA constructs, each DNA construct comprising a coding sequence in operative association with a promoter DNA sequence, wherein the at least two DNA constructs comprise at least two distinct promoter DNA sequences and wherein the coding sequences comprised in said DNA constructs encode related polypeptides,
(b) providing a suitable host cell, and
(c) transforming said host cell with said DNA constructs.

10. The method according to claim 9, wherein step (c) is performed by at least two separate transformation events.

11. A method for the expression of a coding sequence, comprising:
(a) providing at least two DNA constructs, each DNA construct comprising a coding sequence in operative association with a promoter DNA sequence, wherein the at least two DNA constructs comprise at least two distinct promoter DNA sequences and wherein the coding sequences comprised in said DNA constructs encode related polypeptides,
(b) providing a suitable host cell,
(c) transforming said host cell with said DNA constructs,
(d) culturing said host cell under conditions conducive to expression of the coding sequence.

12. The method according to claim 11, wherein step (c) is performed by at least two separate transformation events.

13. A method for the expression of a coding sequence, comprising culturing the recombinant host according to any one of claims 1 to 8 under conditions conducive to expression of the coding sequence.

14. A method for the production of a polypeptide, comprising:
(a) culturing a recombinant host cell comprising at least two DNA constructs, each DNA construct comprising a coding sequence in operative association with a promoter DNA sequence, wherein the at least two DNA constructs comprise at least two distinct promoter DNA sequences and wherein the coding sequences comprised in said DNA constructs encode related polypeptides, under conditions conducive to expression of the polypeptide,
(b) optionally recovering the polypeptide from the culture broth, and
(c) optionally purifying the polypeptide.

15. A method for the production of a polypeptide, comprising:
(a) culturing the recombinant host cell according to any one of claims 1 to 8 under conditions conducive to expression of the polypeptide,
(b) optionally recovering the polypeptide from the culture broth, and
(c) optionally purifying the polypeptide.

16. A method for the production of a metabolite, comprising:
(a) culturing the recombinant host cell according to claim 8 under conditions conducive to production of the metabolite,
(b) optionally recovering the metabolite from the culture broth, and
(c) optionally purifying the metabolite.
